# FASCICULE DE BREVET EUROPEEN

(11) **EP 0 320 495 B1**
(45) Date de publication et mention de la délivrance du brevet: **02.08.2000**
(21) Numéro de dépôt: 89101328.6
(22) Date de dépôt: 22.01.1987
(51) Int. Cl.: C12P 21/02, C12N 15/00, C12N 5/00, G01N 33/569, A61K 39/21

(54) **Procédé de fabrication récombinante des protéines derivées de HIV-2 et culture cellulaire exprimant des protéines de HIV-2**
Prozess zur rekombinanten Herstellung von HIV-2 - Proteinen sowie Zellen, die HIV-2 - Proteine exprimiert
Process for the recombinant production of proteins of HIV-2 and cells expressing HIV-2 proteins

(30) Priorité: 22.01.1986 FR 8600910; 22.01.1986 FR 8600911; 06.02.1986 FR 8601635; 13.02.1986 FR 8601985; 18.03.1986 FR 8603881; 24.03.1986 FR 8604215; 03.03.1986 US 835228; 06.10.1986 US 916080; 21.11.1986 US 933184
(43) Date de publication de la demande: 14.06.1989
(62) Demande divisionnaire de: 87400151.4
(73) Titulaire: INSTITUT PASTEUR, 75724 Paris Cédex 15 (FR)
(72) Inventeur: Montagnier, Luc, F-92350 Le Plessis-Robinson (FR); Chamaret, Solange, F-75015 Paris (FR); Guetard, Denise, F-75015 Paris (FR); Alizon, Marc, F-75005 Paris (FR); Clavel, François, F-75016 Paris (FR); Guyader, Mireille, F-75016 Paris (FR); Sonigo, Pierre, F-75015 Paris (FR); Brun-Vezinet, Françoise, F-75005 Paris (FR); Rey, Marianne, F-75004 Paris (FR); Rouzioux, Christine, F-75015 Paris (FR); Katlama, Christine, F-75004 Paris (FR)
(74) Mandataire: Gutmann, Ernest

(56) Documents cités:
- NATURE, vol. 313, 7 février 1985, pages 450-458; M.A. MUESING et al.: "Nucleic acid structure and expression of the human AIDS/lymphadenopathy retrovirus"
- THE LANCET, no. 8469/8470, 21/28 décembre 1985, pages 1387-1389; F. BARIN et al.: "Serological evidence for virus related to simian T-lymphotropic retrovirus III in residents of West Africa"
- COMPTES RENDUS DE L'ACADEMIE DES SCIENCES PARIS, tome 302, série III, no. 13, 7 avril 1986, pages 485-488, Académie des Sciences; F. CLAVEL et al.: "LAV type II: un second rétrovirus associé au SIDA en Afrique de l'Ouest"
- NATURE, vol. 324, 18/25 décembre 1986, pages 691-695; F. CLAVEL et al.: "Molecular cloning and polymorphism of the human immune deficiency virus type 2"
- NATURE, vol. 326, 16 avril 1987, pages 662-669; M. GUYADER et al.: "Genome organization and transactivation of the human immunodeficiency virus type 2"
- BRUN-VEZINET, F. ET AL.: "Lymphadenopathy-associated virus type 2 in AIDS and AIDS-related complex",THE LANCET,17.01.87, pages 128-132

## Description

Cette demande de brevet est une demande divisionnaire à la demande EP.87/400.151.4. déposée le 22.01.1987.

L'invention est relative à une nouvelle classe de virus, ayant la capacité de provoquer des lymphadénopathies susceptibles d'être relayées ensuite par le syndrome d'immuno-déficence acquise (SIDA) chez l'homme. Sont ainsi décrits des antigènes susceptibles d'être reconnus par des anticorps induits chez l'homme par cette nouvelle classe de virus , et les anticorps induits par des antigènes obtenus à partir de ces virus.

La présente demande décrit en outre, des séquences d'ADN cloné soit présentant une analogie de séquence, soit une complémentarité avec l'ARN génomique du virus précité. Elle décrit aussi des procédés de préparation de ces séquences d'ADN cloné.

La demande de brevet décrit aussi des polypeptides contenant des séquences d'amino-acides codées par les séquences d'ADN cloné , ainsi que des applications de ces antigènes au diagnostic in vitro chez l'homme de potentialités de certaines formes du SIDA et, en ce qui concerne certains d'entre eux, à la production de compositions immunogènes et de compositions vaccinantes contre ce rétrovirus. De même la demande décrit les applications aux mêmes fins des susdits anticorps et, pour certains d'entre eux, leur application à la production de principes actifs de médicaments contre ces SIDAS humains.

L'invention décrit enfin l'application des séquences d'ADN cloné et de polypeptides obtenus à partir de ces séquences comme sondes dans des kits de diagnostic.

L'isolement et la caractérisation d'un premier rétrovirus, dénommé LAV, dont avait été reconnue la responsabilité dans le développement du SIDA, a fait l'objet d'une description dans un article de F. BARRE-SINOUSSI et al dès 1983 (Science, vol. 220, n° 45-99, 20, p. 868-871. L'application au diagnostic de la présence d'anticorps contre le virus de certains extraits de ce dernier, et plus particulièrement de certaines de ses protéines, a été plus spécialement décrite dans la demande de brevet européen n° 138.667. Depuis, d'autres souches similaires et des variants du LAV ont été isolés. On rappellera pour mémoire ceux connus sous les désignations HTLV-III et ARV.

En application des nouvelles règles de nomenclature publiées dans Nature, en mai 1986, les rétrovirus susceptibles d'induire chez l'homme les susdites lymphadenopaties et le SIDA, seront désignés globalement par "HIV", abréviation de l'expression anglaise "Human immunodeficiency Virus" (ou virus d'immunodéficience humaine). Le sous-ensemble des rétrovirus formés par LAV et de ces variants a initialement été désigné par les expressions "LAV type I" ou "LAV-I". Le même sous-ensemble sera désigné ci-après par la désignation HIV-1, étant entendu que l'expression LAV sera encore conservée pour désigner celle parmi les souches de rétrovirus (notamment LAAV, IDAV-1 et IDAV-2) appartenant à la classe des virus HIV-1 décrite dans la susdite demande de brevet européen 138.667, qui a été utilisée dans les essais comparatifs décrits plus loin, à savoir LAV_{BRU}, qui a été déposée à la Collection des Cultures Nationales de Microorganismes (CNCM) de l'Institut Pasteur de Paris, France, le 15 Juillet 1983, sous le n° I-232.

Le nouveau rétrovirus faisant l'objet du présent brevet et les souches de virus qui en sont proches et qui, comme lui sont susceptibles de se multiplier dans des lymphocytes humains, antérieurement appelées "LAV type II". ou "LAV II" sont désormais appelées "HIV-2", étant entendu que les désignations de certains isolats de HIV-2 décrits plus loin seront suivies des trois lettres faisant référence aux malades à partir desquels ils ont été isolés.

On peut définir l'ensemble "HIV-2" comme un ensemble de virus ayant in vitro un tropisme pour des lymphocytes T4 humains, et qui ont un effet cytopathogène à l'égard de ces lymphocytes, lorsqu'ils s'y multiplient, pour alors causer soit des poly-adénopathies généralisées et persistantes, soit un SIDA. Les rétrovirus HIV-2 se sont révélés distincts des virus de type HIV-1 dans les conditions évoquées plus loin. Comme ces derniers, ils sont distincts des autres rétrovirus humains déjà connus (HTLV-I et HTLV-II).

Bien qu'il y ait une assez grande variabilité génétique du virus, les différentes souches HIV-1 isolées à ce jour, à partir des malades américains, européens, haïtiens et africains ont en commun des sites antigéniques conservés sur leurs principales protéines : protéine du noyau (core) p25 et glycoprotéine d'enveloppe gp110 et protéine transmembranaire gp41-43. Cette parenté permet par exemple à la souche prototype LAV d'être utilisée comme souche d'antigènes pour la détection d'anticorps contre tous les virus de la classe HIV-1, chez toutes les personnes qui les portent, quelle que soit leur origine. Cette souche est donc utilisée actuellement pour la détection d'anticorps anti-HIV-1 chez les donneurs de sang et les malades par les techniques d'immunofluorescence, notamment par les techniques dites ELISA, "Western Blot" (ou immuno-empreintes) et "RIPA" abréviation anglaise de "Radio Immunoprecipitation Assay" (test de radio-immunoprécipitation).

Cependant dans une étude sérologique effectuée avec un lysat de HIV-1 sur des malades originaires de l'Afrique de l'Ouest, il a été observé que certains d'entre eux donnaient des réactions séro-négatives ou très faiblement positives, alors qu'ils présentaient les signes cliniques et immunologiques du SIDA Barin, F. et al (Lancet ii, 1387, 1985).

C'est à partir des lymphocytes mis en culture de l'un de ces malades, que l'on a isolé un premier rétro-virus HIV-2, dont la structure en microscopie électronique et le profil des protéines en gel d'électrophorèse SDS ont une similitude avec ceux de HIV-1. Mais ce nouveau rétro-virus HIV-2 ne présente globalement qu'une faible parenté avec HIV-1, tant du point de vue de l'homologie antigénique de ses protéines que de l'homologie de son matériel génétique.

CLAVEL et al (Compte Rendu de l'Académie des Sciences - Tome 302, Avril 1986), relatent l'isolement d'un rétrovirus ayant des propriétés morphologiques et biologiques similaires à celles de LAV-1 préalablement isolé (en mai 1983) mais dont la parenté antigénique et moléculaire avec LAV-1 est très éloignée. Ce virus a été isolé chez deux malades africains atteints de SIDA. L'éloignement de ce nouveau virus par rapport à LAV-1 est attesté par l'absence de réaction lors de tests d'hybridation avec une sonde LAV-1 dans des conditions stringentes.

CLAVEL et al (Nature, vol. 324, 18-25, Décembre 1986) décrivent les résultats des travaux de clonage, et concluent que les protéines de HIV-1 et HIV-2 ont des tailles différentes et que leur réactivité sérologique croisée est restreinte à la protéine majeure du noyau, alors que la glycoprotéine d'enveloppe de HIV-2 n'est pas immunoprécipitée par des sérums positifs pour HIV-1. Les résultats des tests d'hybridation entre le génome de HIV-1 et celui de HIV-2, confirment que HIV-2 n'est pas un variant de HIV-1, qu'en fait ces rétrovirus sont différents.

Brun-Vézinet et al (The Lancet, 1987, pages 128-132) décrivent la caractérisation d'isolats de HIV-2 et décrivent les antigènes de rétrovirus HIV-2, ainsi que des tests de réaction croisée entre les antigènes de HIV-1 et les antigènes de HIV-2.

Ce nouveau rétrovirus ou des rétrovirus ayant des propriétés antigéniques et immunologiques équivalentes, peuvent donc constituer des sources d'antigènes pour le diagnostic de l'infection par ce virus et des variants qui induisent un SIDA, du type de celui que l'on avait observé pour les premières fois chez des malades africains ou des personnes ayant séjourné en Afrique.

Le premier isolement de ce virus a été réalisé à partir du sang, prélevé sous héparine, d'un malade de 28 ans, hétérosexuel, qui n'avait jamais été transfusé et qui n'était pas toxicomane. Il présentait depuis 1983 une importante diarrhée chronique, un amaigrissement important (17 kg) avec de la fièvre par intermittence. Plus récemment il avait présenté des infections à Candida et Serratia, dont une candidose oesophagienne, typique du SIDA.

Ce patient présentait également une anémie, une anergie cutanée, une lymphopénie, un rapport lymphocytes T4/lymphocytes T8 de 0,15, avec un taux en lymphocytes T4 inférieur à 100 par mm³ de sérum. Ses lymphocytes en culture ne répondaient pas à la stimulation par la phytohémoglutinine et la concanavaline A. On a également diagnostiqué chez ce malade des bactériémies récurrentes dues à S. enteriditis, des cryptosporidioses, des infections dues à Isospora belli et des toxoplasmoses cérébrales.

L'ensemble de ces signes témoignait des symptômes "complexes liés au SIDA" ou "ARC" (abréviation anglaise de "AIDS Related Complex"), du type de ceux causés par le virus HIV-1. Ces différentes observations étaient également conformes aux critères apliqués par le Centre de Contrôle des Maladies (Center of Disease Control ou CDC) d'Atlanta, Etats-Unis.

La mise en culture des lymphocytes de ces malades et l'isolement du rétrovirus ont été effectués selon la technique déjà décrite pour l'isolement du HIV-1 (1) dans l'article de BARRE-SINOUSSI et Coll. et la demande de brevet européen n° 84 401834/0 138 667. On les rappelle brièvement ci-après. Des lymphocytes stimulés pendant 3 jours par de la phytohémagglutinine (PHA) ont été cultivés en milieu de culture RPM1-16-40 additionné de 10 % de sérum de veau foetal et de 10⁻⁵M β-mercaptoéthanol, d'interleukine-2 et de sérum anti-interféron α humain.

La production de virus a été suivie par son activité transcriptase inverse. Dans le surnageant de culture, le pic d'activité virale est apparu entre le 14ème et le 22ème jour, puis a diminué. Le déclin et la mort de la culture cellulaire ont suivi. Comme avec le HIV-1, des coupes de lymphocytes infectés par HIV-2 révèlent en microscopie électronique des virions parvenus à maturité et des particules virales bourgeonnant à la surface des cellules infectées. Les lignées cellulaires utilisées pour réaliser les cultures de ces virus isolés peuvent être selon les cas, les lignées cellulaires du type HUT, CEM, MOLT ou de toute lignée lymphocytaire immortalisée portant dès récepteurs T4.

Le virus a ensuite été propagé sur des cultures de lymphocytes de donneurs de sang, puis sur des lignées continues d'origine leucémique, telles que HUT 78. Il a été caractérisé comme étant sensiblement distinct du HIV-I par ses antigènes et son acide nucléique. Le virus a été purifié comme décrit dans les documents antérieurs déjà mentionnés. Un premier isolat de ce virus a été déposé à la C.N.C.M. le 19 décembre 1985, sous le n° I-502. Il a été désigné postérieurement sous le nom LAV-II MIR. Un second isolat a été déposé à la C.N.C.M le 21 février 1986 sous le n° I-532. Ce second isolat a pour nom de référence LAV-II ROD. Il sera quelquefois fait simplement référence plus loin, à MIR ou à ROD.

La demande de brevet décrit tout variant des virus précédents ou tout virus équivalent (par exemple tel que HIV-2 IRMO et HIV-2 EHO déposés à la CNCM le 19 Décembre 1986 sous les numéros I-642 et I-643 respectivement) contenant des protéines de structure qui présentent les mêmes propriétés immunologiques que celles des virus HIV-2 déposés à la CNCM sous les numéros I-502, ou I-532. Il sera encore revenu plus loin sur les définitions critères d'équivalence.

La demande décrit aussi un procédé de production du virus HIV-2 ou de variants de celui-ci dans les lignées cellulaires permanentes dérivées de lymphocytes T4, ou portant le phénotype T4, ce procédé consistant à cultiver ces lignées préalablement infectées avec le virus HIV-2, et notamment lorsque le niveau d'activité de la transcriptase inverse (ou reverse-transcriptase) a atteint un seuil déterminé, à récupérer les quantités de virus libérées dans le milieu de culture.

Une lignée permanente préférée en vue de la culture de HIV-2 est, par exemple, du type cellules HUT 78. Une lignée de HUT 78, infectée par HIV-2, a été déposée le 6 février 1986 à la CNCM sous le n° I-519. La culture est, par exemple, réalisée comme suit :

Les cellules HUT 78 (10⁶/ml) sont mises en cocultures avec des lymphocytes humains normaux infectés (10⁶/ml). Le milieu de culture est du RPMI 1640 avec 10% de sérum de veau foetal. Au bout de 15 à 21 jours, on observe un effet cytopathogène des cellules HUT 78. On dose la reverse transcriptase une semaine après cette observation, dans le surnageant de culture. On peut alors commencer à récupérer le virus à partir de ce surnageant.

Une autre lignée préférée pour la culture appartient aux lignées des cellules connues sous la désignation CEM.

L'infection, puis la culture des cellules CEM infectées peuvent notamment être réalisées comme suit.

On procède à la coculture de lymphocytes T4 préalablement infectés avec le virus HIV-II et de cellules non infectées de la lignée CEM pendant le temps nécessaire à l'infection des CEM. On poursuit d'ailleurs ensuite les conditions de culture dans un milieu approprié, par exemple celui décrit ci-après et, lorsque l'activité transcriptase inverse des cellules infectées a atteint un niveau suffisant, on récupère le virus produit à partir du milieu de culture.

On a notamment réalisé une co-culture dans les conditions précisées ci-après de lymphocytes T4 humains qui avaient été infectés cinq jours auparavant avec une souche de virus HIV-II provenant d'un patient désigné ci-après "ROD", d'une part, et de CEM, d'autre part.

Les lymphocytes T4 infectés, préalablement activés par la phytohémaglutinine, se sont révélés posséder une activité de transcriptase inverse de 5 000 cpm/10⁶ lymphocytes T normaux trois jours après le début de l'infection. La culture a été poursuivie jusqu'à ce que l'activité transcriptase inverse mesurée ait atteint 100 000 cpm dans le surnageant. Ces lymphocytes T4 ont alors été mis en contact avec les cellules CEM (3.10⁶ lymphocytes T normaux infectés) et incubées dans le milieu de culture suivant : RPMI 1640 contenant 2,92 mg/ml de L glutamine, 10 % de sérum de veaux foetal décomplémenté, 2 µg/ml de polybrène, 0,05 % de sérum anti-interféronalpha, 100 000 µg/ml de pénicilline, 10 µg/ml de streptomycine et 10 000 µg/ml de néomycine.

Le milieu de culture est changé deux fois par semaine.

Les mesures d'activité transcriptase inverse mesurée dans le surnageant ont été les suivantes :

| | |
|---|---|
| - au jour 0 | 1 000 (bruit de fond) |
| - au jour 15 | 20 000 |
| - au jour 21 | 200 000 |
| - au jour 35 | 1 000 000 |

Une culture CEM infectée par le virus HIV-2 a été déposée à la Collection Nationale de Culture de Micro-organismes de l'Institut Pasteur (C.N.C.M) sous le n° I-537 le 24 mars 1986.

Quelques caractéristiques des antigènes et des acides nucléiques, entrant dans la constitution de HIV-2, résultent des essais réalisés dans les conditions indiquées ci-après. Elles seront souvent mieux appréciées par comparaison avec des caractéristiques du même type se rapportant à d'autres types de rétrovirus, notamment HIV-1 et SIV.

Il sera fait référence dans ce qui suit aux dessins dans lesquels :
- les figures 1a, 1b et 1c se rapportent à des essais d'immunoprécipitation croisée entre des sérums respectivement de patients infectés par HIV-1 et HIV-2, et de macaques rhésus infectés avec STLV-III, d'une part, et des extraits viraux de HIV-1, d'autre part ;
- les figures 2a et 2b présentent des résultats comparatifs quant aux mobilités électrophorétiques des protéines de HIV-1, HIV-2 et STLV-III respectivement, dans des gels de polyacrylamide-SDS ;
- la figure 3 présente des résultats d'hybridation croisée entre des séquences génomiques de HIV-1, HIV-2 et STLV-III, d'une part, et des sondes contenant différentes séquences subgénomiques du virus HIV-1, d'autre part.
- la figure 4 est une carte de restriction de l'ADNc dérivé de l'ARN de HIV-2 ROD ;
- la figure 5 est une carte de restriction d'un fragment E2 de l' ADNc dérivé de HIV-2, ce fragment contenant une région correspondant à la région LTR 3' de HIV-2 ;
- la figure 6 est la séquence nucléotidique d'une partie de E2, cette séquence correspondant à la région U3/R de HIV-2 ;
- la figure 7 fait apparaïtre :
   - d'une part et schématiquement, des éléments de structure de HIV-1 (figure 5A) et, alignée avec une région contenant le LTR 3' de HIV-1, la séquence dérivée de la région E2 de l'ADNc de HIV-2 et,
   - d'autre part, les mises en alignement au prix d'un certain nombre de délétions et d'insertions, des nucléotides communs, contenus respectivement dans la séquence dérivée de la région E2 de HIV-2, et dans la séquence correspondante de HIV-1 (fig. 5 B)

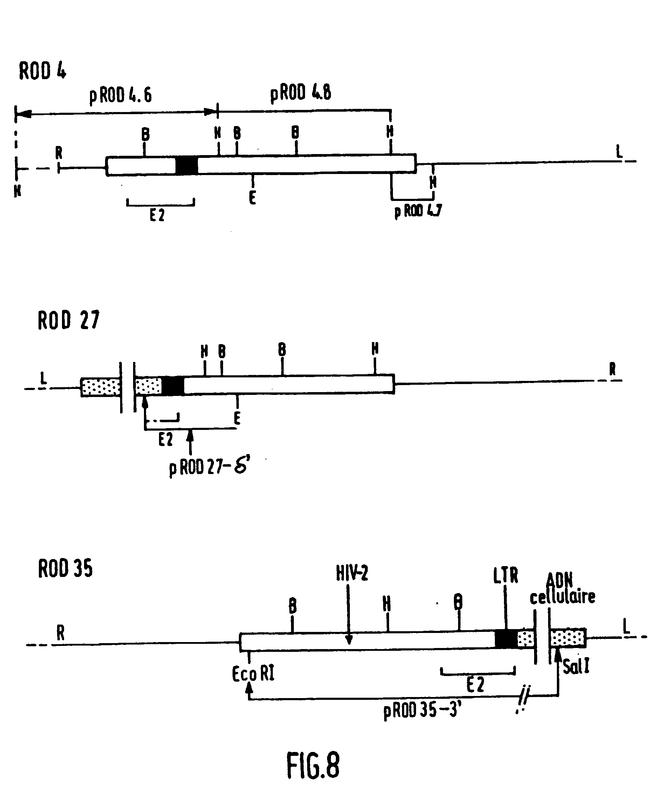

- la figure 8 montre schématiquement les structures de plusieurs clones d'un phage λ modifié par divers insérats issus de l'ADNc dérivé d'HIV-2 (clones ROD4, ROD27 et ROD35) ; on y a également représenté schématiquement des séquences dérivées à leur tour de ROD4, ROD27 et ROD35, sous-clonées dans un plasmide pvc 18, ces dernières séquences étant placées en correspondance avec les régions de ROD4, ROD27 et ROD35 dont elles sont respectivement issues.

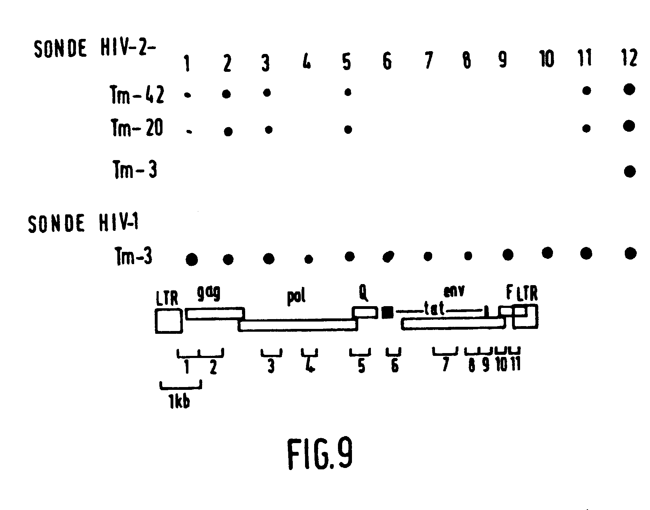

- la figure 9 fait apparaître les intensités relatives d'hybridation entre
   a/ onze fragments prélevés à partir de différentes régions du génome complet de HIV-1 (schématiquement représentés dans la partie inférieure de la figure d'une part, et l'ADNc de HIV-2, contenu dans ROD4, d'autre part, et
   b/ des fragments issus de HIV-II avec le même ADNc.

D'une façon générale, les antigènes de HIV-2 utilisés dans les tests comparatifs dont la description suit sont issus de la souche de HIV-2 MIR déposée à la C.N.C.M. sous le n° I-502, et les séquences d'ADN dérivés de l'ADN génomique de HIV-2 sont issus de la souche HIV-2 ROD déposée à la C.N.C.M. sous le n° I-532.

L'invention concerne un procédé de fabrication de l'une des protéines de HIV-2 (p12, p16 ou p26) ou d'une protéine ayant la structure de gp140, ou de parties déterminées de ces protéines, ce procédé étant caractérisé par l'insertion de la séquence d'acide nucléique correspondante dans un vecteur susceptible de transformer un hôte cellulaire choisi et de permettre l'expression d'un insérat contenu dans ce vecteur, par la transformation de cet hôte choisi par ledit vecteur contenant ladite séquence nucléique, par la culture de l'hôte cellulaire transformé et par la récupération et la purification de la protéine exprimée.

En particulier, l'invention concerne un procédé de fabrication d'une protéine ou d'une glycoprotéine ou d'une partie de protéine ou de glycoprotéine consistant dans le produit d'expression d'une séquence d'acide nucléique dérivée du génome d'un rétrovirus, caractérisé par
- l'insertion, dans un vecteur susceptible de transformer un hôte cellulaire choisi et de permettre l'expression dans cet hôte d'un insérat contenu dans ce vecteur, de la susdite séquence d'acide nucléique, dérivée d'un rétrovirus humain HIV-2 capable d'infecter des lymphocytes T4 humains et susceptible de provoquer un SIDA chez l'homme, ledit rétrovirus HIV-2 étant représenté par les isolats déposés à la CNCM sous les n° I-502, I-532, I-642 et I-643 ou à l'ECACC sous les n° 87.01.1001 et 87.01.1002, ou l'insertion de la susdite séquence d'acide nucléique dérivée d'un variant de ce rétrovirus dont la glycoprotéine majeure d'enveloppe est reconnue par des anticorps formés contre la glycoprotéine majeure d'enveloppe gp140 d'un rétrovirus HIV-2,
- l'introduction dans l'hôte choisi, dudit vecteur contenant ladite séquence nucléique,
- la culture de l'hôte cellulaire tranformé et
- la récupération et la purification de la protéine ou de la partie de protéine exprimée par ladite séquence d'acide nucléique, ladite protéine ou partie de protéine étant apte à produire une réaction immunologique spécifique avec des anticorps dirigés contre le rétrovirus HIV-2.

L'invention a aussi pour objet une culture cellulaire caractérisée en ce qu'elle est transformée par un ADN recombinant contenant un acide nucléique dérivé d'une partie au moins de l'ARN du rétrovirus humain HIV-2 ou de l'un de ses variants, dans des conditions permettant la production d'une protéine ou d'une partie de protéine selon le procédé ci-dessus décrit, ledit acide nucléique ayant la capacité de s'hybrider avec l'ARN dudit rétrovirus humain HIV-2 et n'hybridant pas dans les conditions stringentes avec les sondes 1 à 4 comprenant respectivement les nucléotides 990-1070, 990-1260, 2170-2240, 3370-3640 de l'ADN dérivé du génome de HIV-1.

Le procédé de l'invention peut être appliqué à la fabrication d'une protéine ou d'une partie de protéine utilisable dans des réactions de détection d'anticorps dirigés contre le rétrovirus HIV-2.

### I - ANTIGENES, NOTAMMENT PROTEINES ET GLYCOPROTEINES

Le virus initialement cultivé dans HUT 78 a été marqué métaboliquement par la ³⁵S- cystéine et la ³⁵S-méthionine, les cellules infectées étant incubées en présence de Ces acides aminés radioactifs en milieu de culture dépourvu de l'acide aminé non marqué correspondant, pour une période de 14 à 16 heures, en particulier selon la technique décrite dans l'article répertorié en (21) dans la bibliographie présentée à la fin de la description, pour ce qui est du marquage à la ³⁵ S- cystéine. Le surnageant est ensuite clarifié, puis le virus ultracentrifugé une heure à 100 000 g sur un coussin de 20 % de saccharose. Les principaux antigènes du virus sont séparés par électrophorèse dans un gel de polyacrylamide (12,5 %), dans des conditions dénaturantes (SDS) ou dans un gel de polyacrylamide (10 %) + bisacrylamide (0,13 %) avec SDS (0,1 % en concentration finale). On utilise comme poids moléculaire de référence les marqueurs colorés suivants

| BRL : | |
|---|---|
| myosine : | 200 Kd |
| phosphorylase B : | 97,4 Kd |
| BSA : | 68 Kd |
| ovalbumine : | 43 Kd |
| α chymotrypsine : | 25,7 Kd |
| β lactoglobuline : | 18,4 Kd |
| lysozyme : | 14,3 Kd |

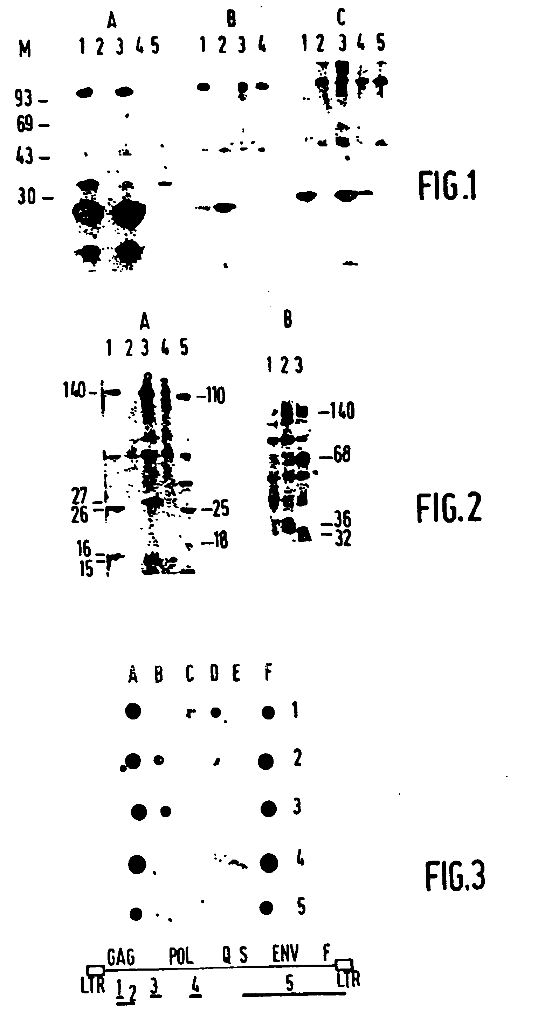

D'autres marqueurs de poids moléculaires ont été utilisés dans d'autres essais. Il est en particulier fait référence aux figures 1a, 1b et 1c qui renvoient à d'autres marqueurs de poids moléculaires connus (sous la lettre M. dans ces figures). Les antigènes sont encore mieux distingués après immunoprécipitation (RIPA) ou par immunoempreintes (Western blot) en utilisant les anticorps présents dans le sérum du malade : leurs poids moléculaires apparents déterminés par leurs migrations apparentes sont très proches de ceux des antigènes du HIV-1.

D'une façon générale il est précisé que dans ce qui suit les nombres qui suivaient les indications "p" et/ou "gp" correspondent aux poids moléculaires approximatifs des protéines et/ou glycoprotéines correspondantes divisés par 1000. Par exemple la p36 a un poids moléculaire de l'ordre de 36 000. Il est cependant entendu que ces valeurs de poids moléculaires peuvent varier dans un intervalle pouvant atteindre 5%, voire 10% ou même plus, selon les techniques utilisées pour les déterminations de ces poids moléculaires.

La répétition des essais a permis de mieux cerner les poids moléculaires apparents des antigènes de HIV-2. Ainsi on a constaté que les poids moléculaires des trois protéines du noyau (core), auxquelles on avait initialement attribué des poids moléculaires de l'ordre de 13.000, 18.000 et 25.000 respectivement, avaient en fait des poids moléculaires apparents plus proches des valeurs suivantes : 12.000, 16.000 et 26.000, respectivement. Ces protéines sont ci-après respectivement désignées par les abréviations p12, p16 et p26.

De même l'existence de bandes protéine ou glycoprotéine dont les poids moléculaires apparents ont été appréciés à des valeurs qui pouvaient s'étager de 32.000 à 42.000-45.000. La répétition des mesures a finalement permis de préciser une bande correspondant à un poids moléculaire apparent de 36.000. Dans ce qui suit, cette bande est désignée par l'abréviation p36. Une autre bande à 42.000-45.000 (p42) est constamment observée aussi. L'une ou l'autre des p36 ou p42 constitue vraisemblablement une glycoprotéine transmembranaire du virus.

Une glycoprotéine majeure d'enveloppe ayant un poids moléculaire de l'ordre de 130-140 Kd est constamment observée : cette glycoprotéine est ci-après désignée par l'expression gp140. Il convient de noter que, d'une façon générale, les poids moléculaires sont appréciés avec une précision de plus ou moins 5 %, cette précision pouvant même devenir un peu moindre pour les antigènes de haut poids moléculaire, comme on l'a constaté pour la gp140 (poids moléculaire de 140 Kd ± 10 %). L'ensemble de ces antigènes sont peu ou pas reconnus (lorsqu'ils sont marqués par la ³⁵S-cystéine) par des sérums de malades contenant des anticorps anti-HIV-1 dans les systèmes de détection utilisés au laboratoire ou par utilisation des tests mettant en oeuvre des lysats de HIV-1, tels que ceux commercialisés par DIAGNOSTICS PASTEUR sous la marque "ELAVIA". Seule la protéine p26 a été faiblement immunoprécipitée par de tels sérums. La protéine d'enveloppe ne l'a pas été. Le sérum du malade infecté par le nouveau virus (HIV-2) reconnaït faiblement une protéine p34 du HIV-1. Dans le système de détection utilisé, les autres protéines du HIV-1 n'ont pas été reconnues.

En revanche le HIV-2 possède certaines protéines présentant une certaine parenté immunologique avec des protéines ou glycoprotéines structurales semblables, séparables dans des conditions analogues, à partir d'un rétrovirus récemment isolé à partir de singes macaques rhésus captifs, de l'espèce den macaques rhésus, alors que cette parenté immunologique tend à s'effacer pour d'autres protéines ou glycoprotéines. Ce dernier rétrovirus, qui est présumé être l'agent étiologique de SIDAs chez les singes, a été désigné par les chercheurs qui l'ont isolé (références bibliographiques (16-18) ci-après) sous l'appellation "STLV-III_{ma c}". Pour la commodité du langage, il ne sera plus désigné dans ce qui suit que par l'expression "STLV-III" (ou encore par l'expression SIV, abréviation anglaise de ^{"}Simian Immunodeficiency Virus" (virus d'immunodéficience du singe)).

Un autre rétrovirus, désigné "STLV-III_{A GM}", (ou SIV_{AG M}) a été isolé chez des singes verts sauvages. Mais, contrairement au virus présent chez le singe macaque rhésus, la présence de "STLV-III_{A GM}" ne semble pas induire une maladie du type SIDA chez le singe vert d'Afrique.

Toutefois, la parenté immunologique des protéines et des glycoprotéines structurales, et par conséquent la parenté de leurs séquences nucléiques, de HIV-II d'une part,et des rétrovirus STLV-III_{m ac} et STLV-III_{A GM} d'autre part, reste limitée. Des expériences ont permis d'établir une première distinction entre les rétrovirus capables d'infecter l'homme ou le singe, il en ressort ceci :
- le virus HIV-II ne se multiplie pas de façon chronique dans les lymphocytes du singe macaque rhésus, lorsqu'il a été injecté in vivo et dans des conditions opératoires permettant le développement du virus STLV-III_{ma c} telles qu'elles ont été décrites par N.L. Letvin et al, Science (1985)vol 230, 71-75.

Cette apparente inaptitude de HIV-2 à se développer chez le singe dans conditions naturelles, permet de différencier biologiquement le virus HIV-II d'une part, et les isolats du virus STLV-III, d'autre part.

En mettant en oeuvre les mêmes techniques que celles rappelées plus haut, il a été constaté que l'on pouvait également obtenir à partir de STLV-III :
- une protéine principale du noyau p27, ayant un poids moléculaire de l'ordre de 27 kilodaltons,
- une glycoprotéine majeure d'enveloppe, gp140,
- une protéine vraisemblablement transmembranaire p32, qui n'est pas observée en RIPA lorsque le virus a au préalable été marqué par la ³⁵S-cystéine, mais qui peut être observée dans les essais d'immunoempreintes (Western blots), sous forme de bandes larges.

La glycoprotéine majeure d'enveloppe de HIV-2 s'est révélée être plus proche immunologiquement de la glycoprotéine majeure d'enveloppe de STLV-III que de la glycoprotéine majeure d'enveloppe de HIV-1.

Ces constatations s'imposent non seulement au niveau des poids moléculaires : 130-140 kilodaltons pour les glycoprotéines majeures de HIV-2 et de STLV-III, contre environ 110 pour la glycoprotéine majeure d'enveloppe de HIV-1, mais aussi au niveau des propriétés immunologiques, puisque des sérums prélevés à partir de malades infectés par HIV-2, et plus particulièrement des anticorps formés contre la gp140 de HIV-2 reconnaissent la gp140 de STLV-III, alors que dans des essais semblables les mêmes sérums et les mêmes anticorps de HIV-2 ne reconnaissent pas la gp110 de HIV-1. Mais les sérums anti-HIV-1 qui n'ont jamais réagi avec la gp140 de HIV-2 précipitent une protéine de 26 Kdal marquée par la ³⁵S-cystéine, contenue dans les extraits de HIV-II.

La protéine majeure du noyau (core) de HIV-2 semble présenter un poids moléculaire moyen (environ 26.000) intermédiaire entre celui de la p25 de HIV-1 et la p27 de STLV-III.

Ces observations résultent des essais réalisés avec des extraits viraux obtenus à partir du HIV-2 isolé à partir de l'un des patients susmentionnés. Des résultats similaires ont été obtenus avec des extraits viraux du HIV-2 isolé à partir du second patient.

Des cellules infectées respectivement avec HIV-1, HIV-2 et STLV-III ont été incubées dans un milieu contenant 200 µCi/ml de ³⁵S-cystéine dans un milieu exempt de cystéine non marquée pendant 16 heures. Les surnageants clarifiés ont été centrifugés à 60.000 g pendant 90 minutes. Les culots ont été lysés dans un tampon RIPA(1), immunoprécipités avec différents sérums, puis soumis à une électrophorèse sur gel de polyacrylamide chargé en dodécylsulfate de sodium (SDS-PAGE).

Les résultats observés sont illustrés par les figures 1a, 1b et 1c.

Dans la figure 1a, sont présentés les résultats d'immunoprécipitation observés entre un extrait viral de HIV-1 obtenu à partir d'une lignée cellulaire CEM C1.13 et les sérums respectifs suivants : - sérum positif anti-HIV-1 (bande 1),
- sérum obtenu du premier patient mentionné plus haut (bande 2),
- sérum d'un porteur africain sain d'anticorps anti-HIV-1 (bande 3),
- sérum obtenu à partir d'un macaque infecté avec STLV-III (bande 4) et
- sérum du deuxième patient susmentionné (bande 5).

Dans la figure 1b, sont rapportés les résultats d'immunoprécipitation observés entre les antigènes de HIV-2 obtenus à partir du premier patient, après culture préalable sur des cellules HUT-78, et différents sérums, plus particulièrement le sérum du premier patient sus-mentionné (bande 1), le sérum positif anti-HIV-1 (bande 2), le sérum du macaque infecté par STLV-III (bande 3), le sérum du second patient susdit (bande 4).

Enfin, la figure 1c illustre les résultats d'immunoprécipitation observés entre les antigènes d'un isolat de STLV-III otenu à partir d'un macaque présentant un SIDA de singe. Les sérums utilisés et auxquelles se réfèrent les bandes 1 à 5 sont les mêmes que ceux rappelés plus haut en rapport avec la figure 1a.

M se réfère aux marqueurs myosine (200 Kd) galactosidase (130 Kd), sérum albumine bovine (69 Kd), phosphorylase B (93 Kd), ovalbumine (46 Kd) et carbonate anhydrase (30 Kd).

Les figures 2a et 2b présentent les résultats comparatifs quant aux mobilités électrophorétiques des protéines de HIV-1, HIV-2 et STLV-III.

La figure 2a se rapporte aux essais réalisés avec des extraits de virus marqués par la ³⁵S-cystéine, après immunoprécipitation sur SDS-PAGE. Les différentes bandes se rapportent aux extraits de virus suivants : virus obtenu à partir du patient 1 et immunoprécipité par le sérum provenant du même patient (bande 1), extrait du même virus immunoprécipité avec un sérum témoin négatif provenant d'une personne ne portant pas d'anticorps anti-HIV-1 ou anti-HIV-2 (bande 2), extrait de virus STLV-III immunoprécipité par un sérum provenant d'un macaque infecté par STLV-III (bande 3), immunoprécipitations observées entre des extraits du même virus et d'un sérum témoin négatif (bande 4), extrait de HIV-1 immunoprécipité par un sérum d'un patient européen infecté par le SIDA.

La figure 1b présente les résultats obtenus dans des essais de Western-blot (immunoempreinte). Des lysats cellulaires provenant de cellules HUT-78 non infectées ou infectées ont été soumis à une électrophorèse sur SDS-PAGE, puis transférés électrophorétiquement sur un filtre de nitrocellulose, avant d'être mis a réagir avec le sérum du premier patient susmentionné (sérum dilué au 1/100e). Le filtre de nitrocellulose a ensuite été lavé et la détection des anticorps fixés révélée avec des IgG anti-humaines de chèvre marquées par ¹²⁵I.

Les taches observées dans des bandes 1, 2 et 3 se rapportent respectivement aux essais d'agglutination entre le susdit sérum et des extraits de cellules HUT-78 non infectées (bande 1), des extraits de cellules HUT-78 infectées avec un virus HIV-2 (bande 2) et des extraits de cellules HUT-78 infectées par STLV-III (bande 3). Les nombres qui apparaissent dans les marges de chacune des bandes correspondent aux poids moléculaires approximatifs des protéines virales les plus représentatives (poids moléculaires en kilodaltons).

### II - ACIDES NUCLEIQUES

### 1) les ARNs du rétrovirus HIV-2

L'ARN du virus, déposé sur filtre d'après la technique du "spot blot" n'a pas hybridé, dans des conditions stringentes avec l'ADN du HIV-1.

Par "conditions stringentes" on entend : les conditions dans lesquelles la réaction d'hybridation entre l'ARN du HIV-2 et la sonde choisie marquée radioactivement au p³² (ou marquée de façon différente), puis les lavages de la sonde sont faits. L'ybridation, sur membrane, est faite à 42°C en présence d'une solution aqueuse à 50% (volume/volume) de 0,1% SDS/5x55c, notamment formamide pendant 18 heures. La membrane sur laquelle a été faite la réaction d'hybridation est lavée ensuite à 65°C dans un tampon contenant 0,1 % de SDS et 0,1 X SSC.

Par "conditions non stringentes", on entend les conditions dans lesquelles la réaction d'hybridation et les lavages sont faits. L'hybridation est réalisée en mettant en contact avec la sonde choisie marquée au P³² (ou marquée autrement), à savoir à 42°C dans un tampon 5 x SSC, 0,1% SDS contenant 30 % de formamide pendant 18 heures. Le lavage de la membrane est réalisé à 50°C avec un tampon contenant 0,1 % de SDS et 2 X SSC.

On a également réalisé des essais d'hybridation avec une sonde d'hybridation constituée par un plasmide recombinant pBT1 obtenu par clonage de l'ADN du HIV-1 provenant de λJ19 (Cell 1985, vol. 40, p.9) dans le vecteur pUC18. En conditions non stringentes, on n'a observé qu'une très faible hybridation entre l'ARN de HIV-2 et l'ADN cloné dérivé de HIV-1.

D'autres sondes contenant des séquences clonées de HIV-1 ont été utilisées :
a) Des sondes simple brin de l'ADN du HIV-1 subgénomique élaborées à partir de sous-clones du génome du HIV-1 et insérées dans le phage M13. Les régions clonées concernaient le gène protéase ou le gène "endonucléase".
   Seule une sonde de la région endonucléase de HIV-1 (séquence de nucléotides comprise entre les bases n° 3760 et 4130) a donné une hybridation faible en conditions non stringentes avec le HIV-2. La sonde "protéase" (séquence de nucléotides de HIV-1 comprise entre les bases n° 1680 et 1804) n'a pas hybridé, même en conditions non stringentes avec le HIV-2.
b) Une sonde pRS3 constituée par la séquence codant pour la région "enveloppe" du HIV-1 (sous-clonage dans pUC18) n'a pas donné d'hybridation en conditions non stringentes avec HIV-2.

La technique du "spot blot"est appelée aussi "dot blot" (transfert par taches).

Des résultats d'hybridation supplémentaires entre des ARNs génomiques de HIV-1, HIV-2 et STLV-III, d'une part, et des sondes contenant différentes séquences sub-génomiques du virus HIV-1, d'autre part, apparaissent dans la figure 3.

Les surnageants des différents milieux de culture (à raison de 0,5 à 1 ml pour chaque tache) ont été centrifugés pendant 5 minutes à 45.000 tours par minute ; les culots ont été remis en suspension dans un tampon NTE contenant 0,1 % de SDS et déposés sur filtre de nitrocellulose. Celui-ci a été prétrempré dans un milieu 2 x SSC (NaCl 0,3 M, citrate de sodium 0,03 M). Après cuisson (pendant 2 heures à 80°C), les filtres ont été hybridés avec diverses sondes contenant des sous-fragments génomiques de HIV-1, dans des conditions non stringentes (30 % de formamide, 5 x SSC, 40°C), lavés avec une solution 2 x SSC et contenant 0,1 % de SDS, à 50°C, puis autoradiographiés pendant 48 heures à -70°C avec des écrans d'amplification.

Les sondes 1-4 sont des sondes à brin unique obtenues par la méthode de "coupe principale" (prime cut method) telle que décrite en (25). En bref des fragments mono-brins issus du virus M13 et portant des insérats de HIV-I subgénomiques (30) ont été ligaturés à des fragments d'oligomères (17 nucléotides) issus de M13 (BIOLABS). Le brin complémentaire a été ensuite synthétisé avec l'enzyme de Klenow dans un tampon TM (Tris 10 mM, pH 7,5, MgCl₂, 10 mM) en présence de dATP, dGTP, dTTP et dCTB marqués par du ³²P en alpha (Amersham, 3000 Ci/mMol). L'ADN a ensuite été digéré par les enzymes de restriction appropriés, dénaturé par la chaleur et soumis à électrophorèse sur un gel dénaturant de polyacrylamide (contenant 6 % d'acrylamide, de l'urée 8 N dans un tampon TDE). Le gel a ensuite été autoradiographié pendant 5 minutes . La sonde a ensuite été découpée et éluée dans un tampon de NaCl 300 mM, SDS 0,1 %. Des activités spécifiques (AS) de ces sondes à brin unique ont été estimées à 5.10⁸-10⁹ désintégrations par minute/microgramme (dpm/µg).

Les séquences caractéristiques contenues dans les différentes sondes étaient les suivantes :
sonde 1 : nucléotides 990-1070,
sonde 2 : nucléotides 980-1260,
sonde 3 : nucléotides 2170-2240,
sonde 4 : nucléotides 3370-3640.

Les numérotations des nucléotides qui précédent sont celles envisagées dans l'article référencé en (30).

Enfin la sonde 5 consiste en un plasmide pUC-18 portant le fragment EcoR1-Sac1 du HIV cloné dans λJ19 (31), lequel a fait l'objet d'une translation de coupure (nick-translation) pour obtenir un AS d'approximativement 10⁸ dpm/µg.

Les dispositions relatives des fragments subgénomiques contenus dans les sondes vis-à-vis du génome entier de HIV-1 sont schématisées à la figure 3. Les différentes taches correspondent respectivement :
- tache A : virus obtenus à partir d'une culture de cellules CEM C1.13 infectées par HIV-1,
- tache B : virus obtenus à partir de cellules HUT-78 infectées par STLV-III,
- taches C et D : isolats respectivement obtenus à partir des virus des deux patients africains susmentionnés,
- tache E : extrait cellulaire témoin négatif obtenu à partir de cellules HUT-78 non infectées,
- tache F : virus obtenu à partir d'un patient du Zaïre affecté par le SIDA et ayant été cultivé sur des lymphocytes T normaux en présence de TCGF.

Toutes les taches ont été obtenues avec une quantité de virus correspondant à 25.000 dpm en activité transcriptase inverse, sauf pour les taches C : 15.000 dpm.

Les observations faites ont été les suivantes :

Les ARNs génomiques des deux isolats de HIV-2, obtenus à partir de particules virales purifiées, n'ont hybridé avec aucune des sondes dans les conditions stringentes sus-indiquées, bien que les particules virales aient été isolées et purifiées à partir de surnageants de cultures de cellules hautement infectées témoignant d'une activité transcriptase inverse élevée.

Dans les conditions non stringentes sus-indiquées, les observations suivantes ont été faites.

Toutes les sondes ont hybridé de façon intense avec les ARNs génomiques obtenus des préparations témoins de HIV-1 et d'un autre isolat obtenu à partir d'un patient du Zaïre souffrant du SIDA.

Deux des sondes obtenues (nucléotides 990-1070 et 990-1260, toutes deux issues de la région gag de HIV-1) ont hybridé légèrement avec les taches des extraits des rétrovirus HIV-2; l'une seulement de ces deux sondes (nucléotides 990-1260) a aussi montré une légère hybridation avec la tache de STLV-III (figure 3). En ce qui concerne la sonde contenant un fragment de la région pol (nucléotides 2170-2240), on a observé une hybridation avec STLV-III et, mais de façon beaucoup plus faible, avec l'ARN de HIV-2. L'autre sonde de la région pol (nucléotides 3370-3640) n'a donné d'hybridation avec aucune des taches de HIV-2 et de STLV-III.

Enfin, la sonde modifiée par translation de coupure et contenant la totalité du gène env et le LTR (nucléotides 5290-9130) de HIV-1 n'a hybridé ni avec les ARNs de STLV-III, ni avec ceux de HIV-2.

On remarquera encore qu'une autre sonde, qui contenait l'extrémité 5' du cadre de lecture de pol de HIV-1 (correspondant à la région protéase) n'a hybridé ni avec les ARNs de HIV-2, ni avec les ARNs de STLV-III.

Il résulte donc encore de ce qui précède que le virus HIV-2 paraît plus éloigné, sur le plan structural, du virus HIV-1 qu'il ne l'est de STLV-III. HIV-2 diffère néanmoins de façon significative de STLV-III, ce qui vient à l'appui des résultats différents observés quant aux capacités infectieuses des virus HIV-2 pratiquement nulles chez les singes, comparés aux capacités infectieuses certaines des STLV-III chez les mêmes espèces de singes.

Les cartes de restriction et les séquences de l'ARN génomique de HIV-2 ou des cADNs obtenus à partir de ces ARNs génomiques sont accessible à l'homme du métier, dès lors que les souches de HIV-2 déposées à la C.N.C.M. peuvent, après multiplication appropriée, mettre en ses mains le matériel génétique nécessaire aux déterminations de ces cartes de restriction et séquences nucléotidiques. Les conditions dans lesquelles la carte de restriction du génome de l'un des isolats de HIV-2 de cette invention ont été établies et les conditions dans lesquelles certaines des parties de ADNc dérivées de ces génomes ont été séquencées sont décrites ci-après.

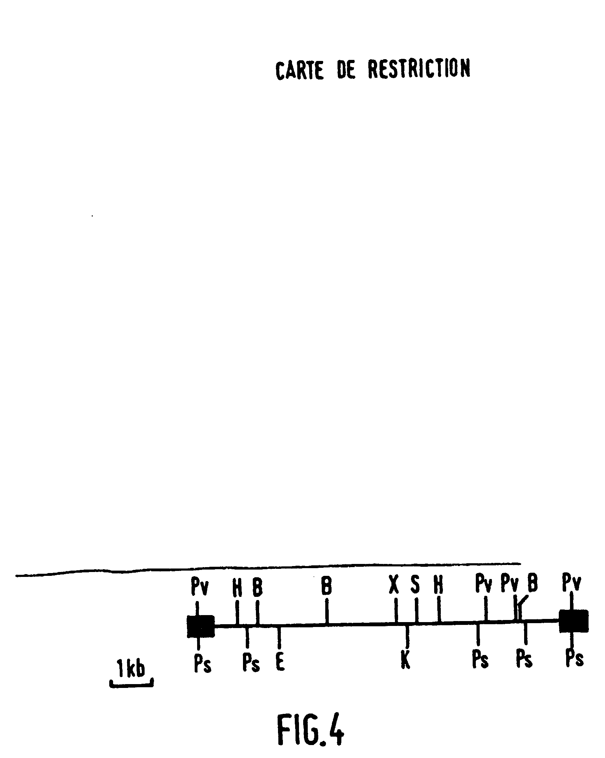

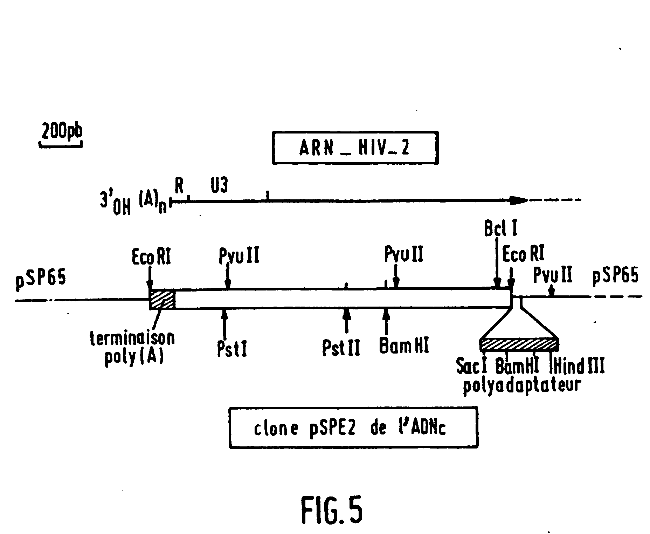

La carte de restriction du génome d'un rétrovirus représentatif des rétrovirus HIV-2 est représentée dans la figure 4. La carte de restriction d'un fragment important de cet ADNc apparaît à la figure 5. Enfin une partie de ce dernier fragment a été séquencé.

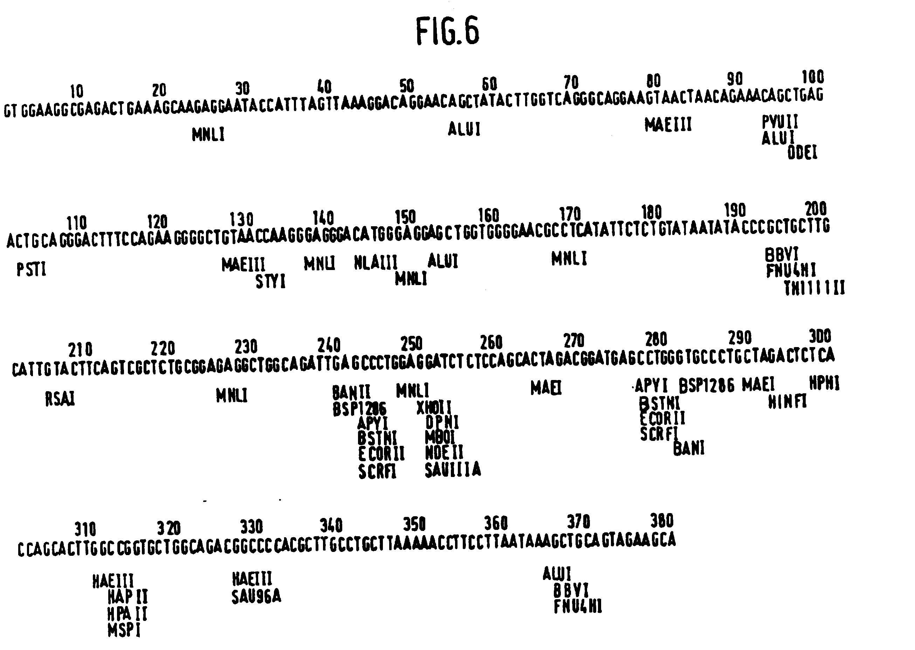

Cette séquence et un certain nombre des sites de restriction qu'elle contient apparaissent à la figure 6. L'ADNc entier cloné -ou des fragments clonés de cet ADNc-peuvent eux-mêmes être utilisés comme sondes d'hybridation spécifiques.

### 2) ADNc et fragments de cet ADNc, respectivement dérivés de l'ARN de HIV-2

Les conditions dans lesquelles l'ADNc susdit a été obtenu, sont décrites ci-après.

La première étape de fabrication de cet ADNc a compris la production d'un oligo (dT) servant d'amorce ou d'un brin d'ADNc initiateur, par la réalisation d'une réaction endogène activée par un détergent, utilisant la transcriptase inverse de HIV-2, sur des virions purifiés, obtenus à partir de surnageants de cellules CEM infectées. La lignée cellulaire CEM était une lignée cellulaire lymphoblastoïde CD4+ décrite par G.E. Foley et al. dans Cancer 18: 522-529 (1965), dont le contenu est considéré comme faisant partie de la présente description. Ces cellules CEM utilisées sont infectées avec un isolat ROD, qui s'est révélé produire de façon continue des quantités importantes de HIV-2.

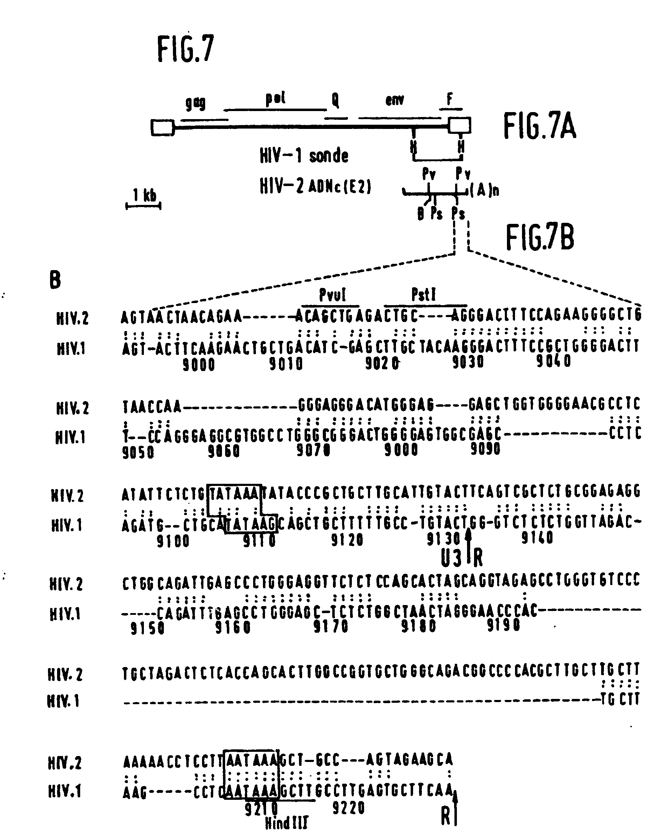

Après la synthèse du second brin (en présence de nucléotides et d'une ADN polymérase bactérienne), les cADNs double-brins ont été insérés dans un vecteur de phage bactérien M13 TG130. Une banque de phages de 10⁴ phages recombinants M13 a été obtenue et soumise à un tri in situ avec une sonde HIV-1. Celle-ci contient un fragment de 1.5kb issu de l'extrémité 3' de l'ADNc dérivée de l'ARN de l'isolat LAV (montré dans la figure 7 A). Environ 50 plaques positives ont été détectées, purifiées et caractérisées par une hybridation croisée des insérats et séquençage des extrémités.

Cette procédure a permis l'isolement de différents clones contenant des séquences approximativement complémentaires de l'extrémité 3' de l'ARN polyadénylé de la région de "répétition longue terminale" LTR (abréviation anglaise de "long terminal repeat" de HIV-1, décrite par S. Wain Hobson et al. dans Cell 40: 9-17 (1985)) dont le contenu est considéré ici comme faisant partie de la description.

Le plus important des insérats du groupe concerné de clones de M13 hybridant avec la région LTR3' de HIV-1, est un clone désigné par E2, d'environ 2 kb. Comme la région LTR3' de HIV-1, le clone E2 contient un signal AATAAA situé à environ 20 nucléotides en amont d'une partie terminale poly A, et une région LTR3' correspondant à celle de HIV-2. Après séquençage partiel, cette région LTR3' de HIV-2 s'est révélée présenter une parenté éloignée avec le domaine homologue de HIV-1.

La figure 5 est une carte de restriction du fragment de E2 (zone rectangulaire allongée) incorporé dans le plasmide pSPE2 qui le contient. Il comprend une partie de la région R, la région U3 de HIV-2. Le dessin ne fait pas apparaître les limites des régions R et U3.

La séquence d'une partie de E2 apparaît à la figure 6. Les positions de sites de restriction spécifiques y sont indiquées. Le faible degré de parenté entre les régions LTR3' de HIV-1 et HIV-2 est illustré par la figure 7. En effet, à peu près 50% seulement des nucléotides des deux LTR peuvent être mis en alignement (homologue de séquence d'environ 50%) au prix d'une centaine d'insertions ou de délétions. En comparaison, l'homologie de séquence des régions correspondantes des isolats américains et africains différents variants de HIV-1 est supérieure à 95%, sans insertion ni délétion.

Le clone E2 a été utilisé comme sonde spécifique d'HIV-2, pour l'identification sur filtre d'hybridation des séquences issues de HIV-2, contenues dans d'autres clones.
Cette sonde détecte également l'ARN génomique de HIV-2 dans des conditions stringentes. Elle permet, de même, la détection par la méthode dite de "Southern blot" sur l'ADN de cellules CEM ou analogues infectées avec un isolat ROD ou d'autres isolats de HIV-2. Aucun signal n'est détecté dans les mêmes conditions de stringence dans des essais d'hybridation de cette sonde avec des ADNc issus de cellules non infectées ou de cellules infectées par HIV-1. Ces résultats ont confirmé la nature exogène de HIV-2 vis-à-vis de HIV-1. Une espèce d'environ 10 kb, correspondant probablement à l'ADN viral non intégré, a été détecté à titre principal dans l'ADN non digéré de cellules infectées par HIV-2. Un autre ADN ayant une taille apparente de 6 kb correspondant peut être à une circulaire de l'ADN viral, a été détecté aussi.

Les autres parties du génome de HIV-2 ont été identifiées également. A cet effet, une banque génomique a été construite dans le phage lambda L47. Le phage lambda L47.1 a été décrit par W.A.M. Loenen et al. dans Gène 10:249-259 (1980), publication dont le contenu est considéré comme faisant partie de la présente description.

La banque génomique est construite avec des fragments obtenus par digestion de l'ADN provenant de la lignée cellulaire CEM infectée avec HIV-2_{R OD}, après digestion avec l'enzyme Sau3AI.

Environ 2 x 10⁶ plaques de recombinants ont été triées in situ avec un clone contenant l'insérat E2 marqué de l'ADNc de HIV-2. Dix phages recombinants ont été détectés sur plaques et purifiés. Les cartes de restriction de trois de ces phages, caractérisés par leur capacité d'hybridation en "Southern blot" avec l'insérat E2 dans des conditions stringentes, ainsi qu'avec des sondes subgénomiques de HIV-1 dans des conditions non-stringentes.

Un clone portant un insérat de 9.5 kb et dérivant de l'ADN viral circulaire entier, contenant le génome complet de HIV-2, a été identifié. Il a été désigné "Lambda ROD 4". Les deux autres clones, Lambda ROD 27 et Lambda ROD 35 dérivant de provirus intégrés, portant des séquences LTR des séquences codantes virales et des séquences adjacentes d'ADN cellulaire. Les différentes séquences découlent de la figure 8.

Des fragments des clones Lambda ont été sous-clonés dans plasmide vecteur plasmidique pUC 18. Les fragments issus de λ ROD 4, λ ROD 27 et λ ROD 35 et respectivement sous-clonés dans le susdit vecteur plasmide apparaissant également à la figure 8. Les sous-clones suivants ont été obtenus.
- pROD 27-5 dérive de Lambda ROD 27, contient une région de 5,2 kb du génome de HIV-II et des séquences cellulaires adjacentes (5'LTR et séquence virale codante 5' autour d'un site ECo RI).
- pROD 4.8 dérivé de Lambda ROD 4, contient un fragment Hind III d'environ 5 kb. Ce fragment correspond à la partie centrale du génome HIV-2.
- pROD 27-5' et pROD 4.8 contiennent des insérats de HIV-2 qui se chevauchent mutuellement.
- pROD 4.7 contient un fragment HIND III de 1.8 kb de Lambda ROD 4 ; ce fragment est placé dans la direction 3 vis-à-vis du fragment sous-cloné dans p ROD 4.8 et contient environ 0.8 kb de séquences virales codantes et une partie située entre les sites de clonage Bam H1 et Hind III du bras gauche du phage Lambda (Lambda L 47.1).
- pROD 35 contient toutes les séquences HIV-2 codantes dans la direction 3, vis-à-vis du site Eco R1, l'extrémité 3' LTR et environ 4 kb de séquences nucléiques adjacentes d'origine cellulaire.
- pROD 27-5' et pROD 35 contenus dans E.Coli HB 101, ont été déposés à la CNCM sous les numéros respectifs I-626 et I-633. le 21 Novembre 1986.
- pROD 4.7 et pROD 4.8, contenus dans E.Coli TG1, ont été déposés à la CNCM respectivement sous les numéros 1-627 et 1-628, le 21 Novembre 1986.

Le génome complet de HIV-2 ROD, dont la carte de restriction apparaît à la figure 4, a été reconstitué à partir de pROD 35, linéarisé au préalable avec Eco R1, de pROD 27-5'. L'insérat Eco R1 de pROD 27-5 a été ligaturé dans l'orientation correcte dans le site EcoRI de pROD 35.

Le degré de parenté entre HIV-2 et les autres rétrovirus humains ou simiens a été apprécié par des expériences d'hybridation mutuelle. L'homologie relative entre les différentes régions de génomes de HIV-1 et de HIV-2 a été déterminée par des essais d'hybridation de fragments respectivement issus de génome de HIV-1 cloné et de Lambda ROD 4 marqué radioactivement. Les positions relatives de ces fragments (numérotés de 1 à 11) vis à vis du génome de HIV-1 apparaîssant au bas de la figure 9.

Même dans des conditions de très faible stringence (Tm-42°C.) les génomes de HIV-1 et HIV-2 n'hybrident qu'aux niveaux de leurs gênes respectifs gag (taches 1 et 2), des régions reverse transcriptase dans pol (tache 3), des régions d'extrémités de pol, des gènes Q (ou sor) (tache 5) et des gènes F (ou 3' orf) et 3' LTR (tache 11). Le fragment HIV-1 utilisé pour détecter les premiers clones ADNc de HIV-2 correspond au sous-clone de la tache 11, qui hybride relativement bien avec HIV-2 dans des conditions non stringentes. Un signal provenant de la tache 5 est le seul persistant après un lavage stringent. Le gène d'enveloppe, la région du gène tat et une partie de pol apparaissent très divergents. Ces données, ainsi que la séquence obtenue avec LTR (figure 3) démontrent que HIV-2 n'est pas (en tous les cas au niveau de son enveloppe) un variant de HIV-1.

On observe que HIV-2 est plus proche de SIV (décrit par M.D.Daniel et al. dans Science 228: 1201-1204 (1985), dont le contenu doit être considéré comme faisant partie de la présente description) qu'il ne l'est de HIV-1.

Toutes les protéines de SIV, y compris la protéine de l'enveloppe, sont immuno-précipitées par des sérums de patients infectés par HIV-2, tandis que la réactivité sérologique croisée de HIV-1 et HIV-2 est limitée aux protéines du noyau. Cependant SIV et HIV-2 peuvent être distingués par les différences mentionnées plus haut au niveau des poids moléculaires de leurs protéines.

En ce qui concerne les séquences nucléotidiques, on remarque aussi que HIV-2 a une parenté avec SIV.

De plus, la caractérisation de HIV-2 permet aussi de délimiter le domaine de la glycoprotéine d'enveloppe qui est responsable de la fixation du virus à la surface des cellucles cibles et de l'internalisation ultérieure du virus. L'interaction se fait par l'intermédiaire de la molécule CD4 elle-même et il semble que HIV-1 et HIV-2 utilisent le même récepteur. Aussi, bien qu'il existe de grandes différences entre les gènes env de HIV-1 et HIV-2, les domaines homologues restreints des enveloppes des deux HIV peuvent-ils être considérés comme constituants de fixation à un récepteur commun des lymphocytes T4. Ces sites sont appelés à constituer des épitopes porteurs de l'immunogénicité de peptides qui pourraient être utilisés pour susciter chez l'homme une réponse immunitaire protectrice contre les virus HIV.

Des séquences avantageuses pour la constitution de sondes dans des réactions d'hybridation avec le matériel génétique de patients porteurs de virus ou de provirus, notamment pour détecter la présence d'ARN de virus HIV-é dans leurs lymphocytes contiennent une séquence nucléotidique résultant de combinaison des fragments de 5 kb de HindIII de ROD 4 et de l'ADNc E2. Les essais peuvent être réalisés selon toutes méthodes notamment selon les techniques de "Northern blot", "Southern blot" et "dot blot".

Des caractéristiques supplémentaires de l'invention apparaîtront encore de façon non limitative au cours de la description qui suit d'exemples d'identification de certaines parties du génome rétroviral et de la production d'un certain nombre d'ADNs recombinants mettant en jeu diverses parties d'un ADNc dérivé du génome rétroviral de HIV-2.

### EXEMPLES

### EXEMPLE I

### Sonde ADN, pour l'utilisation dans les kits de diagnostic de HIV-2.

Un ADNc complémentaire de l'ARN du génome obtenu à partir de virions purifiés, a été préparé selon la méthode suivante :

Le surnageant obtenu après 48 heures de culture de cellules CEM infectées par un isolat HIV-2 ROD de HIV-2 a été ultracentrifugé. Le culot de centrifugation contenant le virion a été centrigué sur gradient de sucrose pour former un nouveau culot de centrifugation substantiellement par la même méthode que celle décrite dans la demande de brevet européen 84/401 234/0.138.667 déjà mentionnée.

La préparation de HIV-2 purifié a été utilisée pour la synthèse d'ADNc en mettant en oeuvre une réaction endogène, activée par un détergent.

En résumé, la préparation du virion a été ajoutée à un mélange de réaction contenant 50mM Tris-HCl, 5mM MgCl₂, 10 mM DTT, 0.025% du détergent commercialisé sous la marque TRITON, et 50 µM de chacun des 4 déoxynucleoside-triphosphates et un initiateur oligo (dT). La réaction a été conduite pendant 90 minutes à 37°C.

Après l'extraction au phénol des protéines présentes dans le premier milieu de réaction, la seconde chaîne de l'ADNc a été synthétisée en présence de RNAse, ADN polymerase 1 de E.coli et des 4 déoxynucléotides, pendant 1 heure à 15°C et 1 heure à 22°C. Des extrémités franches ont été créées sur cette double chaîne ADNc, par l'action de la T4 ADN polymérase. Tous les réactifs de ce procédé sont disponibles dans le commerce (kit ADNc de la Société AMERSHAM) et ont été utilisés comme recommandé par le fournisseur.

Après (1) ligation d'adaptateurs (linkers) contenant un site EcoR1 (commercialisés par Pharmacia) aux extrémités franches de l'ADNc en présence d'une ligase ADN T4 (commercialisée par BIOLABS)(2), digestion de ces "linkers" par l'endonucléase de restriction EcoR1, et (3) élimination des fragments de "linker" par une filtration sur gel (colonne du gel commercialisé sous la marque ULTROGEL) AcA 34 (LKB-IBF), l'ADNc est inséré dans un vecteur M 13 TG 130 clivé par EcoR1. Une banque d'ADNcs a été obtenue après transformation de la souche de E.coli TG1. On a obtenu approximativement 10⁴ plaques de recombinants M13.

Pour sélectionner dans la banque d'ADNcs des clones M13 recombinants contenant l'ADNc de HIV-2, la technique d'hybridation sur plaque a été utilisée. L'ADN des plaques M13 a été transféré sur des filtres de nitrocellulose, et hybridé à des sondes de HIV-1 subgénomiques dérivées du clone "lambda J19" d'un virus LAV (ou HIV) décrit dans la demande de brevet européen. Cette sonde comprenait un insérat constitué par une partie ayant une longueur approximative de 1500 paires de bases (pb) de l'ADN de HIV-1. Cet insérat était délimité par deux sites de restriction HindIII respectivement à l'intérieur du cadre de lecture ouverte du gène "env" et dans le segment R de l'extrémité 3' LTR de HIV-1. Cette sonde contenait l'extrémité 3' du gène env, la totalité du gène F, le segment U3 et une partie du segment R de LTR, d'une longueur approximative de 1500 paires de bases (pb).

La sonde contenant l'insérat HindIII de 1,5kb a été marquée avec du ³²P dCTP et dTTP (3000 Ci x 10⁻³ mole) par incubation de la sonde en présence d'initiateurs et d'ADN polymerase I de Klenow, pendant 4 heures à 15°C (en utilisant un kit de AMERSHAM). Les essais d'hybridation des clones d'ADNc de la banque ont été conduits pendant toute la nuit dans des conditions de faible stringence, au sein d'une solution d'un milieu d'hybridation contenant 5X SSC, 5X Denhart, 25% de formamide, 100 µg/ml d'ADN de sperme de saumon dénaturé et la sonde marquée (2 x 10⁷ cpm avec une spécificité de 10⁹ cpm/ug) à 37°C. Les filtres ont été soumis à trois étapes de lavage, successivement en présence des trois solutions dont les compositions sont indiquées ci-après: Lavage n° 1:5XSSC, 0.1% SDS,à 25°C pendant 4x15 minutes Lavage n° 2:2XSSC, 0.1% SDS,à 42°C pendant 2x30 minutes Lavage n° 3:0.1XSSC,0.1% SDS, à 65°C pendant 2x30minutes Chaque lavage est suivi par une autoradiographie des filtres.

Plusieurs clones positifs ont été détectés après le lavage n° 1 et l'ont encore été après le lavage n° 2. Cependant, tous les signaux disparaissaient après le lavage n° 3. Ceci indique que les clones positifs n'avaient avec le génome de HIV-1 qu'une faible parenté, néanmoins suffisante pour effectuer la susdite sélection. Les clones positifs ont été repiqués, redéposés sur des plaques et de nouveau hybridés avec la même sonde dans les conditions de stringence correspondant au lavage n° 1. La majorité d'entre-eux étaient encore positifs.

Les clones ont aussi été sélectionnés en mettant en oeuvre une sonde d'ADN humain total dans des conditions de stringence moyenne et par hybridation dans 5X SSC, 5X Denhart, et 40% de formamide, suivie d'un lavage dans 1X SSC, 0.1% SDS, à 50°C. Aucun des clones préalablement positifs n'a été détecté, par conséquent ne correspondait pas à l'ADN répétitif spécifique ou à l'ADNc de l'ARN ribosomal.

Les clones recombinants M13 positifs ont été cultivés dans un milieu liquide et caractérisés :

### (1) Taille de leur insertion :

Un ADN type simple brin de M13 a été obtenu à partir de chaque clone individuel, et la synthèse du second brin a été conduite avec une séquence initiatrice 17-mer M13 et l'enzyme de Klenow. Les insérats ont été excisés à l'aide d'EcoR1 (BOEHRINGER) et analysés par électrophorèse sur gel d'agarose. La plupart des inserats contenaient de 200 à 600 et 200 pb, à l'exception du clone dénommé E2.1, qui avait approximativement une longueur de 2 Kpb.

### (2) Analyse de la séquence nucléotidique :

Plusieurs clones ont été partiellement séquencés, en utilisant la méthode dideoxy de Sanger et al. décrite dans Proc. Natl. Acad. Sci. 74:5463-7 (1977).
Différents clones indépendants contenaient des séquences nucléotidiques similaires, à l'exception des chaînes poly-A à leurs extrémités 3' dont les longueurs étaient différentes. Ces résultats démontrent que ces clones ADNc étaient dérivés de la matrice d'ARN. L'analyse de séquence détaillée de ces clones ADNc, comprenant l'extrémité 3' du génome de HIV-2, a montré une parenté limitée avec HIV-1.

### (3) Hybridation avec l'ARN et l'ADN génomique de HIV-2.

(a) Obtention de l'ARN génomique de HIV-2 : Un surnageant infecté a été centrifugé (50.000 rotations, 30 minutes). Le culot du dépôt a été resuspendu dans 10mM Tris pH 7.5, 1mM EDTA, 0.1% SDS. L'un des clones d'insertion F1.1 a été marqué et utilisé comme sonde pour l'hybridation avec l'ARN génomique de différents isolats viraux, conformément à la technique de "dot-blot".

La technique "dot-blot" comprenait les étapes suivantes :
(i) déposer l'échantillon (lysat HIV-2) en taches sur une membrane de nitrocellulose préalablement trempée dans 20X SSC (3M NaCl, 0.3M de citrate de sodium) et séchée à l'air, (ii) faire cuire la membrane pendant 2 heures à 80°C., et (iii) effectuer l'hybridation.

Cette hybridation a été conduite dans des conditions de haute stringence (5X SSC, 5X Denhart, 50% formamide à 42°C). Elle a été suivie d'un lavage dans 0.1X SSC, 0.1% SDS à 65°C. Dans ces conditions, la sonde hybride fortement aux tâches provenant de deux isolats indépendants de HIV-2, incluant le LAV-II ROD, dont l'ADNc cloné était issu. Un faible signal d'hybridation a été détecté avec la tache formée par STLV-IIImac (Virus T-Lymphotropique de Singe (également connu comme "SIV") de type III, macaque), et aucune hybridation n'a été détectée avec les isolats de HIV-1.

Les expériences de "Southern Blot", mettant en jeu le clone E-2.1 contenant l'inserat de 2Kb en tant que sonde marquée au ³²P, ne manifestèrent aucune hybridation avec des ADNs de cellules non infectées, mais détectaient des bandes dans des cellules détachées infectées par HIV-2 dans des conditions de forte stringence. HIV-2 montre un polymorphisme à des niveaux de sa carte de restriction équivalents à ceux des cartes de restriction de HIV-1. Avec de l'ADN cellulaire complet de cellules infectées, deux sortes de signaux sont détectés par la méthode de "Southern Blot" : (1) dans des fractions d'ADN ayant des poids moléculaires PM d'environ 20kb et plus dans le cas de formes intégrées du virus, et (2) dans les fractions de PM plus faibles (9,10kb), dans le cas de virus non intégré au génome.

Ces caractéristiques sont hautement spécifiques d'un rétrovirus.

Certaines expériences effectuées avec STLV-III (SIV-3) de cellules infectées ont permis de constater que le rétrovirus simien est relativement distant de HIV-2 (le signal est uniquement détecté dans des conditions de faible stringence). Ces expériences montrent que les susdites sondes permettent la détection spécifique de HIV-2.

### (4) Sous-clonage de l'ADNc de HIV-2 dans un vecteur plasmide bactérien :

Le clone M13 positif, E 2-1 a été sélectionné et sous-cloné dans un vecteur plasmide. L'ADN du phage M13 (TG 130) recombinant E-2 a été purifié sous forme d'un ADN simple brin (M-13-ROD-E2) contenant l'inserat de 2 kb, contenant la portion 3' du génome de HIV-2 (obtenu à partir de HIV-2 ROD). Cet inserat a été transféré dans le plasmide pSP65, décrit par Melton, D.A. dans 357 Nucleic Acid Res. 12:035-7056 (1984).

Une seconde chaîne a été construite in vitro en présence de la séquence d'initiateur 17 mer (AMERSHAM), des quatre nucléotides A,C,T,G, et de l'ADN polymerase I (Klenow). L'inserat EcoR1 a été excisé par digestion EcoR1 et purifiée sur gel d'agarose, puis ligué à pSP65, lui-même préalablement digéré avec EcoR1. Le mélange de ligation a été utilisé pour transformer la souche E.coli DH1 et des recombinants ont été sélectionnés grâce à leur capacité de résistance à l'ampicilline. Les recombinants identifiés ont été cultivés sur milieu LB (Luria medium) contenant 50 µg/ml d'ampicilline. Ces plasmides recombinants ont été purifiés et contrôlés quant à la présence du fragment inséré correct.

L'un des clones obtenus désigné par la référence pSPE2, a été déposé à la CNCM à PARIS, France, sous le n° d'accès I-595 le 5 Septembre 1986.

Les insérats dérivés des ADNc de HIV-2 et qui se trouvaient insérés dans la sonde susdite contenaient la séquence de nucléotides qui a été définie plus haut, en rapport avec une partie de E2.

### EXEMPLE II

### Clonage d'un ADNc complémentaire à l'ADN complémentaire à l'ARN génomique de virions de HIV-2.

Des virions HIV-2 ont été purifiés à partir de 5 litres d'un surnageant d'une culture d'une lignée CEM infectée avec un isolat ROD. Un premier brin d'ADNc a été synthétisé au contact de virus purifié sédimenté, en présence d'un initiateur oligo (dt) et en mettant en oeuvre une réaction endogène activée par un détergent, selon la technique décrite par Alizon et al, Nature 312 : 757-760 (1984). Les hybrides ARN-ADNc ont été purifiés par une extraction par un mélange phénolchloroforme et par une précipitation à l'éthanol. Le second brin d'ADNc a été produit en présence d'ADN polymérase-I/RNAse H selon la méthode décrite par Gubler et Hoffman. La description de cet article est considérée comme faisant partie de la présente description.

L'ADNc double brin a été muni d'extrémités franches en présence d'ADN polymérase T4 en utilisant les constituants d'un kit de synthèse d'ADNc commercialisé par AMERSHAM.

Des adaptateurs (linker) Eco R1 commercialisés par PHARMACIA, ont été fixés à l'extrémité de l'ADNc; l'ADNc ainsi modifié a été inséré après digestion en présence de Eco R1 dans un vecteur de phage déphosphorilé M13tg130 lui-même digéré par Eco R1, également commercialisé par AMERSHAM. Une bande d'ADNc a été obtenu après transformation de la souche E.coli TG1. Des plaques de recombinants (10⁴) ont été triés in situ sur des filtres permettant des répliques par hybridation avec le clone J19 contenant le fragment Hind III de 1,5kb déjà mentioné plus haut, issu de HIV-1.

Les filtres ont été préhybridés en présence d'un milieu contenant 5 x SSC, 5 x solution DENHARDT, formamide à 25%, de l'ADN dénaturé de sperme de saumon (100 microgrammes/mml) à 37°C, pendant 4 heures, puis hybridé pendant 16 heures dans le même tampon (Tm -42°C) en présence d'un supplément de sonde marquée (4 x 10⁷cbm), pour fournir une solution finale de tampon d'hybridation contenant 10⁶cpm/ml). Le lavage a été fait avec une solution 5 x SSC, 0,1% SDS à 25°C pendant 2 heures (étant entendu que 20 x SSC correspond à une solution NaCl 3M et de citrate de sodium 0,3M. Les plaques répondant positivement ont été purifiées et les ADNs mono-brins de M13 ont été préparés et leurs extrémités ont été séquencées selon la méthode de Sanger et al.

### Hybridation d'un ADN de cellules infectées par HIV-1 et HIV-2 et d'ARNs de HIV-1, HIV-2 et de virions SIV respectivement avec une sonde dérivée d'un ADNc cloné de HIV-2.

Les ADNs ont été extraits de cellules CEM infectées produisant de façon continue respectivement HIV-1 et HIV-2. Des échantillons d'ADN de ces deux rétrovirus, digérés pour certains par 20 ug de PstI, non digérés pour d'autres ont été soumis à une electrophorèse sur gel d'agarose à 0,8% et transféré par la méthode "Southern^{"} sur membrane de nylon. Des petits volumes de surnageant infecté, repris dans un tampon NTE contenant 0,1% de SDS ayant les mêmes activités de transcriptase inverse ont été déposés sur nitrocellulose, qui avait été trempé auparavant dans une solution 2 x SSC.

Une préhybridation a été faite dans une solution contenant 50% de formamide, 5 x SSC, 5 x Denhardt, et 100 mg/ml d'ADN de sperme de saumon dénaturé pendant 4 heures A 42°C. Une hybridation a été conduite dans le même tampon, auquel avait été ajouté 10% de sulphate de dextran, et 10⁶ cpm/ml. d'insérat marqué E2 (activité spécifique 10⁹ cpm/ug) pendant 16 heures à 42°C. Deux lavages ont ensuite été faits avec une solution 0.1 x SSC, 0.1% SDS pendant 30 mn chacun. Après l'exposition pendant 16 heures à un écran d'intensification la tache Southern est déshybridée dans 0.4 N NaOH, neutralisée et réhybridée dans les mêmes conditions à la sonde HIV-1 marquée avec 10⁹ cpm/ug.

### EXEMPLE III

### Clonage dans le Phage Lamdba de l'ADN complet du provirus de HIV-2.

L'ADN des cellules CEM infectées par HIV-IIROD (figure 2, bandes a et c) est partiellement digéré avec Sau3AI. La fraction 9-15 kb a été sélectionnée sur gradient de sucrose a 5-40% et liguée aux bras BamHI du vecteur lambda L47.1. Les plaques (2 x 10⁶) obtenues après empaquetage in vitro et dépôt sur souche E.coli LA 101 ont été triées in situ par hybridation avec l'insérat de l'ADNc cloné E2. Approximativement, 10 clones positifs ont été purifiés sur plaque et propagés sur E.coli C600 recBC. Les clones lamdba ROD 4, ROD 27 et ROD 35 ont été amplifiés et leurs ADN caractérisés par établissement de leurs cartes de restriction et par hybridation par la méthode de "Southern" avec le clone ADNc de HIV-II dans des conditions stringentes et avec les sondes gag-pol de HIV-1 dans des conditions non stringentes.

La figure 8 présente de façon schématique les structures de 3 des phages recombinants obtenus ROD 4, ROD 27 et ROD 35.

Les parties rectangulaires allongées de ces schémas correspondent à des séquences pro-virales issues des ADNs des cellules CEM initialement infectées, les parties blanches correspondant à des séquences rétro-virales, les parties grisées à des parties des ADNs cellulaires et les parties en noir au LTR dans lesdites fréquences virales.

Les traits fins désignés par les lettres L et R correspondent aux bras issus du vecteur de phage Lambda L47.1 ayant servi au clonage.

Certains des sites des restrictions ont également été indiqués : il s'agit plus particulièrement des sites suivants :
B : BamHI ; E : EcoRI ; H: HindIII ; K: KpnI ; Ps: PstI;Pv : PvuII ; S: SacI ; X: XbaI.

Ces séquences virales ont des parties en commun avec la séquence E2. Les positions relatives de ces parties déterminées par hybridation avec E2, apparaissent également dans les figures.

ROD 4 est dérivé d'un ADN viral circulaire. ROD 27 et ROD 35 sont dérivés de provirus intégrés dans une structure ADN cellulaire.

Enfin, on voit apparaître dans ces figures les insérats sous-clonés dans les conditions qui ont été indiquées plus haut et leurs positions relatives vis-à-vis des séquences correspondantes de ROD 4, ROD 27 et ROD 35.

Il s'agit plus particulièrement des insérats des plasmides pROD 27-5', pROD 35-3', pROD 4.6, pROD 4.8 et pROD 4.7.

La figure 9 est une représentation des intensités relatives des taches d'hybridation qui ont été réalisées entre ROD-4 et des sous-fragments 1 à 11 respectivement issus des différentes parties d'un ADN à chaîne unique issu de sous-clone de M13 contenant un acide nucléique dérivé du génome entier de LAV. Les positions relatives de ces divers fragments vis-à-vis du génome entier de LAV (déterminé par séquençage) sont indiquées dans la partie inférieure de la figure. Le point 12 correspond à une tache témoin réalisé en mettant en oeuvre un ADN témoin du phage Lambda.

Les essais d'hybridation dans la méthode de transfert par tache (dot-blot) ont été réalisés dans les conditions de faible stringence de l'exemple II. En utilisant à titre de sonde, le recombinant Lambda ROD 4 contenant l'ADNc total de DIV 2. Les lavages ont ensuite été faits successivement dans les conditions suivantes :
2 x SSC, 0.1% SDS à 25°C. (Tm -42°C), 1 x SSC, 0.1 % SDS à 60°C. (Tm -20°C), et 0.1 x SSC, 0.1% SDS à 60°C. (Tm -3°C).

Les taches représentées ont été obtenues après exposition radiographique pendant 1 nuit.

### EXEMPLE IV

### Test de diagnostic in vitro de la présence du virus HIV-2 dans un milieu biologique.

### MATERIEL ET METHODES

### PATIENTS

Les patients infectés par HIV-2 ont été sélectionnés parmi des personnes admises à l'hôpital Egas Moniz de LISBONNE pour hospitalisation ou pour consultation, entre septembre 1985 et septembre 1986.

Pour réaliser cette sélection, tous les individus d'origine africaine ou ayant séjourné en Afrique ont subi un test sérologique à la fois pour rechercher la présence d'anticorps contre HIV-I (immuno-fluorescence-IFA- et/ou ELISA) et contre HIV-2 (IFA). Seuls les patients pour lesquels on a prouvé sérologiquement l'infection avec HIV-2 ont été retenus dans cette étude.

### Isolation du virus.

Chez 12 patients, l'isolation de HIV a été conduite comme on le rappelle brièvement. Les lymphocytes périphériques sanguins des patients (PBL) ont été stimulés avec PHA, cultivés en coculture avec des PBLs normaux humains stimulés par PHA et maintenus en présence d'interleukine-2 (IL-2). Les cultures ont été contrôlées afin de repérer la présence éventuelle d'un effet cytopathogénique (CPE) et afin de mesurer l'activité de transcriptase inverse (RT) dans le surnageant.

### Test d'immunofluorescence (IFA)

Des plaques IFA ont été préparées comme suit : des cellules MOLT-4 infectées avec HIV-2 ont été lavées 2 fois dans PBS et étalées sur des lames de verre IFA (10⁴ cellules par puits), puis séchées à l'air et fixées par de l'acétone à froid. Pour le test IFA, ces cellules ont été soumises à une réaction, le sérum de test étant dilué à 1/10, pendant 45' à 37°C, ont été lavées, séchées et soumises à une réaction avec de la fluorescéine conjuguée à des IgG, A, M anti-humaines de chèvre (dilué à 1/100) pendant 30 minutes à 37°C. Après le lavage, les cellules ont été colorées avec du bleu Evans, à 0,006%, montées dans un mélange de 90% de glycérol, 10% de PBS et examinées au microscope à fluorescence.

### ELISA

Certains sérums de patients ont été examinés pour déterminer la présence d'anticorps anti-HIV-1 en utilisant les tests sérologiques disponibles dans le commerce sous la dénomination ELAVIA (Pasteur Diagnostics) ou ABBOTT.

### Test de radio-immunoprécipitation (RIPA)

Des cellules CEM infectées avec HIV-1 ou HIV-2 ont été cultivées en présence de ³⁵S cystéine (200 microCi/ml) pendant 16 heures. Le surnageant a été recueilli, les particules virales ont été centrifugées et lysées sur un tampon RIPA (Tris-HC1 50mM pH 7.5, NaC1 150mM, EDTA 1mM, 1% Triton X100, Sodium deoxycholate 0.1%, SDS 0.1%). Pour chaque réaction, 50 microlitres de lysat dilué correspondant à 10⁵ cpm ont été mis en réaction avec 5 microlitres d'un sérum test pendant 18 heures à 4°C. Des complexes immuns ont été liés grâce à une protéine A fixée sur du Sépharose PHARMACIA lavés, et élués pendant une ébullition de 2 minutes. Les antigènes élués ont été analysés par électrophorèse sur gel SDS-polyacrylamide et par autographie.

### Hybridation Dot-blot

Des virus isolés dans des PBLs de patients ont été centrifugés et lysés dans Tris-HCl 10mM pH 7.5, NaCl 10mM, EDTA 1mM, SDS 0.5%. Un microlitre de chaque lysat, correspondant à une activité RT de 50.000 cpm a été déposé sur un support de nitrocellulose. Une hybridation et un lavage ont été réalisés dans des conditions de forte stringence: hybridation dans 6X SSC, 5X Denhart, 50% de formamide, pendant 18 heures à 42°C ; et lavage dans 0.1X SSC, 0.1% SDS à 65°C. On a utilisé des sondes HIV-1 et HIV-2, marquées au 32p (activité spécifique de 10⁸ cpm/microgramme). La sonde HIV-1 correspond au génome complet de l'isolat LAV_{BR U}, et la sonde HIV-2 dérive d'un ADN cloné de 2 kilobases de l'isolat LAV-2_{R OD}.

### RESULTATS

### Population des patients

30 patients chez lesquels on décelait une infection par HIV-2 par mise en évidence sérologique ou virale, ont été étudiés. Parmi ces 30 patients on comptait 12 hommes et 18 femmes. La moyenne d'âge était de 35 ans pour un intervalle de 11 à 55 ans. Tous les patients excepté 1, étaient restés plusieurs années en Afrique de l'Ouest : 26 étaient nés et vivaient en Guinée-Bissau et 2 étaient originaires des Iles du Cap-Vert. 1 patient était un garçon de 11 ans de l'Angola, qui avait vécu dans les Iles du Cap-Vert pendant plusieurs années. Le seul européen de la population étudiée était un Portugais de 40 ans qui avait vécu pendant 8 ans au Zaïre, mais démentait tout séjour en Afrique de l'Ouest.

### Présentation clinique

Parmi les 30 patients, 17 avaient un SIDA, conformément aux critères reconnus CDC. Le symptôme majeur chez ces patients était la diarrhée chronique, accompagnée dans la plupart des cas d'une perte de poids de plus de 10 kg. en 1 an. Chez 10 patients la diarrhée était associée avec la présence d'une infection intestinale soudaine ; pour 7 cas, l'agent pathogène était le seul Isospora belli, 1 patient était atteint uniquement par Cryptosporidium et 2 avaient à la fois les 2 agents pathogènes. Dans 3 cas, aucun agent pathogène intestinal inattendu n'a été révélé. Parmi les 17 cas de malades atteints de SIDA, on a diagnostiqué chez 8 des candidoses oesophagiennes. 6 patients atteints du SIDA présentaient des symptômes respiratoires. Une tuberculose pulmonaire a été diagnostiquée chez 2 d'entre eux et une microbactérie différente non identifiée a été décelée chez l'un d'entre eux.
2 patients souffraient d'une aspergilose pulmonaire à la suite d'une tuberculose. 2 autres patients atteints du SIDA présentaient des phases récurrentes de pneumonie sans identification de l'agent pathogène, et 1 patient avait une pneumonie du type pneumocystis carinii, qui fut diagnostiquée uniquement post-mortem. Quatre des 17 patients atteints du SIDA avaient un sarcome de Kaposis: dans 3 cas il est apparu comme limité à la peau et chez un autre patient un examen post-mortem a révélé des lésions disséminées au niveau viscéral. Des désordres du système nerveux central sont apparus chez 2 patients atteints du SIDA : 1 avait un lymphome cérébral, et l'autre avait une encéphalite subaiguë de cause inconnue.
4 patients présentaient les symptômes du complexe lié au SIDA (ARC) :
2 avaient des lymphadénopathies diffuses avec fièvre persistante, 1 avait une diarrhée chronique avec perte de poids, et 1 avait des phases récurrentes de bronchopneumonie et des lymphadénopathies multiples.

Parmi les 9 patients restants, 6 n'avaient aucun symptôme rattachable à l'infection par HIV, 1 était atteint uniquement d'une tuberculose pulmonaire , 1 avait uniquement des lymphadénopathies diffuses persistantes et 1 présentait une syphillis neurologique. Pendant la période d'étude de 12 mois, 7 patients sont morts, tous présentant un SIDA.

### Etude sérologique

Chaque patient fait l'objet d'au moins un test sérologique pour rechercher la présence d'anticorps contre à la fois HIV-1 et HIV-2. Tous les sérums des patients ont été testés par test IFA afin de repérer les anticorps contre HIV-2 et tous se sont révélés positifs. Parmi eux, 21 furent aussi testés pour les anticorps contre HIV-2 par un test RIPA : tous ont précipité de façon claire la glycoprotéine de haut poids moléculaire de l'enveloppe du virus, désignée par gp140, et 16 d'entre eux ont réagi également avec la protéine majeure du noyau p26, alors qu'une seulement réagit avec une autre protéine virale du noyau, désignée par p16.

Dans ces sérums on a recherché la présence d'anticorps présentant des réactions croisées avec HIV-I en utilisant les différents tests. Un test IFA a été utilisé pour 24 sérums : 12 sont négatifs, 10 sont faiblement réactifs, et 2 positifs. Dans un test ELISA, 21 sérums ont été testés : 16 étaient négatifs, et 5 étaient positifs. Finalement, on a recherché dans 11 sérums la présence d'anticorps contre les protéines de HIV-1 par la méthode RIPA.
3 d'entre eux n'ont absolument pas réagi, et ceci avec aucune protéine virale, 2 seulement ont précipité avec la protéine p34, issue du gène pol, et 5 ont réagi avec la protéine p25, protéine majeure du noyau. 2 sérums ont réagi seulement faiblement avec la glyco-protéine gp110 de l'enveloppe du virus HIV-1. Ces deux sérums ainsi que tous les sérums positifs lors des tests IFA ou ELISA concernant les anticorps anti HIV-1 ont réagi fortement avec la gp140 de HIV-2 dans un test RIPA, indiquant une infection avec HIV-2 plutôt qu'avec HIV-1. Seulement 1 patient, qui n'est pas inclu dans la population étudiée, a été reconnu sérologiquement comme étant infecté par HIV-1 et non par HIV-2. Ce patient était une femme de 21 ans de l'Afrique Centrale (Ile Sao Tome) présentant un SIDA.

### Isolation du virus

L'isolation des virus dans les lymphocytes périphériques du sang a été pratiquée sur 12 patients. Un HIV a été isolé chez 11 d'entre deux, grâce à la présence d'un effet cytopathogénique, et à la mesure d'un pic des particules associées à l'activité de transcriptase inverse dans les surnageants de culture.

Ces 11 isolats ont été identifiés comme étant des isolats de HIV-2 en utilisant la technique d'hybridation "dot-blot". Les taches virales obtenues à partir de 10 isolats ont montré une hybridation forte dans les conditions stringentes d'hybridation et de lavage avec une sonde HIV-2 dérivée d'un ADNc cloné de HIV-2, alors qu'aucune d'entre-elles n'hybridait avec les sondes HIV-1 dans les mêmes conditions. Un isolat hybridait seulement faiblement avec une sonde HIV-2, mais n'hybridait pas avec une sonde HIV-1.

### Evaluation immunologique

13 patients ont été étudiés pour évaluer le nombre de leurs lymphocytes circulant identifiés comme étant des cellules T auxiliaires (helper) (CD4+) et le rapport de : cellules T auxiliaires/cellules T suppresseur. Parmi ces patients, 11 avaient un SIDA : leurs nombres de cellules T auxiliaires était de 243 à 300 par microlitre, et leur rapport cellules T auxiliaires/ cellules T suppresseur moyen était de 0,25 à 0,15. 1 patient présentait des signes cliniques de ARC, avait un nombre de lymphocytes T helper de 240/microlitre et un ratio de 0.18. Un autre patient, présentant une syphillis neurologique et aucun symptôme lié de façon évidente à HIV, avait un nombre de lymphocytes T helper de 690 par microlitre et un ratio de 0.82.

### DISCUSSION

Dans cette étude, l'infection par HIV-2 a été mise en évidence chez 30 patients africains d'Afrique de l'Ouest présentant soit un SIDA, soit des symptômes du complexe ARC ou n'ayant aucun signe clinique rattaché au SIDA. Les résultats permettent néanmoins de conclure du fait que HIV-2 doit être considéré comme un agent éthiologique majeur du SIDA chez les patients d'Afrique de l'Ouest. Les résultats sérologiques et viraux qui ont été observés indiquent que l'infection par HIV-2 n'était pas souvent associée avec une infection par HIV-1 parmi ces patients.

En dépit de différences antigéniques et génétiques importantes, HIV-1 et HIV-2 présentent un tropisme similaire pour les lymphocytes T CD4+, ont des effets cytopatogènes similaires, une morphologie similaire, et partagent des épitopes d'immunoréaction communs pour certaines de leurs protéines constitutives.

Dans la mesure où tous les patients d'Afrique de l'Ouest, infectés avec HIV et étudiés ici ont été reconnus comme étant infectés par HIV-2 alors qu'aucun n'était infecté par HIV-1, le nouveau virus HIV-2 pourrait être la cause majeure du SIDA en Afrique de l'Ouest.

Les symptômes du SIDA liés à HIV-2 ne sont pas différents de ceux du SIDA liés à HIV-1 en Afrique Centrale : Le symptôme le plus commun consiste dans des diarrhées chroniques, avec perte importante de poids, troubles dûs la plupart du temps à Isospora belli et/ou Cryptosporidium. La fréquence d'autres infections soudaines, telles que des candidoses, des microbactéries (incluant M. tuberculosis) et des toxoplamoses comparable à ce que l'on reconnaissait chez les Africains atteints d'un SIDA lié à HIV-1. La pneumonie du type Pneumocystis carinii, considérée comme une complication très commune chez les malades atteints du SIDA aux USA et en Europe, a été décelée seulement une fois lors de notre étude, et des méningites de type cryptococcal n'ont pas été détectées.

Néanmoins, les anomalies immunologiques décelées parmi des patients atteints de SIDA lié à une infection par HIV-2 sont identiques à celles décrites lors de SIDA lié à HIV-1.

Parmi les 30 patients qui possédaient dans leur sérum des anticorps réagissant avec les antigènes HIV-2, seuls 7 d'entre-eux avaient des anticorps spécifiques contre HIV-1 détectables par des tests IFA ou ELISA. Lors d'un test RIPA, on observe que ces 7 patients présentent des anticorps réagissant contre les protéines majeures du noyau p25 (HIV-1) ou p26 (HIV-2), qui ont en commun des épitopes fortement immunoréactifs. 5 patients ne présentent pas de tels anticorps : ces 5 patients ont des réponses négatives à des tests ELISA vis-à-vis de HIV-1. 2 d'entre eux ont des réponses négatives à des tests IFA et 3 d'entre eux ont des réponses très faibles. Cependant, bien que certains d'entre eux n'aient pas été totalement étudiés, 9 patients ont été observés qui possédaient dans leur sérum des anticorps de la protéine virale p26 du noyau de HIV-2 et qui présentaient une réaction faible ou une réaction négative à des tests IFA ou ELISA spécifiques de HIV-1. Ces résultats font ressortir l'importance de l'incorporation d'antigène HIV-2 dans des tests sérologiques HIV utilisés on Afrique et peut-être dans d'autres endroits.

Un rétrovirus a été isolé des lymphocytes périphériques de 11 patients. Dans tous les cas la croissance virale a été obtenue en 2 semaines, caractérisée par la présence d'une activité de transcriptase inverse dans le surnageant de culture et par un effet cytopatogénique. Cependant, cet effet cytopatogénique est apparu d'importance variable pour les différents isolats : certains isolats présentaient un nombre important de syncytia de grande taille accompagné d'une lyse cellulaire importante, alors que d'autres présentaient seulement peu de syncytia de taille limitée, et affectant rarement la viabilité de la culture.

Tous les ARNs sauf celui d'un unique isolat hybrident avec une sonde dérivée d'un ADNc cloné de HIV-2 représentant la terminaison 3' du génome, dans des conditions de fortes stringences. Aucun n'hybride avec une sonde HIV-1 dans les mêmes conditions. Ceci démontre que les isolats infectant ces patients appartiennent au même type de virus. Un isalat seulement hybride mais rarement avec une sonde HIV-2 à la fois dans des conditions de forte et de faible stringence, et n'hybride pas avec HIV-1. Ce virus a cependant été isolé chez un patient possédant dans son sérum des anticorps capables de réagir avec tous les antigènes de HIV-2 lors d'un test RIPA.

D'une façon générale, la demande de brevet décrit, outre le virus HIV-2 et ses variants, tout virus équivalent infectieux pour l'homme présentant des caractéristiques immunologiques propres au HIV-2. D'une façon générale, la demande de brevet se réfère à tout virus qui, outre les propriétés que possède les virus HIV-2 déposés à la CNCM, présente encore les caractéristiques suivantes.

La cible préférentielle du rétrovirus HIV-2 est constituée par les cellules Leu 3 (ou lymphocytes T4) humaines et pour des cellules "immortalisées" dérivées de ces lymphocytes T4, par exemple les cellules des lignées HUT-78 considérées dans le cadre de cette demande. En d'autres termes, il a un tropisme spécifique pour ces cellules. Il peut étre cultivé dans des lignées permanentes du type HUT, CEM, MOLT ou analogue exprimant la protéine T4. Il n'est pas infectieux pour les lymphocytes T8. Il est cytoxique pour les lymphocytes T4 humains. Le caractère cytopathique des virus HIV-2 à l'égard des lymphocytes T4 se manifeste notamment par l'apparition de cellules multinuclées. Il a une activité de transcriptase inverse nécessitant la présence d'ions Mg²⁺ et présentant une forte affinité pour le poly(adénylate-oligodéoxythymidylase) (poly(A)-oligo (dT) 12-18). Il a une densité d'environ 1,16 dans un gradient de sucrose. Il a un diamètre moyen de 140 nanomètres et un noyau ayant un diamètre moyen de 41 nanomètres. Les lysats de ce virus contiennent une protéine p26 qui ne croise pas immunologiquement avec la protéine p24 du virus HTLV-I ou du virus HTLV-II. Ces protéines p26 présentent donc un poids moléculaire un peu plus élevée (d'environ 1000) que les p25 correspondantes des HIV-1 et un peu moins élevéed (de nouveau de l'ordre d'environ 1000) que les p27 correspondantes des SIV. Les lysats de HIV-2 contiennent en outre une protéine p16 qui n'est pas reconnue immunologiquement par la protéine p19 de HTLV-I ou de HTLV-II dans des essais de radioimmunoprécipitation RIPA (abréviation de l'expression anglaise "radioimmunoprecipitation assay"). Ils contiennent en outre une glycoprotéine d'enveloppe ayant un poids moléculaire de l'ordre de 130.000-140.000 qui ne croise pas immunologiquement avec la gp110 du HIV-1, mais qui, par contre, croise immunologiquement avec la glycoprotéine d'enveloppe gp140 de STLV-III. Ces lysats contiennent encore des protéines ou glycoprotéines, marquables par la ³⁵S-cystéine, ayant des poids moléculaires respectivement de l'ordre de 36.000 et 42.000-45.000. L'ARN génomique de HIV-2 n'hybride pas avec l'ARN génomique de HIV-1 dans des conditions strinqentes. Dans des conditions non stringentes, il n'hybride, ni avec la séquence nucléotidique dérivée de HIV-1 et contenant le gene env et le LTR qui le jouxte. En particulier il n'hybride pas avec la séquence de nucléotides 5290-9130 de HIV-1, ni avec des séquences de la région pol du génome de HIV-1, en particulier avec la séquence de nucléotides 2170-2240. Dans des conditions non stringentes, il hybride faiblement avec des séquences de nucléotides de la région de HIV-1, notamment les séquences de nucléotides 990-1070 et 990-1260.

Il est à remarquer que tout rétrovirus infectieux pour l'homme susceptible d'induire un SIDA ayant les propriétés essentielles susdites et dont l'ARN génomique est susceptible de s'hybrider dans les conditions stringentes avec ceux des souches virales de HIV-2 déposées à la C.N.C.M. (ou avec un cADN ou fragment de cADN dérivés de ces ARNs génomiques doit être considéré comme un équivalent de HIV-2.

La présente demande fait encore référence à chacun des antigènes, notamment protéines et glycoprotéines à l'état purifié, tels qu'ils peuvent être obtenus à partir de HIV-2. On parle de protéines ou glycoprotéines "purifiées" lorsque ces protéines ou glycoprotéines ne conduisent respectivement qu'à des bandes uniques en électrophorèse sur gel de polyacrylamide, notamment dans les conditions expérimentales qui ont été indiquées plus haut. Tout procédé approprié de séparation et/ou de purification pour obtenir chacune d'entre elles peut être utilisé. On mentionnera à titre d'exemple de technique pouvant être mise en oeuvre celle décrite par R.C. MONTELARO et Coll., J. of Virology, juin 1982, pp. 1029-1038.

D'une façon générale, la demande décrit tous antigènes, notamment protéines, glycoprotéines ou polypeptides issus d'un HIV-2 et ayant des propriétés immunologiques équivalentes à ceux ou celles du HIV-2. Deux antigènes sont dits "équivalents" dans le cadre de cet exposé, dès lors qu'ils sont reconnus par les mêmes anticorps, notamment d'anticorps isolables à partir d'un sérum obtenu à partir d'un patient ayant fait une infection avec un HIV-2, ou dès lors qu'ils répondent aux conditions "d'équivalence immunologique" indiquées ci-après.

Parmi les polypeptides, protéines ou glycoprotéines équivalents, doivent être rangés des fragments des antigènes qui précèdent (ou des ppeptides reconstitués par synthèse chimique), dès lors qu'ils donnent lieu à des réactions immunologiques croisées avec les antigènes dont ils sont dérivés. En d'autres termes, l'invention concerne tout polypeptide ayant, en commun avec les susdits anti-gènes, des épitopes identiques ou semblables, susceptibles d'être reconnus par les mêmes anticorps. Font partie de ce dernier type de polypeptides les produits d'expression de séquences correspondantes des ADNs codant pour les séquences polypeptidiques correspondantes.

Le virus HIV-2 s'est avéré utilisable comme source d'antigènes pour la détection d'anticorps chez toutes personnes ayant été mises en contact avec le virus HIV-2.

D'une façon générale, toute composition utilisable pour le diagnostic de la présence dans un sérum fluide biologique, notamment de personnes ayant été mises au contact de HIV-2, d'anticorps contre l'un au moins des antigènes de HIV-2 peut être appliquée au diagnostic sélectif de la variété correspondante du SIDA, en mettant en oeuvre les techniques de diagnostic, telles que décrites dans la demande de brevet européen citée ci-dessus, si ce n'est que l'on utilise des extraits, lysats ou encore antigènes purifiés de HIV-2 au lieu de ceux de HIV-I. A cet égard, La demande fait référence à des compositions contenant l'une au moins des protéines p12, p16, p26, s'agissant des protéines internes, ou l'une au moins des glycoprotéines p36 ou gp140. On mentionnera à titre d'exemples de compositions, celles qui contiennent simultanément :
- la p26 et la gp36,
- la p26, les p36 et gp140
- les p12, p16 et p26,
- les p16, p26 et gp 140 .....

Il va de soi que ces compositions n'ont que valeur d'exemples. La demande fait aussi référence aux extraits ou lysats viraux contenant l'ensemble de ces protéines et/ou glycoprotéines ou toutes fractions dont auront au préalable été séparées une ou plusieurs des protéines ou glycoprotéines susdites.

Des compositions associant des protéines et/ou glycoprotéines d'un HIV-2, avec des protéines et/ou glycoprotéines d'un HIV-1, sont par exemple :
- soit des protéines du noyau (cors) de HIV-1 et de HIV-2, notamment les p25 d'un HIV-1 et p26 d'un HIV-2, ou encore soit la p18 d'un HIV-1 et la p16 d'un HIV-2,
- soit des glycoprotéines d'enveloppe d'un HIV-1 et des glycoprotéines d'enveloppe d'un HIV-2, notamment la gp110 de HIV-1 et la gp140 de HIV-2, ou encore la p42 de HIV-1 et la p36 ou p42-45 de HIV-2,
- soit naturellement des mélanges de protéines et/ou glycoprotéines d'un HIV-1 et de protéines et/ou glycoprotéines d'un HIV-2.

De telles compositions, utilisées pour le diagnostic, permettent par conséquent des opérations de diagnostic du SIDA ou des symptômes qui lui sont associés, qui s'étendent sur un plus large spectre des agents étiologiques responsables. Il va sans dire que l'utilisation pour les opérations de diagnostic de compositions qui ne contiennent que des protéines et/ou glycoprotéines de HIV-2 n'en reste pas moins utile pour des diagnostics plus sélectifs de la catégorie de rétrovirus qui peut être tenue pour responsable de la maladie.

La demande fait aussi référence aux ADNs ou fragments d'ADNs, plus particulièrement ADNs et fragments d'ADNs clonés obtenus à partir de l'ARN ou de cADNs dérivés de l'ARN du rétrovirus HIV-2 ou à tous ADNs équivalents, notamment tout ADN présentant des homologies de séquences avec l'ADN du HIV-2, en particulier avec les séquences codant pour les régions env et pol de la souche de HIV-2 déposée à la C.N.C.M., au moins égales à 50%, de préférence à 70%, et plus avantageusement encore à 90 % , ou tout ADN (ou ARN) équivalent capable d'hybrider avec l'ADN ou l'ARN du HIV-2 dans la technique du "spot blot" dans des conditions non stringentes telles que définies ci-dessus.

Des sérums peuvent être produits chez l'animal par inoculation à celui-ci de HIV-2. On décrit plus particulièrement les anticorps polyclonaux plus spécifiquement orientés contre chacun des antigènes, notamment protéines ou glycoprotéines du virus , ou les anticorps monoclonaux qui peuvent être produits par des techniques classiques, ces anticorps monoclonaux étant orientés respectivement plus spécifiquement contre les différentes protéines du HIV-2.

Ces anticorps polyclonaux ou monoclonaux sont utilisables dans différentes applications. On mentionnera essentiellement leur utilisation pour neutraliser les protéines correspondantes, voir inhiber l'infectivité du virus entier. Ils peuvent également être utilisés, par exemple, pour mettre en évidence des antigènes viraux dans les préparations biologiques ou pour réaliser des opérations de purification des protéines et/ou glycoprotéines correspondantes, par exemple par leur utilisation dans des colonnes de chromatographie d'affinité.

Il est entendu que, d'une façon générale, la littérature technique disponible (notamment celle dont les références bibliographiques sont rappelées dans le cadre de la présente description) en ce qui concerne le HIV-1 et le virus dénommé HTLV-III décrit des techniques qui s'appliquent dans des conditions semblables à l'isolement du virus HIV-2 ou de virus équivalents, à l'obtention à partir de ces virus de leurs différents constituants (notamment protéines, glycoprotéines, polypeptides, acides nucléiques). On peut également faire appel aux enseignements de cette littérature technique pour ce qui est de l'application des différents constituants concernés, notamment à des opérations de diagnostic des formes correspondantes de SLA ou de SIDA.

Un procédé de diagnostic in vitro du SIDA, qui comprend par exemple la mise en contact d'un sérum ou d'un autre milieu biologique provenant d'un malade faisant l'objet du diagnostic avec une composition contenant l'une au moins des protéines ou glycoprotéines du HIV-2, ou encore un extrait ou lysat du virus, et la détection de la réaction immunologique. Des exemples de telles compositions ont été indiqués plus haut.

Des méthodes préférées mettent en jeu par exemple des réactions immunoenzymatiques de type ELISA ou d'immunofluoresence. Les titrages peuvent être des mesures par immunofluorescence directe ou indirecte ou des dosages immunoenzymatiques directs ou indirects.

Ainsi la demande de brevet décrit aussi des extraits de virus (soit d'un extrait d'un ou plusieurs HIV-2 seulement, soit d'un mélange d'extraits originaires d'un ou plusieurs HIV-2, d'une part, et d'un ou plusieurs HIV-1, d'autre part), ces extraits étant marqués. On peut utiliser tout type de marqueur approprié : enzymatique, fluorescent, radioactif, etc..

De tels titrages comprennent par exemple :
- le dépôt de quantités déterminées de l'extrait ou des compositions visées conformes à la présente invention dans les puits d'une microplaque de titrage ;
- l'introduction dans ces puits de dilutions croissantes du sérum contenant essentiellement les anticorps dont la présence doit être détectée in vitro ;
- l'incubation de la microplaque ;
- le lavage soigneux de la microplaque avc un tampon approprié ;
- l'introduction dans les puits de la microplaque d'anticorps marqués spécifiques d'immunoglobulines humaines, le marquage étant réalisé par une enzyme choisie parmi celles qui sont capables d'hydrolyser un substrat de telle sorte que ce dernier subit alors une modification de son absorption des radiations, au moins dans une bande de longueurs d'ondes déterminée et
- la détection, de préférence de façon comparative par rapport à un témoin, de l'importance de l'hydrolyse du substrat en tant que mesure des risques potentiels ou de la présence effective de la maladie.

Le diagnostic décrit ci-dessus peut faire appel à des "kits", lesquels comprennent :
- un extrait ou une fraction plus purifiée des types de virus indiqués ci-dessus, cet extrait ou fraction étant marqué, par exemple de façon radioactive, enzymatique ou immunofluorescence ;
- des anti-immunoglobulines humaines ou une protéine A (fixée de façon avantageuse sur un support insoluble dans l'eau, tel que des billes d'agarose) ;
- un extrait de lymphocytes obtenus à partir d'une personne en bonne santé ;
- des tampons et, le cas échéant, des substrats pour la visualisation du marquage.

Le diagnostic du virus HIV-2 ou d'un variant grâce à la mise en oeuvre des sondes décrites précédemment par un procédé faisant appel à différentes étapes rappelées ci-après, peut mettre en oeuvre des étapes spécifiquement prévues pour faire apparaître les propriétés caractéristiques du virus HIV-2.

Les cADNs ou leurs fragments (ou recombinants les contenant) sont utilisables en tant que sondes, pour le diagnostic de la présence ou non de virus HIV-2 dans des échantillons de sérums ou d'autres liquides ou tissus biologiques obtenus à partir de patients suspectés d'être porteurs du virus HIV-2. Ces sondes sont de préférence marquées également (marqueurs radio-actifs, enzymatiques, fluorescents, etc.). Des sondes particulièrement interessantes pour la mise on oeuvre du procédé de diagnostic du virus HIV-2 ou d'un variant de HIV-2 peuvent être caractérisées on ce qu'elles comprennent la totalité ou une fraction de l'ADNc complémentaire du génome du virus HIV-2 ou encore notamment les fragments contenus dans les divers clones identifiés ci-dessus. On mentionnera plus particulièrement une fraction de l'ADNc de HIV-2 contenu dans le clone E2, plus particulièrement la séquence de l'extrémité 3' (LTR) et/ou de l'extrémité 5' de la séquence HIV du clone E2 précité, ou encore l'ADNc contenant la région env, de l'ADNc du virus HIV-2.

Les sondes mises on oeuvre dans ce procédé de diagnostic du virus HIV-2 et dans les kits de diagnostic, ne sont en aucune façon réduites aux sondes décrites précédemment. Elles comprennent au contraire toutes les séquences nucléotidiques issues du génome du virus HIV-2, d'un variant de HIV-2 ou d'un virus proche par sa structure, dès lors qu'elles permettent la détection dans des fluides biologiques de personnes susceptibles de développer un SIDA d'anticorps dirigés contre un HIV-2. Naturellement l'utilisation de séquences nucléotidiques issues d'un HIV-2, initialement infectieux pour l'homme, est néanmoins préférée.

La détection peut être réalisée de toutes façons en soi connues, notamment par une mise en contact de ces sondes soit avec les acides nucléiques obtenus à partir des cellules contenues dans ces sérums ou autres milieux biologiques, par exemple liquides céphalo-rachidiens, salive, etc.., soit avec ces milieux eux-mêmes dès lors que leurs acides nucléiques auront été rendus accessibles à l'hybridation avec ces sondes, et ce dans des conditions permettant l'hybridation entre ces sondes et ces acides nucléiques et par une détection de l'hybridation éventuellement produite. Le susdit diagnostic mettant en jeu des réactions d'hybridation peut également être réalisé à l'aide de mélanges de sondes respectivement originaires d'un HIV-1 et d'un HIV-2, dès lors qu'il n'est pas nécessaire de faire une différence entre le type de virus HIV recherché.

D'une façon générale, le procédé de diagnostic de la présence ou non du virus HIV-2 ou d'un variant dans des échantillons de sérums ou d'autres liquides ou tissus obtenus à partir de patients suspectés d'être porteurs du virus HIV-2 comprend les étapes suivantes :
1) la fabrication d'un sonde marquée,
2) au moins une étape d'hybridation conduite dans des conditions stringentes, par mise en contact de l'ADN de cellules de l'échantillon du patient suspect avec ladite sonde marquée sur une menmbrane appropriée,
3) le lavage de ladite membrane avec une solution assurant la conservation de ces conditions stringentes de l'hybridation,
4) la détection de la présence ou non du virus HIV-2 par une méthode d'immunodétection.

Selon une variante du procédé précité l'hybridation précitée est conduite dans des conditions non stringentes et le lavage de la membrane est réalisé dans des conditions adaptées à celles de l'hybridation.

Les virus HIV-2 sont caractérisés par le fait que leur ARN viral est en correspondance avec un ADNc dont les régions contenant les gènes gag et env comportent respectivement les séquences nucléotidiques qui suivent (GAGRODN et ENVRN). Elles résultent du séquençage des régions correspondantes de l'ADNc correspondant au génome de HIV-2 ROD. Elles sont mises en correspondance avec les acides aminés qu'elles codent.

GAGRODN

MetGlyAlaArgAsnSerValLeuArgGlyLysLysAlaAspGlu
ATGGGCGCGAGAAACTCCGTCTTGAGAGGAAAAAAGCAGATGAA

LeuGluArgIleArgLeuArgProGlyGlyLysLysLysTyrArg
TTAGAAAGAATCAGGTTACGGCCCGGCGGAAAGAAAAAGTACAGG

LeuLysHisIleValTrpAlaAlaAsnLysLeuAspArgPheGly
CTAAAACATATTGTGTGGGCAGCGAATAAATTGGACAGATTCGGA
100
LeuAlaGluSerLeuLeuGluSerLysGluGlyCysGlnLysIle
TTAGCAGAGAGCCTGTTGGAGTCAAAAGAGGGTTGTCAAAAAATT

LeuThrValLeuAspProMetValProThrGlySerGluAsnLeu
CTTACAGTTTTAGATCCAATGGTACCGACAGGTTCAGAAAATTTA
200
LysSerLeuPheAsnThrValCysValIleTrpCysIleHisAla
AAAAGTCTTTTTAATACTGTCTGCGTCATTTGGTGCATACACGCA

GluGluLysValLysAspThrGluGlyAlaLysGlnIleValArg
GAAGAGAAAGTGAAAGATACTGAAGGAGCAAAACAAATAGTGCGG
300
ArgHisLeuValAlaGluThrGlyThrAlaGluLysMetProSer
AGACATCTAGTGGCAGAAACAGGAACTGCAGAGAAAATGCCAAGC

ThrSerArgProThrAlaProSerSerGluLysGlyGlyAsnTyr
ACAAGTAGACCAACAGCACCATCTAGCGAGAAGGGAGGAAATTAC
400
ProValGlnHisValGlyGlyAsnTyrThrHisIleProLeuSer
CCAGTGCAACATGTAGGCGGCAACTACACCCATATACCGCTGAGT

ProArgThrLeuAsnAlaTrpValLysLeuValGluGluLysLys
CCCCGAACCCTAAATGCCTGGGTAAAATTAGTAGAGGAAAAAAAG

PheGlyAlaGluValValProGlyPheGlnAlaLeuSerGluGly
TTCGGGGCAGAAGTAGTGCCAGGATTTCAGGCACTCTCAGAAGGC
500
CysThrProTyrAspIleAsnGlnMetLeuAsnCysValGlyAsp
TGCACGCCCTATGATATCAACCAAATGCTTAATTGTGTGGGCGAC

HisGlnAlaAlaMetGlnIleIleArgGluIleIleAsnGluGlu
CATCAAGCAGCCATGCAGATAATCAGGGAGATTATCAATGAGGAA
600
AlaAlaGluTrpAspValGlnHisProIleProGlyProLeuPro
GCAGCAGAATGGGATGTGCAACATCCAATACCAGGCCCCTTACCA

AlaGlyGlnLeuArgGluProArgGlySerAspIleAlaGlyThr
GCGGGGCAGCTTAGAGAGCCAAGGGGATCTGACATAGCAGGACA
700
ThrSerThrValGluGluGlnIleGlnTrpMetPheArgProGln
ACAAGCACAGTAGAAGAACAGATCCAGTGGATGTTTAGGCCACAA

AsnProValProValGlyAsnIleTyrArgArgTrpIleGlnIle
AATCCTGTACCAGTAGGAAACATCTATAGAAGATGGATCCAGATA
800
GlyLeuGlnLysCysValArgMetTyrAsnProThrAsnIleLeu
GGATTGCAGAAGTGTGTCAGGATGTACAACCCGACCAACATCCTA
AspIleLysGlnGlyProLysGluProPheGlnSerTyrValAsp
GACATAAAACAGGGACCAAAGGAGCCGTTCCAAAGCTATGTAGAT
900
ArgPheTyrLysSerLeuArgAlaGluGlnThrAspProAlaVal
AGATTCTACAAAAGCTTGAGGGCAGAACAAACAGATCCAGCAGTG
LysAsnTrpMetThrGlnThrLeuLeuValGlnAsnAlaAsnPro
AAGAATTGGATGACCCAAACACTGCTAGTACAAAATGCCAACCCA
AspCysLysLeuValLeuLysGlyLeuGlyMetAsnProThrLeu
GACTGTAAATTAGTGCTAAAAGGACTAGGGATGAACCCTACCTTA
1000
GluGluMetLeuThrAlaCysGlnGlyValGlyGlyProGlyGln
GAAGAGATGCTGACCGCCTGTCAGGGGGTAGGTGGGCCAGGCCAG
LysAlaArgLeuMetAlaGluAlaLeuLysGluValIleGlyPro
AAAGCTAGATTAATGGCAGAGGCCCTGAAAGAGGTCATAGGACCT
1100
AlaProIleProPheAlaAlaAlaGlnGlnArgLysAlaPheLys
GCCCCTATCCCATTCGCAGCAGCCCAGCAGAGAAAGGCATTTAAA
CysTrpAsnCysGlyLysGluGlyHisSerAlaArgGlnCysArg
TGCTGGAACTGTGGAAAGGAAGGGCACTCGGCAAGACAATGCCGA
1200
AlaProArgArgGlnGlyCysTrpLysCysGlyLysProGlyHis
GCACCTAGAAGGCAGGGCTGCTGGAAGTGTGGTAAGCCAGGACAC
IleMetThrAsnCysProAspArgGlnAlaGlyPheLeuGlyLeu
ATCATGACAAACTGCCCAGATAGACAGGCAGGTTTTTTAGGACTG
1300
GlyProTrpGlyLysLysProArgAsnPheProValAlaGlnVal
GGCCCTTGGGGAAAGAAGCCCCGCAACTTCCCCGTGGCCCAAGTT
ProGlnGlyLeuThrProThrAlaProProValAspProAlaVal
CCGCAGGGGCTGACACCAACAGCACCCCCAGTGGATCCAGCAGTG
AspLeuLeuGluLysTyrMetGlnGlnGlyLysArgGlnArgGlu
GATCTACTGGAGAAATATATGCAGCAAGGGAAAAGACAGAGAGAG
1400
GlnArgGluArgProTyrLysGluValThrGluAspLeuLeuHis
CAGAGAGAGAGACCATACAAGGAAGTGACAGAGGACTTACTGCAC
LeuGluGlnGlyGluThrProTyrArgGluProProThrGluAsp
CTCGAGCAGGGGGAGACACCATACAGGGAGCCACCAACAGAGGAC
1500
LeuLeuHisLeuAsnSerLeuPheGlyLysAspGln
TTGCTGCACCTCAATTCTCTCTTTGGAAAAGACCAG

ENVRN

MetMetAsnGlnLeuLeuIleAlaIleLeuLeuAlaSerAlaCys
ATGATGAATCAGCTGCTTATTGCCATTTTATTAGCTAGTGCTTGC

LeuValTyrCysThrGlnTyrValThrValPheTyrGlyValPro
TTAGTATATTGCACCCAATATGTAACTGTTTTCTATGGCGTACCC

ThrTrpLysAsnAlaThrIleProLeuPheCysAlaThrArgAsn
ACGTGGAAAAATGCAACCATTCCCCTCTTTTGTGCAACCAGAAAT
100
ArgAspThrTrpGlyThrIleGlnCysLeuProAspAsnAspAsp
AGGGATACTTGGGGAACCATACAGTGCTTGCCTGACAATGATGAT

TyrGlnGluIleThrLeuAsnValThrGluAlaPheAspAlaTrp
TATCAGGAAATAACTTTGAATGTAACAGAGGCTTTTGATGCATGG
200
AsnAsnThrValThrGluGlnAlaIleGluAspValTrpHisLeu
AATAATACAGTAACAGAACAAGCAATAGAAGATGTCTGGCATCTA

PheGluThrSerIleLysProCysValLysLeuThrProLeuCys
TTCGAGACATCAATAAAACCATGTGTCAAACTAACACCTTTATGT
300
ValAlaMetLysCysSerSerThrGluSerSerThrGlyAsnAsn
GTAGCAATGAAATGCAGCAGCACAGAGAGCAGCACAGGGAACAAC

ThrThrSerLysSerThrSerThrThrThrThrThrProThrAsp
ACAACCTCAAAGAGCACAAGCACAACCACAACCACACCCACAGAC
400
GlnGluGlnGluIleSerGluAspThrProCysAlaArgAlaAsp
CAGGAGCAAGAGATAAGTGAGGATACTCCATGCGCACGCGCAGAC

AsnCysSerGlyLeuGlyGluGluGluThrIleAsnCysGlnPhe
AACTGCTCAGGATTGGGAGAGGAAGAAACGATCAATTGCCAGTTC

AsnMetThrGlyLeuGluArgAspLysLysLysGlnTyrAsnGlu
AATATGACAGGATTAGAAAGAGATAAGAAAAAACAGTATAATGAA
500
ThrTrpTyrSerLysAspValValCysGluThrAsnAsnSerThr
ACATGGTACTCAAAAGATGTGGTTTGTGAGACAAATAATAGCACA

AsnGlnThrGlnCysTyrMetAsnHisCysAsnThrSerValIle
AATCAGACCCAGTGTTACATGAACCATTGCAACACATCAGTCATC
600
ThrGluSerCysAspLysHisTyrTrpAspAlaIleArgPheArg
ACAGAATCATGTGACAAGCACTATTGGGATGCTATAAGGTTTAGA

TyrCysAlaProProGlyTyrAlaLeuLeuArgCysAsnAspThr
TACTGTGCACCACCGGGTTATGCCCTATTAAGATGTAATGATACC
700
AsnTyrSerGlyPheAlaProAsnCysSerLysValValAlaSer
AATTATTCAGGCTTTGCACCCAACTGTTCTAAAGTAGTAGCTTCT

ThrCysThrArgMetMetGluThrGlnThrSerThrTrpPheGly
ACATGCACCAGGATGATGGAAACGCAAACTTCCACATGGTTTGGC
800
PheAsnGlyThrArgAlaGluAsnArgThrTyrIleTyrTrpHis
TTTAATGGCACTAGAGCAGAGAATAGAACATATATCTATTGGCAT
GlyArgAspAsnArgThrIleIleSerLeuAsnLysTyrTyrAsn
GGCAGAGATAATAGAACTATCATCAGCTTAAACAAATATTATAAT
900
LeuSerLeuHisCysLysArgProGlyAsnLysThrValLysGln
CTCAGTTTGCATTGTAAGAGGCCAGGGAATAAGACAGTGAAACAA
IleMetLeuMetSerGlyHisValPheHisSerHisTyrGlnPro
ATAATGCTTATGTCAGGACATGTGTTTCACTCCCACTACCAGCCG
IleAsnLysArgProArgGlnAlaTrpCysTrpPheLysGlyLys
ATCAATAAAAGACCCAGACAAGCATGGTGCTGGTTCAAAGGCAAA
1000
TrpLysAspAlaMetGlnGluValLysThrLeuAlaLysHisPro
TGGAAAGACGCCATGCAGGAGGTGAAGACCCTTGCAAAACATCCC
ArgTyrArgGlyThrAsnAspThrArgAsnIleSerPheAlaAla
AGGTATAGAGGAACCAATGACACAAGGAATATTAGCTTTGCAGCG
1100
ProGlyLysGlySerAspProGluValAlaTyrMetTrpThrAsn
CCAGGAAAAGGCTCAGACCCAGAAGTAGCATACATGTGGACTAAC
CysArgGlyGluPheLeuTyrCysAsnMetThrTrpPheLeuAsn
TGCAGAGGAGAGTTTCTCTACTGCAACATGACTTGGTTCCTCAAT
1200
TrpIleGluAsnLysThrHisArgAsnTyrAlaProCysHisIle
TGGATAGAGAATAAGACACACCGCAATTATGCACCGTGCCATATA
LysGlnIleIleAsnThrTrpHisLysValGlyArgAsnValTyr
AAGCAAATAATTAACACATGGCATAAGGTAGGGAGAAATGTATAT
1300
LeuProProArgGluGlyGluLeuSerCysAsnSerThrValThr
TTGCCTCCCAGGGAAGGGGAGCTGTCCTGCAACTCAACAGTAACC
SerIleIleAlaAsnIleAspTrpGlnAsnAsnAsnGlnThrAsn
AGCATAATTGCTAACATTGACTGGCAAAACAATAATCAGACAAAC
IleThrPheSerAlaGluValAlaGluLeuTyrArgLeuGluLeu
ATTACCTTTAGTGCAGAGGTGGCAGAACTATACAGATTGGAGTTG
1400
GlyAspTyrLysLeuValGluIleThrProIleGlyPheAlaPro
GGAGATTATAAATTGGTAGAAATAACACCAATTGGCTTCGCACCT

ThrLysGluLysArgTyrSerSerAlaHisGlyArgHisThrArg
ACAAAAGAAAAAAGATACTCCTCTGCTCACGGGAGACATACAAGA
1500
GlyValPheValLeuGlyPheLeuGlyPheLeuAlaThrAlaGly
GGTGTGTTCGTGCTAGGGTTCTTGGGTTTTCTCGCAACAGCAGGT

SerAlaMetGlyAlaArgAlaSerLeuThrValSerAlaGlnSer
TCTGCAATGGGCGCTCGAGCGTCCCTGACCGTGTCGGCTCAGTCC
1600
ArgThrLeuLeuAlaGlyIleValGlnGlnGlnGlnGlnLeuLeu
CGGACTTTACTGGCCGGGATAGTGCAGCAACAGCAACAGCTGTTG

AspValValLysArgGlnGlnGluLeuLeuArgLeuThrValTrp
GACGTGGTCAAGAGACAACAAGAACTGTTGCGACTGACCGTCTGG
1700
GlyThrLysAsnLeuGlnAlaArgValThrAlaIleGluLysTyr
GGAACGAAAAACCTCCAGGCAAGAGTCACTGCTATAGAGAAGTAC

LeuGlnAspGlnAlaArgLeuAsnSerTrpGlyCysAlaPheArg
CTACAGGACCAGGCGCGGCTAAATTCATGGGGATGTGCGTTTAGA
1800
GlnValCysHisThrThrValProTrpValAsnAspSerLeuAla
CAAGTCTGCCACACTACTGTACCATGGGTTAATGATTCCTTAGCA

ProAspTrpAspAsnMetThrTrpGlnGluTrpGluLysGlnVal
CCTGACTGGGACAATATGACGTGGCAGGAATGGGAAAAACAAGTC

ArgTyrLeuGluAlaAsnIleSerLysSerLeuGluGlnAlaGln
CGCTACCTGGAGGCAAATATCAGTAAAAGTTTAGAACAGGCACAA
1900
IleGlnGlnGluLysAsnMetTyrGluLeuGlnLysLeuAsnSer
ATTCAGCAAGAGAAAAATATGTATGAACTACAAAAATTAAATAGC

TrpAspIlePheGlyAsnTrpPheAspLeuThrSerTrpValLys
TGGGATATTTTTGGCAATTGGTTTGACTTAACCTCCTGGGTCAAG
2000
TyrIleGlnTyrGlyValLeuIleIleValAlaValIleAlaLeu
TATATTCAATATGGAGTGCTTATAATAGTAGCAGTAATAGCTTTA

ArgIleValIleTyrValValGlnMetLeuSerArgLeuArgLys
AGAATAGTGATATATGTAGTACAAATGTTAAGTAGGCTTAGAAAG
2100
GlyTyrArgProValPheSerSerProProGlyTyrIleGlnGln
GGCTATAGGCCTGTTTTCTCTTCCCCCCCCGGTTATATCCAACAG

IleHisIleHisLysAspArgGlyGlnProAlaAsnGluGluThr
ATCCATATCCACAAGGACCGGGGACAGCCAGCCAACGAAGAAACA
. . . 2200
GluGluAspGlyGlySerAsnGlyGlyAspArgTyrTrpProTrp
GAAGAAGACGGTGGAAGCAACGGTGGAGACAGATACTGGCCCTGG
. . . . .
ProIleAlaTyrIleHisPheLeuIleArgGlnLeuIleArgLeu
GCGATAGCATATATACATTTCCTGATCCGCCAGCTGATTCGCCTC
. . . .
LeuThrArgLeuTyrSerIleCysArgAspLeuLeuSerArgSer
TTGACCAGACTATACAGCATCTGCAGGGACTTACTATCCAGGAGC
2300 . . . .
PheLeuThrLeuGlnLeuIleTyrGlnAsnLeuArgAspTrpLeu
TTCCTGACCCTCCAACTCATCTACCAGAATCTCAGAGACTGGCTG
. . . .
ArgLeuArgThrAlaPheLeuGlnTyrGlyCysGluTrpIleGln
AGACTTAGAACAGCCTTCTTGCAATATGGGTGCGAGTGGATCCAA
. 2400 . . .
GluAlaPheGlnAlaAlaAlaArgAlaThrArgGluThrLeuAla
GAAGCATTCCAGGCCGCCGCGAGGGCTACAAGAGAGACTCTTGCG
. . . .
GlyAlaCysArgGlyLeuTrpArgValLeuGluArgIleGlyArg
GGCGCGTGCAGGGGCTTGTGGAGGGTATTGGAACGAATCGGGAGG
. . 2500 . .
GlyIleLeuAlaValProArgArgIleArgGlnGlyAlaGluIle
GGAATACTCGCGGTTCCAAGAAGGATCAGACAGGGAGCAGAAATC
. . . .
AlaLeuLeu***GlyThrAlaValSerAlaGlyArgLeuTyrGlu
GCCCTCCTGTGAGGGACGGCAGTATCAGCAGGGAGACTTTATGAA
. . . 2600 .
TyrSerMetGluGlyProSerSerArgLysGlyGluLysPheVal
TACTCCATGGAAGGACCCAGCAGCAGAAAGGGAGAAAAATTTGTA
. . . .
GlnAlaThrLysTyrGly
CAGGCAACAAAATATGGA
. .

Les HIV-2 selon la présente demande sont tels que leurs ARNs présentent des caractéristiques semblables, en particulier des régions gag et env comportant des séquences ayant des homologies de séquence nucléotidique d'au moins 50%, de préférence 70% et plus avantageusement encore 90% avec les séquences gag et env de HIV-2 ROD.

La demande fait plus particulièrement référence aux fragments d'ADNc qui codent respectivement pour les protéines p16, p26 et p12 et qui sont également inclues dans GAGRODN. En particulier elle décrit les séquences s'étendant :
- à partir du nucléotide 1 jusqu'au nucléotide 405 (codant pour p16)
- à partir du nucléotide 406 jusqu'au nucléotide 1155 (codant pour p26) et,
- à partir du nucléotide 1156 jusqu'au nucléotide 1566 (codant pour p12).

Le fragment d'ADNc codant pour la gp140 inclue dans ENVRN et s'étend à partir du nucléotide 1 jusqu'au nucléotide 2574.

La demande décrit aussi les séquences de nucléotides qui se distinguent des précédentes par des substitutions de nucléotides mettant à profit la dégénérescence du code génétique, dès lors que ces substitutions n'entraînent pas une modification des séquences en acides aminés que codent lesdites séquences de nucléotides.

De même la demande décrit les protéines ou glycoprotéines dont les séquences en acides aminés correspondent à celles qui découlent des pages précédentes, ainsi que les peptides équivalents à savoir des peptides se distinguant de ceux qui découlent des pages précédentes par addition, substitution ou délétion d'acides aminés qui n'affectent pas les propriétés immunologiques globales desdits peptides. La glycoprotéine d'enveloppe présente la séquence en acides aminés qui découle de ENVRN.

La demande fait aussi référence à une composition immunogène caractérisée on ce qu'elle est dosée en antigène, tel que la gp 140 du virus HIV-2, de façon à permettre l'administration de doses unitaires de 10 à 500, notamment du 50 à 100 mg par kg de corps.

Les techniques décrites dans la demande de brevet européen 85.905.513 9 déposée le 18 Octobre 1985 pour la production de peptides ou de protéines consistant en des produits d'expression de séquences d'acides nucléiques dérivées du génome de HIV-1 sont applicables également à la production des susdits peptides ou protéines dérivés de HIV-2.

A titre indicatif, les poids moléculaires théoriques (PM) des protéines de HIV-2 sont indiqués, en comparaison avec ceux de HIV-1.

| PM des protéines de HIV-2 en kd | | PM des protéines pour HIV-1 en kd | |
|---|---|---|---|
| gag complet | 58,3 | gag complet | 55,8 |
| p 16 | 15 | p 18 | 14,9 |
| p 26 | 27,6 | | |
| p 12 | 15,8 | | |
| env | 98,6 | env | 97,4 |
| env externe | 57,4 | | |
| env transmembranaire | 41,2 | | |

HIV-2 MIR et HIV-2 ROD ont également été déposés à la "National Collection of Animal Cell Cultures" (ECACC) à SALISBURY (Grande Bretagne) le 9 Janvier 1987 sous les numéros d'accès 87. 01.1001 et 87. 01.1002 respectivement.

De plus, les plasmides pROD35 et pROD27,5 ont été déposés à la "National Collection of Industrial Bacteria" (NCIB) à ABERDEEN (Grande Bretagne) le 9 janvier 1987 sous les numéros d'accès respectifs 12.398 et 12.399.

### BIBLIOGRAPHIE

- 1 -: F. Barré-Sinoussi et al., Science 220. 868 (1983).
- 2 -: L. Montagnier et al., In : Human Tcell leukemia Orlymphoma viruses (Gallo, R.C., Essex, M.E., Gross, L., eds.) Cold Spring Harbor Laboratory, New York, 363 (1984).
- 3 -: M. Popovic, M.G. Sarngadharan, E. Read, R.C. Gallo, Science 224, 497 (1984).
- 4 -: J. Levy et al., Science 225, 840 (1984).
- 5 -: P. Sonigo et al., Cell 42. 369 (1985).
- 7 -: J.W. Curran et al., Science 229, 1352 (1985).
- 8 -: A. Ellrodt et al., Lancet i, 1383 (1984).
- 9 -: P. Piot et al., Lancet ii, 65 (1984).
- 10 -: F. Brun-Vezinet et al., Science 226, 453 (1984).
- 11 -: N. Clumeck et al., N. Engl. J. Med. 313, 182 (1985).
- 12 -: A.B. Rabson and M.A. Martin, Cell 40, 477 (1985).
- 13 -: S. Benn et al., Science 230, 949 (1985).
- 14 -: M. Alizon, Manuscript in preparation.
- 15 -: F. Brun-Vezinet, Unpublished data.
- 16 -: M.D. Daniel et al., Science 228, 1201 (1985).
- 17 -: P.J. Kanki et al., Science 228, 1199 (1985).
- 18 -: N.L. Letwin et al., Science 230, 71 (1985).
- 19 -: A. Gatzar et al., Blood 55, 409 (1980).
- 20 -: D. Klatzmann et al., Science 225, 59 (1984).
- 21 -: L. Montagnier et al., Viralogy 144, 283 (1985).
- 22 -: J.S. Allan et al., Science 228, 1091 (1985).
- 23 -: F. CHIVel, Manuscript in preparation.
- 24 -: F. diMarzo Veronese et al., Science 229, 1402 (1985).
- 25 -: V.S. Kalyanaraman et al., Science 218, 571 (1982).
- 26 -: I.S.Y. Chen, J. McLaughlin, J.C. Gasson, S.C. Clark and D.W. Golde, Nature 305, 502 (1983).
- 27 -: F. Barin et al., Lancet ii, 1387 (1985).
- 28 -: P.J. Kanki, J. Alroy, M. Essex, Science 230, 951 (1985).
- 29 -: H. Towbin et al., Proc. Natl. Acad. Sci. USA 76, 4350 (1979).
- 30 -: S. Wain-Hobson, P. Sonigo, O. Danos, S. Cole et M. Alizon, Cell 40, 9 (1985).
- 31 -: M. Alizon et al., Nature 312, 757 (1984).

## Revendications

1. Procédé de fabrication d'une protéine ou d'une glycoprotéine ou d'une partie de protéine ou de glycoprotéine consistant dans le produit d'expression d'une séquence d'acide nucléique dérivée du génome d'un rétrovirus, caractérisé par
- l'insertion, dans un vecteur susceptible de transformer un hôte cellulaire choisi et de permettre l'expression dans cet hôte d'un insérat contenu dans ce vecteur, de la susdite séquence d'acide nucléique, dérivée d'un rétrovirus humain HIV-2 capable d'infecter des lymphocytes T4 humains et susceptible de provoquer un SIDA chez l'homme, ledit rétrovirus HIV-2 étant représenté par les isolats déposés à la CNCM sous les n° I-502, I-532, I-642 et I-643 ou à l'ECACC sous les n° 87.01.1001 et 87.01.1002, ou l'insertion de la susdite séquence d'acide nucléique dérivée d'un variant de ce rétrovirus dont la glycoprotéine majeure d'enveloppe est reconnue par des anticorps formés contre la glycoprotéine majeure d'enveloppe gp140 d'un rétrovirus HIV-2,
- l'introduction dans l'hôte choisi, dudit vecteur contenant ladite séquence nucléique,
- la culture de l'hôte cellulaire tranformé et
- la récupération et la purification de la protéine ou de la partie de protéine exprimée par ladite séquence d'acide nucléique, ladite protéine ou partie de protéine étant apte à produire une réaction immunologique spécifique avec des anticorps dirigés contre le rétrovirus HIV-2.

2. Procédé de fabrication d'une protéine ou d'une partie de protéine selon la revendication 1, caractérisé en ce que l'on récupère et on purifie le produit d'expression de tout ou partie de la séquence d'acide nucléique suivante contenant les nucléotides 1 à 1566, correspondant a la séquence gag de l'ARN génomique du rétrovirus HIV-2.

GAGRODE .

ATGCGGCGCCAGAAACTCCGTGTTGACAGGGAAAAAGCAGATGAA

TTACAAAGAATCACGTTACCGCCCCGCCCAAAGAAAAAGTACAGG

CTAAAACATATTGTGTGGGCAGCCAATAAATTGGACAGATTCCGA
100

TTAGCACAGACCCTGTTCGAGTCAAAACACGGTTGTCAAAAAATT

CTTACAGTTTTAGATCCAATGGTACCGACAGGTTCAGAAAATTTA
200

AAAAGTCTTTTTAATACTGTCTGCCGTCATTTGGTGCATACACCGA

GAAGAGAAAGTCAAAGATACTGAAGCAGCAAAACAAATAGTGCGG
300

AGACATCTAGTGGCAGAAACAGGAACTGCAGAGAAAATGCCAAGC

ACAAGTAGACCAACAGCACCATCTAGCGACAAGGCAGGAAATTAC
400

CCAGTGCAACATGTAGGCGGCAACTACACCCATATACCGCTGACT

CCCCGAACCCTAAATGCCTGGGTAAAATTAGTAGACGAAAAAAAG

TTCGGGGCAGAAGTAGTGCCAGGATTTCACGCACTCTCAGAAGG[?]
500

TGCACGCCCTATGATATCAACCAAATGCTTAATTGTGTCGGCGAC

CATCAAGCAGCCATGCAGATAATCAGCGACATTATCAATCACCAA
600

GCAGCACAATGGGATGTCCAACATCCAATACCAGGCCCCTTACCA

GCCCGGCCAGCTTAGACAGCCAAGGGCATCTGACATACCAGGCACA
700

ACAAGCACAGTACAAGAACAGATCCAGTGCATGTTTACGCCACAA

AATCCTGTACCAGTACCAAACATCTATACAAGATGGATCCAGATA
800
GGATTCCAGAAGTGTGTCACGATGTACAACCCGACCAACATCCTA
GACATAAAACAGCCACCAAAGGAGCCCGTTCCAAAGCTATGTACAT
900
ACATTCTACAAAAGCTTGAGGGCAGAACAAACAGATCCAGCAGTG
AACAATTCGATGACCCAAACACTGCTAGTACAAAATGCCAACCCA
GACTGTAAATTAGTGCTAAAAGGACTAGGGATGAACCCTACCTTA
1000
GAAGAGATGCTGACCGCCTGTCAGGGCGTAGGTGGCCCAGGCCAG
AAAGCTAGATTAATGGCAGAGGCCCTGAAAGAGGTCATAGGACCT
1100
GCCCCTATCCCATTCGCAGCAGCCCAGCAGAGAAAGGCATTTAAA
TGCTGGAACTGTGGAAAGGAAGGGCACTCCG[?]CAAGACAATGCCGA
1200
GCACCTAGAAGGCAGGGCTGCTGGAAGTGTGGTAAGCCAGGACAC
ATCATGACAAACTGCCCAGATAGACAGGCAGGTTTTTAGGACTG
1300
GGCCCTTGGGGAAAGAAGCCCCGCAACTTCCCCGTGGCCCAAGTT
CCGCAGGGGCTGACACCAACAGCACCCCCAGTGGATCCAGCAGTG
CATCTACTCCAGAAATATATGCAGCAAGCCAAAAGACAGACAGAG
1400
GAGAGACAGAGACCATACAACGAAGTCACAGAGGACTTACTGCAC
CTCGAGCAGGGGGAGACACCATACAGGGAGCCACCAACAGAGGAC
1500
TTGCTCCACCTCAATTCTCTCTTTGGAAAAGACCAG

3. Procédé de fabrication d'une protéine ou d'une glycoprotéine ou d'une partie de protéine ou de glycoprotéine selon la revendication 1, caractérisé en ce que l'on récupère et on purifie le produit d'expression de tout ou partie de la séquence d'acide nucléique suivante contenant les nucléotides 1 à 2574, correspondant à la séquence env suivante de l'ARN génomique du rétrovirus HIV-2.

ATGATGAATCAGCTGCTTATTGCCATTTTATTAGCTAGTGCTTGC
TTAGTATATTGCACCCAATATGTAACTGTTTTCTATGGCGTACCC
ACGTGGAAAAATGCAACCATTCCCCTCTTTTGTGCAACCAGAAAT
100
ACCGATACTTGGGGAACCATACAGTGCTTGCCTGACAATGATGAT
TATCAGGAAATAACTTTGAATGTAACAGAGGCTTTTGATGCATGG
200
AATAATACAGTAACAGAACAAGCAATAGAAGATGTCTGGCATCTA
TTCGAGACATCAATAAAACCATGTGTCAAACTAACACCTTTATGT
300
GTAGCAATGAAATGCAGCAGCACAGAGAGCAGCACAGGGAACAAC
ACAACCTCAAAGAGCACAAGCACAACCACAACCACACCCACAGAC
400
CAGGAGCAAGAGATAAGTGAGGATACTCCATGCGCACGCGCAGAC
AACTGCTCAGGATTGGGAGAGGAAGAAACGATCAATTGCCAGTTC
AATATGACAGGATTAGAAAGAGATAAGAAAAAACAGTATAATGAA
500
ACATGGTACTCAAAAGATGTGGTTTGTGAGACAAATAATAGCACA
AATCAGACCCAGTGTTACATGAACCATTGCAACACATCAGTCATC
600
ACAGAATCATGTGACAAGCACTATTGGGATGCTATAAGGTTTAGA
TACTGTGCACCACCGGGTTATGCCCTATTAAGATGTAATGATACC
700
AATTATTCAGGCTTTGCACCCAACTGTTCTAAAGTAGTAGCTTCT

ACATGCACCACGATGATGCAAACGCAAACTTCCACATGGTTTGGC
800
TTTAATGGCACTAGAGCAGAGAATAGAACATATATCTATTGGCAT
GGCAGAGATAATACAACTATCATCAGCTTAAACAAATATTATAAT
900
CTCAGTTTCCATTGTAAGAGGCCACGCAATAAGACAGTGAAACAA
ATAATGCTTATGTCAGGACATGTGTTTCACTCCCACTACCAGCCG
ATCAATAAAAGACCCAGACAAGCATGGTGCTGGTTCAAAGGCAAA
1000
TGGAAAGACGCCATGCAGGAGGTGAAGACCCTTGCAAAACATCCC
AGGTATAGAGGAACCAATGACACAAGGAATATTAGCTTTGCAGCG
1100
CCAGCAAAAGGCTCAGACCCAGAAGTAGCATACATGTGGACTAAC
TGCAGAGGAGAGTTTCTCTACTGCAACATGACTTGGTTCCTCAAT
1200
TGCATAGAGAATAAGACACACCGCAATTATGCACCGTGCCATATA
AAGCAAATAATTAACACATGGCATAAGGTAGGCAGAAATGTATAT
1300
TTGCCTCCCAGGGAAGGGGAGCTGTCCTGCAACTCAACAGTAACC
AGCATAATTGCTAACATTGACTGGCAAAACAATAATCAGACAAAC
ATTACCTTTAGTGCAGAGGTGGCAGAACTATACAGATTGGAGTTG
1400
CCAGATTATAAATTGGTAGAAATAACACCAATTGGCTTCGCACCT

ACAAAAGCAAAAAAGATACTCCTCTGCTCACGGCAGACATACAAGA
1500
GGTGTGTTCGTGCTAGGGTTCTTGGGTTTTCTCGCAACAGCAGGT
TCTGCAATGGCCGCTCGAGCGTCCCTGACCGTGTCGGCTCAGTCC
1600
CGGACTTTACTGGCCGGGATAGTGCAGCAACAGCAACAGCTGTTG
GACGTGGTCAAGAGACAACAAGAACTGTTGCGACTGACCGTCTGG
1700
GGAACGAAAAACCTCCAGGCAAGAGTCACTGCTATAGAGAAGTAC
CTACAGGACCAGGCGCGGCTAAATTCATGGGATGTGCGTTTAGA
1800
CAAGTCTGCCACACTACTGTACCATGGCTTAATGATTCCTTAGCA
CCTGACTGGGACAATATGACGTGGCAGGAATGGGAAAAACAAGTC
CGCTACCTGGAGGCAAATATCAGTAAAAGTTTAGAACAGGCACAA
1900
ATTCAGCAAGAGAAAAATATGTATGAACTACAAAAATTAAATAGC
TGGGATATTTTTGGCAATTGGTTTGACTTAACCTCCTGGGTCAAG
2000
TATATTCAATATGGAGTGCTTATAATAGTAGCAGTAATAGCTTTA
AGAATAGTCATATATGTAGTACAAATGTTAAGTAGGCTTAGAAAG
2100
GGCTATAGGCCTGTTTTCTCTTCCCCCCCCGGTTATATCCAACAG

ATCCATATCCACAAGGACCGGGACAGCCAGCCAACGAAGAAACA
2200
CAACAAGACCGTCCAACCAACCGTCCACACAGATACTGCCCCTGG
CCCATAGCATATATACATTTCCTGATCCGCCAGCTGATTCGCCTC
TTGACCAGACTATACAGCATCTGCACGGACTTACTATCCAGGAGC
2300
TTCCTGACCCTCCAACTCATCTACCAGAATCTCAGAGACTGGCTG
AGACTTAGAACAGCCTTCTTGCAATATCGGTGCCGAGTGGATCGAA
2400
GAAGCATTCCAGGCCGCCCGCGAGGGCTACAAGACAGACTCTTGCG
GGCCGCTGCAGGGGCTTGTCGAGCGTATTCGAACGAATCGGGAGG
2500
GGAATACTCGCCGGTTCCAAGAACGATCAGACAGGCAGCAGAAATC
GCCGTCCTG

4. Procédé de fabrication d'une protéine ou d'une partie de protéine selon la revendication 1, caractérisé en ce que l'on récupère et on purifie une protéine ou partie de protéine correspondant à tout ou partie de la séquence d'acides aminés suivante :

ArgLysAlaPheLys
CysTrpAsnCysGlyLysGluGlyHisSerAlaArgGlnCysArg
AlaProArgArgGlnGlyCysTrpLysCysGlyLysProGlyHis
IleMetThrAsnCysProAspArgGlnAlaGlyPheLeuGlyLeu
GlyProTrpGlyLysLysProArgAsnPheProValAlaGlnVal
ProGlnGlyLeuThrProThrAlaProProValAspProAlaVal
AspLeuLeuGluLysTyrMetGlnGlnGlyLysArgGlnArgGlu
GlnArgGluArgProTyrLysGluValThrGluAspLeuLeuHis
LeuGluGlnGlyGluThrProTyrArgGluProProThrGluAsp
LeuLeuHisLeuAsnSerLeuPheGlyLysAspGln

5. Procédé de fabrication d'une protéine ou d'une partie de protéine selon la revendication 1, caractérisé en ce que l'on récupère et on purifie une protéine ou partie de protéine correspondant à tout ou partie de la séquence d'acides aminés suivante :

MetGlyAlaArgAsnSerValLeuArgGlyLysLysAlaAspGlu
LeuGluArgIleArgLeuArgProGlyGlyLysLysLysTyrArg
LeuLysHisIleValTrpAlaAlaAsnLysLeuAspArgPheGly
LeuAlaGluSerLeuLeuGluSerLysGluGlyCysGlnLysIle
LeuThrValLeuAspProMetValProThrGlySerGluAsnLeu
LysSerLeuPheAsnThrValCysValIleTrpCysIleHisAla
GluGlnLysValLysAspThrGluGlyAlaLysGlnIleValArg
ArgHisLeuValAlaGluThrGlyThrAlaGluLysMetProSer
ThrSerArgProThrAlaProSerSerGluLysGlyGlyAsnTyr

6. Procédé de fabrication d'une protéine selon la revendication 1 caractérisé en ce que l'on récupère et on purifie une protéine ayant les caractéristiques immunologiques de la protéine p12 de HIV-2, reconnue immunologiquement par des anticorps formés contre un rétrovirus humain HIV-2 et ayant un poids moléculaire de 12000 daltons déterminé par électrophorèse dans un gel de polyacrylamide dans des conditions dénaturantes.

7. Procédé de fabrication d'une protéine selon la revendication 1 caractérisé en ce que l'on récupère et on purifie une protéine ayant les caractéristiques immunologiques de la protéine p16 de HIV-2, reconnue immunologiquement par des anticorps formés contre un rétrovirus humain HIV-2 et ayant un poids moléculaire de 16000 daltons déterminé par électrophorèse dans un gel de polyacrylamide dans des conditions dénaturantes.

8. Procédé de fabrication d'une protéine selon la revendication 1 caractérisé en ce que l'on récupère et on purifie une protéine ayant les caractéristiques immunologiques de la protéine p26 de HIV-2, reconnue immunologiquement par des anticorps formés contre un rétrovirus humain HIV-2 et ayant un poids moléculaire de 26000 daltons déterminé par électrophorèse dans un gel de polyacrylamide dans des conditions dénaturantes.

9. Procédé de fabrication d'une protéine selon la revendication 1 caractérisé en ce que l'on récupère et on purifie une protéine ayant les caractéristiques immunologiques de la protéine p36 de HIV-2, reconnue immunologiquement par des anticorps formés contre un rétrovirus humain HIV-2 et ayant un poids moléculaire de 36000 daltons déterminé par électrophorèse dans un gel de polyacrylamide dans des conditions dénaturantes.

10. Procédé de fabrication d'une protéine selon la revendication 1 caractérisé en ce que l'on récupère et on purifie une protéine ayant les caractéristiques immunologiques de la protéine p42 de HIV-2, reconnue immunologiquement par des anticorps formés contre un rétrovirus humain HIV-2 et ayant un poids moléculaire de 42000-45000 daltons déterminé par électrophorèse dans un gel de polyacrylamide dans des conditions dénaturantes.

11. Procédé de fabrication d'une protéine selon la revendication 1 caractérisé en ce que l'on récupère et on purifie une protéine ayant les caractéristiques immunologiques de la glycoprotéine d'enveloppe gp 140 de HIV-2, reconnue immunologiquement par des anticorps formés contre un rétrovirus humain HIV-2 et ayant un poids moléculaire de 130000-140000 daltons déterminé par électrophorèse dans un gel de polyacrylamide dans des conditions dénaturantes.

12. Culture cellulaire caractérisée en ce qu'elle est transformée par un ADN recombinant contenant un acide nucléique dérivé d'une partie au moins de l'ARN du rétrovirus humain HIV-2 ou de l'un de ses variants, dans des conditions permettant la production d'une protéine ou d'une partie de protéine selon le procédé de la revendication 1, ledit acide nucléique ayant la capacité de s'hybrider avec l'ARN dudit rétrovirus humain HIV-2 et n'hybridant pas dans les conditions stringentes avec les sondes 1 à 4 comprenant respectivement les nucléotides 990-1070, 990-1260, 2170-2240, 3370-3640 de l'ADN dérivé du génome de HIV-1.

13. Culture cellulaire selon la revendication 12, caractérisée en ce qu'il s'agit d'une culture de microorganismes notamment de E. coli.

14. Culture cellulaire selon la revendication 12, caractérisée en ce qu'il s'agit d'une culture de cellules eucaryotes, notamment de levures.

15. Culture cellulaire selon la revendication 12, caractérisée en ce qu'il s'agit de cellules eucaryotes supérieures telles que des cellules de mammifères.

16. Culture cellulaire selon l'une quelconque des revendications 12 à 15, caractérisée en ce qu'elle est transformée par un acide nucléique contenant une séquence de nucléotides codant pour la séquence d'acides aminés GAGRODN suivante ou pour une partie de cette séquence ayant la capacité de donner une réaction immunologique avec des anticorps dirigés contre un rétrovirus HIV-2, lorsque ladite séquence est mise en contact avec un sérum d'un patient infecté par ledit rétrovirus HIV-2.

GAGRODE

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArgGluIleIleAsnGluGlu

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle
GlyLeuGlnLysCysValArgMetTyrAsnProThrAsnIleLeu
AspIleLysGlnGlyProLysGluProPheGlnSerTyrValAsp
900
ArgPheTyrLysSerLeuArgAlaGluGlnThrAspProAlaVal
LysAsnTrpMetThrGlnThrLeuLeuValGlnAsnAlaAsnPro
AspCysLysLeuValLeuLysGlyLeuGlyMetAsnProThrLeu
GluGluMetLeuThrAlaCysGlnGlyValGlyGlyProGlyGln
LysAlaArgLeuMetAlaGluAlaLeuLysGluValIleGlyPro
AlaProIleProPheAlaAlaAlaGlnGlnArgLysAlaPheLys
CysTrpAsnCysGlyLysGluGlyHisSerAlaArgGlnCysArg
AlaProArgArgGlnGlyCysTrpLysCysGlyLysProGlyHis
IleMetThrAsnCysProAspArgGlnAlaGlyPheLeuGlyLeu
GlyProTrpGlyLysLysProArgAsnPheProValAlaGlnVal
ProGlnGlyLeuThrProThrAlaProProValAspProAlaVal
AspLeuLeuGluLysTyrMetGlnGlnGlyLysArgGlnArgGlu
GlnArgGluArgProTyrLysGluValThrGluAspLeuLeuHis
LeuGluGlnGlyGluThrProTyrArgGluProProThrGluAsp
LeuLeuHisLeuAsnSerLeuPheGlyLysAspGln

17. Culture cellulaire selon l'une quelconque des revendications 12 à 15, caractérisée en ce qu'elle est transformée par un acide nucléique codant pour la séquence d'acides aminés ci-après ou pour une partie de cette séquence, cette séquence ou partie de cette séquence ayant la capacité de donner une réaction immunologique avec des anticorps contre un rétrovirus HIV-2, lorsque ladite séquence est mise en contact avec un sérum d'un patient infecté par ledit rétrovirus HIV-2.

ArgLysAlaPheLys
CysTrpAsnCysGlyLysGluGlyHisSerAlaArgGlnCysArg
AlaProArgArgGlnGlyCysTrpLysCysGlyLysProGlyHis
IleMetThrAsnCysProAspArgGlnAlaGlyPheLeuGlyLeu
GlyProTrpGlyLysLysProArgAsnPheProValAlaGlnVal
ProGlnGlyLeuThrProThrAlaProProValAspProAlaVal
AspLeuLeuGluLysTyrMetGlnGlnGlyLysArgGlnArgGlu
GlnArgGluArgProTyrLysGluValThrGluAspLeuLeuHis
LeuGluGlnGlyGluThrProTyrArgGluProProThrGluAsp
LeuLeuHisLeuAsnSerLeuPheGlyLysAspGln

18. Culture cellulaire selon l'une quelconque des revendications 12 à 15, caractérisée en ce qu'elle est transformée par un acide nucléique contenant une séquence nucléotidique codant pour tout ou partie de la séquence d'acides aminés ci-après, cette séquence ou partie de cette séquence ayant la capacité de donner une réaction immunologique avec des anticorps contre un rétrovirus HIV-2, lorsque ladite séquence est mise en contact avec un sérum d'un patient infecté par ledit rétrovirus HIV-2.

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

19. Culture cellulaire selon l'une quelconque des revendications 12 à 15, caractérisée en ce qu'elle est transformée par un acide nucléique contenant une séquence nucléotidique codant pour tout ou partie de la séquence d'acides aminés ci-après, cette séquence ou partie de cette séquence ayant la capacité de donner une réaction immunologique avec des anticorps contre un rétrovirus HIV-2, lorsque ladite séquence est mise en contact avec un sérum d'un patient infecté par ledit rétrovirus HIV-2.

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArgGluIleIleAsnGluGln

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

ArgPheTyrLysSerLeuArgAlaGluGlnThrAspProAlaVal

LysAsnTrpMetThrGlnThrLeuLeuValGlnAsnAlaAsnPro.

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGln

20. Application du procédé selon l'une quelconque des revendications 1 à 11, à la fabrication de protéines ou parties de protéines utilisables dans des réactions de détection d'anticorps dirigés contre le rétrovirus HIV-2.

21. Application du procédé selon l'une quelconque des revendications 1 à 11 à la fabrication de protéines ou de parties de protéines immunogènes.

## Claims

1. Process for the production of a protein or glycoprotein or of a part of a protein or glycoprotein consisting of the expression product of a nucleic acid sequence derived from the genome of a retrovirus characterized by
- the insertion in a vector capable of transforming a selected cell host and of permitting the expression in this host of an insert contained in this vector of the above-mentioned nucleic acid sequence derived from a human HIV-2 retrovirus capable of infecting human T4 lymphocytes and capable of causing AIDS in human, said HIV-2 retrovirus being represented by the isolates deposited with the CNCM under the Nos I-502, I-532, I-642 and I-643 or with the ECACC under the Nos 87.01.1001 and 87.01.1002, or the insertion of the above-mentioned nucleic acid sequence derived from a variant of this retrovirus, the major envelope glycoprotein of which is recognized by antibodies formed against the major envelope glycoprotein gp140 of an HIV-2 retrovirus,
- the introduction of said vector containing said nucleic acid sequence into the selected host,
- the culture of the transformed cell host and
- the recovery and purification of the protein or a part of the protein expressed by said nucleic acid sequence, said protein or part of protein being capable of producing a specific immunological reaction with antibodies directed against the HIV-2 retrovirus.

2. Process for the production of a protein or a part of a protein according to Claim 1, characterized in that the expression product of all or part of the following nucleic acid sequence, containing the nucleotides 1 to 1566 corresponding to the gag sequence of the genomic RNA of the HIV-2 retrovirus, is recovered and purified.

CAGIODE

ATCCGCGCCCAGAAACTCCGTCTTGACACGGCAAAAAGCAGATGAA

TTACAAACAATCAGGTTACGGCCCCGCGGAAACAAAAAGTACAGG

CTAAAACATATTGTGTGCCGCACCGAATAAATTGGCACAGATTCCGA
100

TTAGCACACAGCCCTGTTGGAGTCAAAACAGCCTTGTCAAAAAATT

CTTACAGTTTTAGATCCAATGGTACCGACACGTTCAGAAAATTTA
200

AAAAGTCTTTTTAATACTGTCTGCGTCATTTGGTGCATACACGCA

GAAGAGAAAGTGAAAGATACTGAAGGAGCAAAACAAATAGTGCGG
300

AGACATCTAGTGGCAGAAACAGGAACTGCAGAGAAAATGCCAAGC

ACAAGTAGACCAACAGCACCATCTAGCCACAACGGACGAAATTAC
400

CCAGTGCAACATGTAGGCCGCAACTACACCCATATACCGCTGACT

CCCCGAACCCTAAATGCCTGGGTAAAATTAGTAGACGAAAAAAG

TTCGGGCCACAAGTAGTGCCAGGATTTCACGCACTCTCAGAAGGG
500

TGCACCCCCTATGATATCAACCAAATGCTTAATTGTGTGGGCGAC

CATCAAGCAGCCATGCAGATAATCAGCGACATTATCAATGAGGAA
600

GCAGCAGAATGGGATGTGCAACATCCAATACCAGGCCCCTTACCA

GCCGGCCAGCTTAGAGAGCCAAGGGGATCTCACATAGCAGGCACA
700

ACAAGCACAGTACAAGAACAGATCCAGTGCATGTTTACGCCACAA

AATCCTGTACCACTACCAAACATCTATACAACATGCATCCACATA
800
CGATTGCAGAAGTGTGTCAGGATGTACAACCCGACCAACATCCTA
CACATAAAACAGCGACCAAAGGAGCCGTTCCAAACCTATGTACAT
900
AGATTCTACAAAAGCTTGAGGGCAGAACAAACACATCCAGCACTG
ATCAATTCGATGACCCAAACACTGCTAGTACAAAATGCCAACCCA
CACTGTAAATTAGTGCTAAAAGGACTACGGATGAACCCTACCTTA
1000
GAAGACATGCTGACCGCCTGTCAGGGGTAGGTGCCCCACCCCAG
AAAGCTAGATTAATGGCAGAGGCCCTGAAAGAGGTCATAGGACCT
1100
GCCCCTATCCCATTCGCAGCAGCCCAGCAGAGAAAGGCATTTAAA
TGCTGGAACTGTGGAAAGGAAGGGCACTCG[?][?]AAGACAATGCCCGA
1200
GCACCTAGAAGGCAGGGCTGCTGGAAGTGTGGTAAGCCAGGACAC
ATCATGACAAACTGCCCAGATAGACAGGCAGGTTTTTTAGGACTG
1300
GGCCCTTGGCGAAAGAAGCCCCGCAACTTCCCCGTGCCCCAAGTT
CCGCAGGGGCTGACACCAACAGCACCCCCAGTGGATCCAGCAGTG
GATCTACTGCAGAAATATATGCAGCAAGGCAAAACACACACACAG
1400
CACAGACACAGACCATACAAGGAAGTGACAGAGGACTTACTGCAC
CTCGACCAGGGGGAGACACCATACAGGGAGCCACCAACAGAGGAC
1500
TTGCTGCACCTCAATTCTCTCTTTGGAAAAGACCAG

3. Process for the production of a protein or glycoprotein or a part of a protein or glycoprotein according to Claim 1, characterized in that the expression product of all or part of the following nucleic acid sequence, containing the nucleotides 1 to 2574 corresponding to the following env sequence of the genomic RNA of the HIV-2 retrovirus, is recovered and purified.

ATGATCAATCAGCTGCTTATTGCCATTTTATTAGCTAGTGCTTGC

TTAGTATATTGCACCCAATATGTAACTGTTTTCTATGGCGTACCC

ACGTGCAAAAATGCAACCATTCCCCTCTTTTGTGCAACCAGAAAT
100

AGCGATACTTGCGGAACCATACAGTGCTTGCCTGCACAATGATGAT

TATCAGGAAATAACTTTGAATGTAACAGAGGCTTTTGATGCATCG
200

AATAATACAGTAACAGAACAAGCAATAGAAGATGTCTGGCATCTA

TTCGAGACATCAATAAAACCATGTGTCAAACTAACACCTTTATGT
300

GTAGCAATGAAATGCAGCAGCACAGAGAGCAGCACAGGCAACAAC

ACAACCTCAAAGAGCACAAGCACAACCACAACCACACCCACAGAG
400

CAGGAGCAAGAGATAAGTGAGGATACTCCATGCGCACCCGCAGAG

AACTGCCTCAGGATTGCGAGAGGAACAAACGATCAATTGCCAGTTC

AATATCACAGGATTAGAAAGAGATAACAAAAACAGTATAATGAA
500

ACATCGTACTCAAAAGATCTGGTTTCTCAGACAAATAATAGCACA

AATCAGACCCACTGTTACATGAACCATTGCAACACATCAGTCATC
600

ACAGAATCATGTGACAAGCACTATTGCCATGCTATAAGGTTTACA

TACTGTGCACCACCGGGTTATGCCCTATTAAGATGTAATGATACC
700

ACATGCACCAGGATGATGCAAACGCAAACTTCCACATGCTTTGGC
800

TTTAATGGCACTAGAGCAGAGAATAGAACATATATCTATTGGCAT

GGCAGAGATAATAGAACTATCATCAGCTTAAACAATATTATAAT
900

CTCAGTTTGCATTGTAAGAGGCCAGCGAATAAGACAGTGAAACAA

ATAATGCTTATGTCAGGACATGTGTTTCACTCCCACTACCAGCCG

ATCAATAAAAGACCCAGACAAGCATGGTGCTGCTTCAAAGGCAAA
1000

TGGAAAGACGCCATGCAGGAGGTGAAGACCCTTGCAAAACATCCC

AGGTATAGAGGAACCAATGACACAAGGAATATTAGCTTTGCAGCG
1100

CCAGCAAAAGGCTCAGACCCAGAAGTAGCATACATGTGGACTAAC

TGCAGAGGAGAGTTTCTCTACTGCAACATGACTTGGTTCCTCAAT
1200

TGCATACAGAATAAGACACACCGCAATTATGCACCGTGCCATATA

AAGCAAATAATTAACACATGGCATAAGGTACCCAGAAATGTATAT
1300

TTGCCTCCCAGGGAAGGGCAGCTGTCCTGCAACTCAACAGTAACG

AGCATAATTGCTAACATTGACTGGCAAAACAATAATCAGACAAAC

ATTACCTTTAGTGCCAGACGTGGCAGAACTATACAGATTGGAGTTG
1400

CCACATTATAAATTGGTACAAATAACACCAATTGCCTTGGCACCT

ACAAAAGCAAAAAAGCATACTCCTCTGCTCACGGCAGACATACAAGA
1500
CGTGTGTTCGTGCTAGGGTTCTTGCGTTTTCTCGCAACAGCACGT
TCTGCAATGGCCCCTCGAGCGTCCCTGACCGTGTCGGCTCAGTCC
1600
CGGACTTTACTGGCCGGCATAGTGCAGCAACAGCAACAGCTGTTG
GACGTGGTCAAGACACAACAAGAACTGTTGCCACTGACCGTCTGG
1700
GCAACGAAAAACCTCCAGGCAAGAGTCACTGCTATAGAGAAGTAC
CTACAGGACCAGGCGCGGCTAAATTCATGGGCATGTGCGTTTAGA
1800
CAAGTCTGCCACACTACTGTACCATGGGTTAATGATTCCTTAGCA
CCTGACTGGCACAATATGACGTGGCAGGAATGGGAAAAACAAGTC
CGCTACCTCGAGGCAAATATCAGTAAAAGTTTAGAACAGGCACAA
1900
ATTCAGCAAGAGAAAAATATGTATGAACTACAAAAATTAAATAGC
TGGGATATTTTTGGCAATTGGTTTGACTTAACCTCCTGGGTCAAG
2000
TATATTCAATATGGAGTGCTTATAATAGTAGCAGTAATAGCTTTA
AGAATAGTCATATATGTAGTACAAATGTTAAGTAGGCTTAGAAAG
2100
GGCTATAGGCCTGTTTTCTCTTCCCCCCCGGTTATATCCAACAG

ATCCATATCCACAAGGACCCGGGACAGCCAGCCAACGAAGAAACA
2200
CAAGAAGACGGTCGAAGCAACGGTGCAGACAGATACTGCCCTGG
CCGATAGCATATATACATTTCCTGATCCGCCACCTGATTCGCCTC
TTGACCAGACTATACAGCATCTGCAGGGACTTACTATCCAGGAGC
2300
TTCCTGACCCTCCAACTCATCTACCAGAATCTCAGAGACTGGCTG
AGACTTAGAACAGCCTTCTTGCAATATGGGTGCGAGTGGATCGAA
2400
GAAGCATTCCAGGCCGCCGCGAGGGCTACAAGAGAGACTCTTGCG
GGCGCGTGCAGGGGCTTGTCGAGGGTATTCGAACGAATCGGAGG
2500
GGAATACTCGCCGTTCCAAGAACGATCAGACAGGCAGCAGAAATC
GCCGTCCTG

4. Process for the production of a protein or a part of a protein according to Claim 1, characterized in that a protein or part of a protein corresponding to all or part of the following amino acid sequence is recovered and purified.

ArgLysAlaPheLys

CysTrpAsnCysGlyLysGluGlyHisSerAlaArgGlnCysArg

AlaProArgArgGlnGlyCysTrpLysCysGlyLysProGlyHis

IleMetThrAsnCysProAspArgGlnAlaGlyPheLeuGlyLeu

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis

LeuGluGlnGlyGluThrProTyrArgGluProProThrGluAsp

LeuLeuHisLeuAsnSerLeuPheGlyLysAspGln

5. Process for the production of a protein or a part of a protein according to Claim 1, characterized in that a protein or part of a protein corresponding to all or part of the following amino acid sequence is recovered and purified.

MetGlyAlaArgAsnSerValLeuArgGlyLysLysAlaAspGlu
LeuGluArgIleArgLeuArgProGlyGlyLysLysLysTyrArg
LeuLysHisIleValTrpAlaAlaAsnLysLeuAspArgPheGly
LeuAlaGluSerLeuLeuGluSerLysGluGlyCysGlnLysIle
LeuThrValLeuAspProMetValProThrGlySerGluAsnLeu
LysSerLeuPheAsnThrValCysValIleTrpCysIleHisAla
GluGluLysValLysAspThrGluGlyAlaLysGlnIleValArg
ArgHisLeuValAlaGluThrGlyThrAlaGluLysMetProSer
ThrSerArgProThrAlaProSerSerGluLysGlyGlyAsnTyr

6. Process for the production of a protein according to Claim 1, characterized in that a protein is recovered and purified having the immunological properties of the protein p12 of HIV-2, recognized immunologically by antibodies formed against a human HIV-2 retrovirus and having a molecular weight of 12,000 daltons determined by electrophoresis in a polyacrylamide gel under denaturating conditions.

7. Process for the production of a protein according to Claim 1, characterized in that a protein is recovered and purified having the immunological properties of the protein p16 of HIV-2, recognized immunologically by antibodies formed against a human HIV-2 retrovirus and having a molecular weight of 16,000 daltons determined by electrophoresis in a polyacrylamide gel under denaturating conditions.

8. Process for the production of a protein according to Claim 1, characterized in that a protein is recovered and purified having the immunological properties of the protein p26 of HIV-2, recognized immunologically by antibodies formed against a human HIV-2 retrovirus and having a molecular weight of 26,000 daltons determined by electrophoresis in a polyacrylamide gel under denaturating conditions.

9. Process for the production of a protein according to Claim 1, characterized in that a protein is recovered and purified having the immunological properties of the protein p36 of HIV-2, recognized immunologically by antibodies formed against a human HIV-2 retrovirus and having a molecular weight of 36,000 daltons determined by electrophoresis in a polyacrylamide gel under denaturating conditions.

10. Process for the production of a protein according to Claim 1, characterized in that a protein is recovered and purified having the immunological properties of the protein p42 of HIV-2, recognized immunologically by antibodies formed against a human HIV-2 retrovirus and having a molecular weight of 42,000-45,000 daltons determined by electrophoresis in a polyacrylamide gel under denaturating conditions.

11. Process for the production of a protein according to Claim 1, characterized in that a protein is recovered and purified having the immunological properties of the envelope glycoprotein gp 140 of HIV-2, recognized immunologically by antibodies formed against a human HIV-2 retrovirus and having a molecular weight of 130,000 - 140,000 daltons determined by electrophoresis in a polyacrylamide gel under denaturating conditions.

12. Cell culture characterized in that it is transformed by a recombinant DNA containing a nucleic acid derived from at least a part of the RNA of the human HIV-2 retrovirus or of one of its variants under conditions permitting the production of a protein or a part of a protein according to the process of Claim 1, said nucleic acid having the capacity to hybridize with the RNA of said human HIV-2 retrovirus and of not hybridizing under stringent conditions with the probes 1 to 4 comprising respectively the nucleotides 990-1070, 990-1260, 2170-2240, 3370-3640 of the DNA derived from the genome of HIV-1.

13. Cell culture according to Claim 12, characterized in that it is a culture of a microorganism, in particular of E. coli.

14. Cell culture according to Claim 12, characterized in that it is a culture of eukaryotic cells, in particular of yeasts.

15. Cell culture according to Claim 12, characterized in that it consists of higher eukaryotic cells such as mammalian cells.

16. Cell culture according to any one of the Claims 12 to 15, characterized in that it is transformed by a nucleic acid containing a nucleotide sequence coding for the following amino acid sequence GAGRODN or for a part of this sequence which has the capacity to give rise to an immunological reaction with antibodies directed against an HIV-2 retrovirus when said sequence is placed in contact with a serum sample of a patient infected with said HIV-2 retrovirus.

GAGRODH

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArgGluIleIleAsnGluGlu

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGlnArgLysAlaPheLys

CysTrpAsnCysGlyLysGluGlyHisSerAlaArgGlnCysArg

AlaProArgArgGlnGlyCysTrpLysCysGlyLysProGlyHis

IleMetThrAsnCysProAspArgGlnAlaGlyPheLeuGlyLeu

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis

LeuGluGlnGlyGluThrProTyrArgGluProProThrGluAsp

LeuLeuHisLeuAsnSerLeuPheGlyLysAspGln

17. Cell culture according to any one of the Claims 12 to 15, characterized in that it is transformed by a nucleic acid coding for the amino acid sequence below or for a part of this sequence, this sequence or a part of this sequence having the capacity to give rise to an immunological reaction with antibodies directed against an HIV-2 retrovirus when said sequence is placed in contact with a serum sample of a patient infected with said HIV-2 retrovirus.

ArgLysAlaPheLys
CysTrpAsnCysGlyLysGluGlyHisSerAlaArgGlnCysArg
AlaProArgArgGlnGlyCysTrpLysCysGlyLysProGlyHis
IleMetThrAsnCysProAspArgGlnAlaGlyPheLeuGlyLeu
GlyProTrpGlyLysLysProArgAsnPheProValAlaGlnVal
ProGlnGlyLeuThrProThrAlaProProValAspProAlaVal
AspLeuLeuGluLysTyrMetGlnGlnGlyLysArgGlnArgGlu
GlnArgGluArgProTyrLysGluValThrGluAspLeuLeuHis
LeuGluGlnGlyGluThrProTyrArgGluProProThrGluAsp
LeuLeuHisLeuAsnSerLeuPheGlyLysAspGln

18. Cell culture according to any one of the Claims 12 to 15, characterized in that it is transformed by a nucleic acid containing a nucleotide sequence coding for all or a part of the amino acid sequence below, this sequence or a part of this sequence having the capacity to give rise to an immunological reaction with antibodies directed against an HIV-2 retrovirus when said sequence is placed in contact with a serum sample of a patient infected with said HIV-2 retrovirus.

MetGlyAlaArgAsnSerValLeuArgGlyLysLysAlaAspGlu
LeuGluArgIleArgLeuArgProGlyGlyLysLysLysTyrArg
LeuLysHisIleValTrpAlaAlaAsnLysLeuAspArgPheGly
LeuAlaGluSerLeuLeuGluSerLysGluGlyCysGlnLysIle
LeuThrValLeuAspProMetValProThrGlySerGluAsnLeu
LysSerLeuPheAsnThrValCysValIleTrpCysIleHisAla
GluGluLysValLysAspThrGluGlyAlaLysGlnIleValArg
ArgHisLeuValAlaGluThrGlyThrAlaGluLysMetProSer
ThrSerArgProThrAlaProSerSerGluLysGlyGlyAsnTyr

19. Cell culture according to any one of the Claims 12 to 15, characterized in that it is transformed by a nucleic acid containing a nucleotide sequence coding for all or a part of the amino acid sequence below, this sequence or a part of this sequence having the capacity to give rise to an immunological reaction with antibodies directed against an HIV-2 retrovirus when said sequence is placed in contact with a serum sample of a patient infected with said HIV-2 retrovirus.

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArgGluIleIleAsnGluGlu

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGln

20. Use of the process according to any one of the Claims 1 to 11 for the production of proteins or parts of proteins which can be used in reactions to detect antibodies directed against the HIV-2 retrovirus.

21. Use of the process according to any one of the Claims 1 to 11 for the production of immunogenic proteins or parts of immunogenic proteins.

## Patentansprüche

1. Verfahren zur Herstellung eines Proteins oder eines Glycoproteins oder eines Protein- oder Glycoproteinteils, bestehend aus dem Expressionsprodukt einer Nucleinsäuresequenz, welche vom Genom eines Retrovirus abgeleitet ist, gekennzeichnet durch
- die Insertion eines Vektors, der geeignet ist, einen ausgewählten Zell-Wirt zu transformieren und die Expression eines in diesem Vektor bekannten Inserats in diesem Wirt zu gestatten, der oben genannten Nucleinsäuresequenz, abgeleitet von einem humanen HIV-2-Retrovirus, welcher humane T4-Lumphozyten infizieren kann und am Menschen Aids hervorrufen kann, wobei der HIV-2-Retrovirus durch die Isolate dargestellt ist, welche bei CNCM unter den Nummern I-502, I-532, I-642 und I-643 oder bei ECACC unter den Nummern 87.01.1001 und 87.01.1002 hinterlegt sind, oder die Insertion der oben genannten Nucleinsäuresequenz, abgeleitet von einer Variante dieses Retrovirus, dessen Haupt-Hüllenglycoprotein durch Antikörper erkannt wird, welche gegen das Haupthüllenglycoprotein gp140 eines HIV-2-Retrovirus gebildet sind,
- das Einführen des die genannte Nucleinsequenz enthaltenden Vektors in den ausgewählten Wirt,
- die Zucht des transformierten Zell-Wirtes und
- das Gewinnen und die Reinigung des durch die genannte Nucleinsäuresequenz exprimierten Proteins oder Proteinteils, wobei das Protein oder der Proteinteil fähig zur Erzeugung einer spezifischen immunologischen Reaktion mit gegen den HIV-2-Retrovirus gerichteten Antikörpern ist.

2. Verfahren zur Herstellung eines Proteins oder Proteinteils des Proteins gemäß Anspruch 1, dadurch gekennzeichnet, dass man das Expressionsprodukt ganz oder teilweise aus der folgenden Nucleinsäuresequenz gewinnt und reinigt, welche die Nucleotide 1 bis 1566 enthält, entsprechend der gag Sequenz der genomischen RNS des HIV-2-Retrovirus:

CACIODE -

ATCCCCCCCGACAAACTCCCTCTTCACACCCAAAAACCACATCAA

TTACAAACAATCACCTTACCCCCCCCCCAAACAAAACTACACC

CTAAAACATATTCTCTCCCCACCCAATAAATTCCACACATTCCCA
100

TTACCACACACCCTCTTCCACTCAAAACACCCTTCTCAAAAAATT

CTTACACTTTTACATCCAATCCTACCCACACCTTCACAAAATTA
200

AAAACTCTTTTTAATACTCTCTCCCTCATTTCCTCCATACACCCA

CAACAGAAACTCAAACATACTCAACCACCAAACAAATACTCCCC
300

ACACATCTAGTCCCACAAACACCAACTCCACACAAAATCCCAACC

ACAACTACACCAACACCACCATCTACCCACAACCCACCAAATTAC
400

CCACTCCAACATCTACCCCCCAACTACACCCATATACCCCTCACT

CCCCGAACCCTAAATCCCTCCCTAAAATTAGTACACCAAAAAAAC

TTCCCCCCACAACTACTCCCAGCATTTCACCCACTCTCACAACCC
500

TCCACCCCCTATCATATCAACCAAAATCCTTAATTCTCTCCCCCAC

CATCAACCACCCATCCACATAATCACCCACATTATCAATCACCAA
600

CCACCAGAATCCCATCTCCAACATCCAATACCACCCCCCTTACCA

CCCCCCCCACCTTACACACCCCAACCCCATCTCACATACCAGCCACA
700

ACAACCACACTACAACAACACATCCACTCCATCTTTACCCCACAA

AATCCTGTACCACTAGGAAACATGTATACAAGATGGATCCAGATA
800
CGATTCCAGAAGTGTGTCACCATGTATCAACCCGACCAACATCCTA

GACATAAAACAGCGGACCAAAGCACCCGTTCCAAAGCTATCTACAT
900
AGATTCTACAAAACCTTCAGCGCACAACAAACAGATCCAGGACTG

AAGAATTCGATGACCCAAACACTGCTAGTACAAAATGCCAACCCA

GACTGTAAATTACTGCTAAAAGCACTACCCATGAACCCTACCTTA
1000
GAAGACATGCTGACCCCCTGTCAGCGGGTACGTGGGCCACCCCAG

AAAGCTACATTAATGGCAGAGGCCCTGAAACAGGTCATAGGACGT
1100
GCCCCTATCCCATTCCCAGCAGCCCACCAGAGAAACGGATTTAAA

TGCTGGAACTGTGCAAACCAAGGGCACTCCCCAAGACAATGCCGA
1200
GCACCTAGAAGGCAGGGCTGCTGCAAGTGTCCTAAGCCAGGACAC

ATCATGACAAACTGCCCAGATACACACGCACGTTTTTTACGACTG
1300
GGCGCTTGCCGAAAGAAGCCCCCCAACTTCCCCGTGCCCAGTT

CCCCACCCGCTGACACCCACAGCACCCCCACTCGATCCAGCACTG

GATCTACTGCAGAAATATATGCAGCAAGGAAAAGACACACACAG
1400
CAGACACACAGACCATACAAGCAAGTCACAGACCACTTACTGCAG

CTCCACCACCCCCAGACACCATACAGCCAGCCACCAACACACGAC
1500
TTGCTGCACCTCAATTCTCTCTTTGGAAAAGACCAG

3. Verfahren zur Herstellung eines Proteins oder eines Glycoproteins oder eines Protein- oder Glycoproteinteils gemäß Anspruch 1, dadurch gekennzeichnet, dass man das Expressionsprodukt ganz oder teilweise aus der folgenden Nucleinsäuresequenz gewinnt und reinigt, welche die Nucleotide 1 bis 2574 enthält, entsprechend der folgenden env Sequenz der genomischen RNS des HIV-2-Retrovirus:

ATGATCAATCAGCTGCTTATTGCCATTTTATTAGCTAGTCCTTGC

TTAGTATATTGCACCCAATATGTAACTGTTTTCTATGCCGTACCC

ACCTGCAAAAATGCAACCATTCCCCTCTTTTCTGCCAACCACAAAT
100

ACCCATACTTCGGGAACCATACAGTGCTTGCCTCAGAATGATGAT

TATCAGCAAATAACTTTGAATCTAACACAGGCTTTTGATGCATGG
200

AATAATACAGTAACAGAACAAGCAATAGAACATGTGTGCCATCTA

TTCCAGACATCAATAAAACCATGTGTCAAACTAACACCTTTATGT
300

GTAGCAATGAAATGCAGCAGCACAGAGAGCAGCACAGGGAACAAG

ACAACCTCAAAGAGCACAACCACAACCACAACCACACCCACACAG
400

CAGCAGCAACAGATAAGTGAGGATACTCCATGCCCACCCCCAGAG

AACTGCCTCAGGATTGCCAGAGGAACAAACCATCAATTGCCAGTTC

AATATGACACGCATTAGAAACACATAACAAAAACAGTATAATGAA
500

ACAACGTACTCAAAAGATGTGGTTTCTGAGACAAATAATAGCACA

AATCACACCCACTGTTACATCAACCATTGCAACACATCACTCATC
600

ACACAATCATCTCACAAGCACTATTGCCCATGCTATAACCTTTACA

TACTGTCCACCACCGCCTTATGCCCTATTAACATCTAATGATACC
700

AATTATTCACGCTTTCCACC[?]AACTCTTCT[?][?]ACTACTACCTTC[?]

ACATGCACCACGATCATCCAAACCCAAACTTCCACATGCTTTGCC
800
TTTAATGCCACTAGACGCAGAGAATAGAACATATATCTATTGGCAT

GCCAGAGATAATACAACTATGATCAGCTTAAACAATATTATAAT
900
CTCACTTTGCATTCTAACAGCGCAGCGAATAACACAGTGAAACAA

ATAATGCTTATGTCAGCACATGTGTTTCACTCCCACTACCAGCCG

ATCAATAAAACACCCAGACAAGCATGCTCCTGCTTCAAAGGCAAA
1000
TGCAAAGACGCCATGCACGAGGTGAAGACCCTTGCAAAACATCCC

AGGTATAGACGAACGAATGACACAAGCAATATTAGCTTTGCAGCG
1100
CCAGGAAAAGGCTCAGACCCACAAGTACCATACATGTGGACTAAC

TGCAGAGGAGAGTTTGTCTACTGCAACATCACTTGCTTCCTCAAT
1200
TGCATAGAGAATAAGACACACCGCAATTATCCACCCTGCCATATA

AACCAAATAATTAACACATCGGCATAACGTAGCGACAAATGTATAT
1300
TTGCCTCCGACGGAAGGGCAGCTGTCCTCCAAGTCAACACTAACG

AGCATAATTGCTAACATTGACTGCCAAAACAATAATCAGACAAAC

ATTACCTTTACTGCCAGACCTCGCAGAACTATACAGATTGCAGTTC
1400
CCACATTATAAATTCGTACAAATAACACCAATTGCCTTCCCACCT

ACAAAACAAAAAAGATACTCCTCTCCTCACGGCAGACATACAACA
1500
CGTGTGTTCGTGCTAGGGTTCTTGCGTTTTCTCGCAACACCACGT
TCTGCCAATGCGCGGCTCGAGCGTCCCTGACCCTGTCGGCTCAGTCC
1600
CCGACTTTACTCGCCCGGCATACTGCACCAACAGCAACAGCCTGTTG
CACGTCGTCAAGCAGACAACAACAACTGTTCCCACTGACCCGTCTGC
1700
GCAACCGAAAAACCTCCAGGCAAGAGTCACTGCCATACAGAACTAC
CTACAGGACCCAGGCGCCGCTAAATTCATGCGGATGTGCGTTTAGA
1800
GAAGTCTGCCCACACTACTGTACCATCGGTTAATCATTCCTTAGCA
CCTGACTGGGACAATATGACCTCGCAGGAATGCGAAAAACAAGTC
CGCTACCTGCAGGCAAATATCACTAAAAGTTTAGAACAGGCACAA
1900
ATTCAGCAACAGAAAAATATGTATGAACTACAAAAATTAAATACC
TGGATATTTTTGGCAATTGGTTTGACTTAACCTGCTGGGTCAAG
2000
TATATTCAATATGCACTCCTTATAATACTACCAGTAATAGCTTTA
AGAATACTGATATATGTACTACAAATGTTAAGTAGCCTTACAAAG
2100
CGCTATAGCCCTGTTTTCTCTCCCCCCCCCGTTATATCCAACAG

ATCCATATCCACAACGACCCGGGACACCCACCCAACGAAGAAACA
2200
CAACAACACCCTCGAACCAACGGTCGACACAGATACTGCCCCTGC
CCGATAGCATATATACATTTCCTGATCCCCCACCTCATTCCCCTC
TTGACCAGACTATACAGCATCTGCAGCGACTTACTATCCAGCAGC
2300
TTCCTCACCCTCCAACTCATCTACCAGAATCTCACAGACTGGGTG
ACACTTAGAACACCCTTCTTGCAATATGGGTGCGACTCGATCGAA
2400
CAAGCATTCCACGCCCGCCCCGACGGCTACAAGAGAGACTCTTGCG
GGCCCGTCCACGGGCTTGTGGAGGGTATTGCAACCAATCGGGAGG
2500
GGAATACTCGGCGTTCCAACAAGCATCAGACAGGGAGCAGAAATC
GCCCTCCTC

4. Verfahren zur Herstellung eines Proteins oder Proteinteils nach Anspruch 1, dadurch gekennzeichnet, dass man ein Protein oder einen Proteinteil, welcher ganz oder teilweise der folgenden Aminosäuresequenz entspricht, gewinnt und reinigt:

ArgLysAlaPheLys

CysTrpAsnCysGlyLysGluGlyHisSerAlaArgGlnCysArg

AlaProArgArgGlnGlyCysTrpLysCysGlyLysProGlyHis

IleMetThrAsnCysProAspArgGlnAlaGlyPheLeuGlyLeu

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis

LeuGluGlnGlyGluThrProTyrArgGluProProThrGluAsp

LeuLeuHisLeuAsnSerLeuPheGlyLysAspGln

5. Verfahren zur Herstellung eines Proteins oder eines Proteinteils gemäß Anspruch 1, dadurch gekennzeichnet, dass man ein Protein oder einen Proteinteil, welcher ganz oder teilweise der folgenden Aminosäuresequenz entspricht, gewinnt und reinigt:

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

6. Verfahren zur Herstellung eines Proteins gemäß Anspruch 1, dadurch gekennzeichnet, dass man ein Protein mit den immunologischen Eigenschaften des Proteins p12 von HIV-2 gewinnt und reinigt, das immunologisch durch gegen einen humanen HIV-2-Retrovirus gebildete Antikörper erkannt wird und ein Molekulargewicht von 12000 Dalton, bestimmt durch Elektrophorese auf einem Polyacrylamid-Gel unter denaturierenden Bedingungen, aufweist.

7. Verfahren zur Herstellung eines Proteins nach Anspruch 1, dadurch gekennzeichnet, dass man ein Protein mit den immunologischen Eigenschaften des Proteins p16 von HIV-2 gewinnt und reinigt, das immunologisch durch gegen einen humanen HIV-2-Retrovirus gebildete Antikörper erkannt wird und ein Molekulargewicht von 16000 Dalton, bestimmt durch Elektrophorese auf einem Polyacrylamid-Gel unter denaturierenden Bedingungen, aufweist.

8. Verfahren zur Herstellung eines Proteins nach Anspruch 1, dadurch gekennzeichnet, dass man ein Protein mit den immunologischen Eigenschaften des Proteins p26 von HIV-2 gewinnt und reinigt, das immunologisch durch gegen einen humanen HIV-2-Retrovirus gebildete Antikörper erkannt wird und ein Molekulargewicht von 26000 Dalton, bestimmt durch Elektrophorese auf einem Polyacrylamid-Gel unter denaturierenden Bedingungen, aufweist.

9. Verfahren zur Herstellung eines Proteins nach Anspruch 1, dadurch gekennzeichnet, dass man ein Protein mit den immunologischen Eigenschaften des Proteins p36 von HIV-2 gewinnt und reinigt, das immunologisch durch gegen einen humanen HIV-2-Retrovirus gebildete Antikörper erkannt wird und ein Molekulargewicht von 36000 Dalton, bestimmt durch Elektrophorese auf einem Polyacrylamid-Gel unter denaturierenden Bedingungen, aufweist.

10. Verfahren zur Herstellung eines Proteins nach Anspruch 1, dadurch gekennzeichnet, dass man ein Protein mit den immunologischen Eigenschaften des Proteins p42 von HIV-2 gewinnt und reinigt, das immunologisch durch gegen einen humanen HIV-2-Retrovirus gebildete Antikörper erkannt wird und ein Molekulargewicht von 42000 - 45000 Dalton, bestimmt durch Elektrophorese auf einem Polyacrylamid-Gel unter denaturierenden Bedingungen, aufweist.

11. Verfahren zur Herstellung eines Proteins nach Anspruch 1, dadurch gekennzeichnet, dass man ein Protein mit den immunologischen Eigenschaften des Hüllenglycoproteins gp 140 von HIV-2 gewinnt und reinigt, das immunologisch durch gegen einen humanen HIV-2-Retrovirus gebildete Antikörper erkannt wird und ein Molekulargewicht von 130.000 - 140.000 Dalton, bestimmt durch Elektrophorese auf einem Polyacrylamid-Gel unter denaturierenden Bedingungen, aufweist.

12. Zellkultur, dadurch gekennzeichnet, dass sie durch eine rekombinante DNS transformiert wird, welche eine Nucleinsäure enthält, die von einem Teil wenigstens der RNS des humanen HIV-2-Retrovirus oder einer seiner Varianten abgeleitet ist, unter Bedingungen, welche die Erzeugung eines Proteins oder Proteinteils nach dem Verfahren von Anspruch 1 erlaubt, wobei die Nucleinsäure sich mit der RNS des genannten humanen HIV-2-Retrovirus hybridisieren kann und unter den stringenten Bedingungen nicht mit den Sonden 1 bis 4 hybridisiert, welche jeweils die Nucleotide 990-1070, 990-1260, 2170-2240, 3370-3640 der von dem HIV-1-Genom abgeleiteten DNS umfasst.

13. Zellkultur nach Anspruch 12, dadurch gekennzeichnet, dass es sich um eine Mikroorganismenkultur, insbesondere E.coli, handelt.

14. Zellkultur nach Anspruch 12, dadurch gekennzeichnet, dass es sich um eine eukariotische Zellkultur, insbesondere Hefen, handelt.

15. Zellkultur nach Anspruch 12, dadurch gekennzeichnet, dass es sich um höhere eukariotische Zellen, wie Säugetierzellen, handelt.

16. Zellkultur gemäß einem der Ansprüche 12 bis 15, dadurch gekennzeichnet, dass sie durch eine Nucleinsäure transformiert wird, welche eine Nucleotidsequenz enthält, die für die folgende Aminosäuresequenz GAGRODN oder einen Teil dieser Sequenz kodiert, die eine immunologische Reaktion mit gegen einen HIV-2-Retrovirus gerichteten Antikörpern ergeben kann, wenn die genannte Sequenz in Kontakt mit dem Serum eines mit dem genannten HIV-2-Retrovirus infizierten Patienten in Kontakt gebracht wird.

GAGROD[?]

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

ProValGlnHisValGlyGlyAsnTyrThrHisIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArg[?]IleIleAsnGluGlu

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

900
ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGlnArgLysAlaPheLys

CysTrpAsnCysGlyLysGluGlyHisSerAlaArgGlnCysArg

AlaProArgArgGlnGlyCysTrpLysCysGlyLysProGlyHis

IleMetThrAsnCysProAspArgGlnAlaGlyPheLeuGlyLeu

GlyProTrpGlyLysLysProArgAsnPheProValAlaGlnVal

ProGlnGlyLeuThrProThrAlaProProValAspProAlaVal

AspLeuLeuGluLysTyrMetGlnGlnGlyLysArgGlnArgGlu

GlnArgGluArgProTyrLysGluValThrGluAspLeuLeuHis

LeuGluGlnGlyGluThrProTyrArgGluProProThrGluAsp

LeuLeuHisLeuAsnSerLeuPheGlyLysAspGln

17. Zellkultur nach einem der Ansprüche 12 bis 15, dadurch gekennzeichnet, dass sie durch eine Nucleinsäure transformiert ist, die für die nachfolgende Aminosäuresequenz oder einen Teil dieser Sequenz kodiert, wobei diese Sequenz oder der Teil dieser Sequenz eine immunologische Reaktion mit Antikörpern gegen einen HIV-2-Retrovirus ergeben kann, wenn die genannte Sequenz mit einem Serum eines mit dem genannten HIV-2-Retrovirus infizierten Patienten in Kontakt gebracht wird:

ArgLysAlaPheLys
CysTrpAsnCysGlyLysGluGlyHisSerAlaArgGlnCysArg
AlaProArgArgGlnGlyCysTrpLysCysGlyLysProGlyHis
IleMetThrAsnCysProAspArgGlnAlaGlyPheLeuGlyLeu
GlyProTrpGlyLysLysProArgAsnPheProValAlaGlnVal
ProGlnGlyLeuThrProThrAlaProProValAspProAlaVal
AspLeuLeuGluLysTyrMetGlnGlnGlyLysArgGlnArgGlu
GlnArgGluArgProTyrLysGluValThrGluAspLeuLeuHis
LeuGluGluGlyGluThrProTyrArgGluProProThrGluAsp
LeuLeuHisLeuAsnSerLeuPheGlyLysAspGln

18. Zellkultur nach einem der Ansprüche 12 bis 15, dadurch gekennzeichnet, dass sie durch eine Nucleinsäure transformiert wird, welche eine Nucleotidsequenz enthält, die für die Gesamtheit oder einen Teil der nachfolgenden Aminosäuresequenz kodiert, wobei diese Sequenz oder der Teil dieser Sequenz eine immunologische Reaktion mit Antikörpern gegen einen HIV-2-Retrovirus ergeben kann, wenn die genannte Sequenz in Kontakt mit einem Serum eines durch den genannten HIV-2-Retrovirus infizierten Patienten gebracht wird:

MetGlyAlaArgAsnSerValLeuArgGlyLysLysAlaAspGlu

LeuGluArgIleArgLeuArgProGlyGlyLysLysLysTyrArg

LeuLysHisIleValTrpAlaAlaAsnLysLeuAspArgPheGly

LeuAlaGluSerLeuLeuGluSerLysGluGlyCysGlnLysIle

LeuThrValLeuAspProMetValProThrGlySerGluAsnLeu

LysSerLeuPheAsnThrValCysValIleTrpCysIleHisAla

GluGluLysValLysAspThrGluGlyAlaLysGlnIleValArg

ArgHisLeuValAlaGluThrGlyThrAlaGluLysMetProSer

ThrSerArgProThrAlaProSerSerGluLysGlyGlyAsnTyr

19. Zellkultur nach einem der Ansprüche 12 bis 15, dadurch gekennzeichnet, dass sie durch eine Nucleinsäure transformiert ist, welche eine Nucleotidsequenz enthält, die für die Gesamtheit oder einen Teil der nachfolgenden Aminosäuresequenz kodiert, wobei diese Sequenz oder der Teil dieser Sequenz eine immunologische Reaktion mit Antikörpern gegen einen HIV-2-Retrovirus ergeben kann, wenn die genannte Sequenz in Kontakt mit einem Serum eines durch den genannten HIV-2-Retrovirus infizierten Patienten gebracht wird:

ProValGlnHisValGlyGlyArgTyrThrIleIleProLeuSer

ProArgThrLeuAsnAlaTrpValLysLeuValGluGluLysLys

PheGlyAlaGluValValProGlyPheGlnAlaLeuSerGluGly

CysThrProTyrAspIleAsnGlnMetLeuAsnCysValGlyAsp

HisGlnAlaAlaMetGlnIleIleArgGlnIleIleAsnGluGln

AlaAlaGluTrpAspValGlnHisProIleProGlyProLeuPro

AlaGlyGlnLeuArgGluProArgGlySerAspIleAlaGlyThr

ThrSerThrValGluGluGlnIleGlnTrpMetPheArgProGln

AsnProValProValGlyAsnIleTyrArgArgTrpIleGlnIle

GlyLeuGlnLysCysValArgMetTyrAsnProThrAsnIleLeu

AspIleLysGlnGlyProLysGluProPheGlnSerTyrValAsp

ArgPheTyrLysSerLeuArgAlaGluGlnThrAspProAlaVal

LysAsnTrpMetThrGlnThrLeuLeuValGlnAsnAlaAsnPro

AspCysLysLeuValLeuLysGlyLeuGlyMetAsnProThrLeu

GluGluMetLeuThrAlaCysGlnGlyValGlyGlyProGlyGln

LysAlaArgLeuMetAlaGluAlaLeuLysGluValIleGlyPro

AlaProIleProPheAlaAlaAlaGlnGln

20. Verwendung des Verfahrens nach einem der Ansprüche 1 bis 11 zur Herstellung von Proteinen oder Teilen von Proteinen, welche bei Nachweisreaktionen von Antikörpern verwendbar sind, die gegen den HIV-2-Retrovirus gerichtet sind.

21. Verwendung des Verfahrens nach einem der Ansprüche 1 bis 11 zur Herstellung von immunogenen Proteinen oder Teilen davon.
